(19) Europäisches Patentamt / European Patent Office / Office européen des brevets

(11) EP 4 578 855 A1

(12) EUROPEAN PATENT APPLICATION
published in accordance with Art. 153(4) EPC

(43) Date of publication:
**02.07.2025 Bulletin 2025/27**

(21) Application number: **23867460.0**

(22) Date of filing: **18.09.2023**

(51) International Patent Classification (IPC):
***C07D 471/04*** *(2006.01)* ***A61P 7/06*** *(2006.01)*
***A61P 9/10*** *(2006.01)* ***A61K 31/4375*** *(2006.01)*
***A61K 31/5377*** *(2006.01)*

(52) Cooperative Patent Classification (CPC):
**A61K 31/4375; A61K 31/5377; A61P 7/06; A61P 9/10; C07D 471/04**

(86) International application number:
**PCT/CN2023/119468**

(87) International publication number:
**WO 2024/061172 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.09.2022 CN 202211168492**
**02.06.2023 CN 202310650187**

(71) Applicants:
- **Suzhong Pharmaceutical Group Co., Ltd.**
  **Taizhou, Jiangsu 225500 (CN)**
- **Jiangsu Suzhong Pharmaceutical Research Institute Co., Ltd.**
  **Nanjing, Jiangsu 210061 (CN)**

(72) Inventors:
- **TANG, Haitao**
  **Taizhou, Jiangsu 225500 (CN)**
- **TANG, Fan**
  **Taizhou, Jiangsu 225500 (CN)**
- **GE, Haitao**
  **Taizhou, Jiangsu 225500 (CN)**

(74) Representative: **HGF**
**HGF Limited**
**1 City Walk**
**Leeds LS11 9DX (GB)**

(54) **PROLYL HYDROXYLASE INHIBITOR AND USE THEREOF**

(57) The present invention discloses a prolyl hydroxylase domain inhibitor, and belongs to the technical field of medicines. The invention provides a pyridino-triazole compound, a structure of which is shown in the following formula, and the pyridino-triazole compound has higher prolyl hydroxylase domain inhibitory activity and EPO inducing activity, effectively treats and prevents HIF-related and/or EPO-related diseases, and provides a new thought for anemia, ischemia, hypoxia and other diseases.

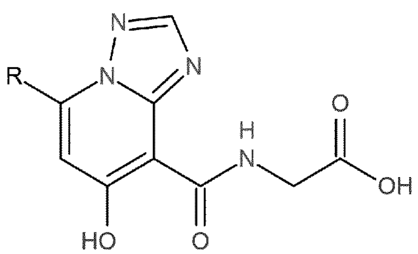

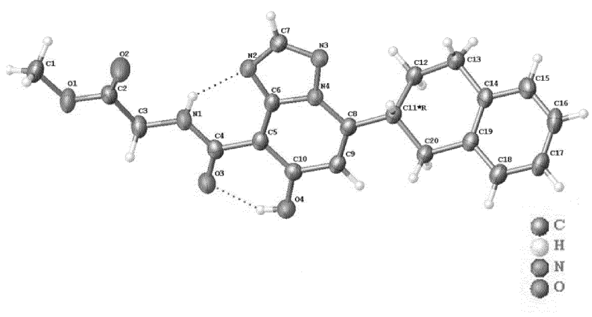

FIG. 2

EP 4 578 855 A1

## Description

### TECHNICAL FIELD

The present invention belongs to the technical field of medicines, and particularly relates to a pyridine-triazole compound with a prolyl hydroxylase domain inhibiting effect and a pharmaceutical composition thereof, and further relates to a preparation method thereof and a pharmaceutical use thereof.

### BACKGROUND

In the case of anemia, trauma, and tissue necrosis and defect, tissues or cells are often in a state of hypoxia. The hypoxia leads to the expression of a series of transcription inducible factors, which are involved in angiogenesis, iron and glucose metabolism, and cell growth and proliferation. Hypoxia inducible factor (HIF) is a transcription factor initiated by somatic cells in the state of hypoxia, which responds to cellular hypoxia by mediating a series of gene regulation in biological cells. The HIF is a heterodimer containing an oxygen-regulated $\alpha$-subunit (HIF$\alpha$) and a constitutively expressed $\beta$-subunit (HIF$\beta$/ARNT). In oxygen-containing (normoxic) cells, the HIF$\alpha$ subunit is rapidly degraded by ubiquitination of an E3 ligase complex of a von Hippel-Lindau tumor suppressor (pVHL). Under the condition of hypoxia, the HIF$\alpha$ is not degraded, and an active HIF$\alpha$/$\beta$ complex accumulates in a nucleus, and activates the expression of various genes, comprising a glycolytic enzyme, a glucose transporter, an erythropoietin (EPO) and a vascular endothelial growth factor (VEGF).

The erythropoietin (EPO) is a naturally occurring hormone generated with the HIF$\alpha$, which stimulates the generation of erythrocyte lipids (erythrocytes) carrying oxygen throughout the body. The EPO is usually secreted by the kidney, and the endogenous EPO is increased under the condition of oxygen reduction (hypoxia). All types of anemia are characterized by a decrease in oxygen carrying capacity of blood, accompanied by similar signs and symptoms, comprising pale skin and mucosa, weakness, dizziness, fatigue and lethargy, thus leading to a decline in quality of life. Anemia is usually related to the patient's condition of leiphemia in erythrocytes or hemoglobin. The common causes of anemia comprise iron, vitamin B12 and folic acid deficiency, and the anemia may also be complicated with chronic diseases, such as inflammatory diseases, comprising diseases with secondary osteomyelitis inhibition. The anemia is also related to renal dysfunction, and most renal failure patients who have regular dialysis suffer from chronic anemia.

Prolyl hydroxylase domain (PHD) is a key factor to regulate the HIF. In a state of normoxia, the PHD may hydroxylate two key proline residues Pro402 and Pro564 of the HIF$\alpha$, so as to increase the affinity with the pVHL, and accelerate the degradation process. In the state of hypoxia and other pathological states, the HIF reaction catalyzed by the PHD is blocked, and the degradation rate of protease is slowed down, resulting in the accumulation of the HIF$\alpha$ in cells, thus causing a series of adaptive responses of cells to the hypoxia. A PHD inhibitor inhibits the PHD and prolongs the effect of the HIF, so as to increase the expression of EPO and other genes, which can effectively treat and prevent HIF-related and/or EPO-related diseases, such as anemia, ischemia and hypoxia.

For example, the U.S. patent application US16757333 discloses a crystalline form of an alkynyl pyridine prolyl hydroxylase domain inhibitor and a preparation method thereof, wherein a structural formula of the alkynyl pyridine prolyl hydroxylase domain inhibitor is as follows:

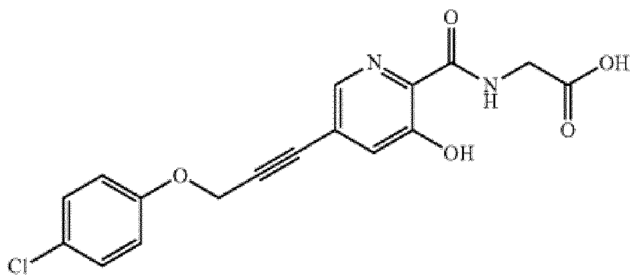

At present, prolyl hydroxylase domain inhibitors have been marketed, comprising Roxadustat from AstraZeneca and Enarodustat from Japan Tobacco Co., Ltd.

Due to the limited development and high demand of the prolyl hydroxylase domain inhibitors, it is urgent to develop such compounds to treat anemia, ischemia and hypoxia.

## SUMMARY

The present invention aims to provide a pyridine-triazole compound as a HIF-PHD inhibitor and a pharmaceutical composition thereof, which may be used for treating various HIF-related or EPO-related diseases, such as anemia.

In order to achieve the above objective of invention, the technical solutions of the present invention are as follows.

In one aspect, the present invention provides a compound as shown in formula (I) or a stereoisomer, geometric isomer, tautomer, nitrogen oxide, hydrate, solvate, pharmaceutically acceptable salt or prodrug thereof;

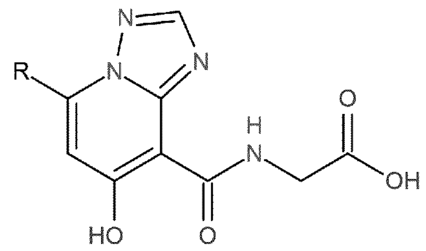

(I)

wherein,

R is a unit of the following atom or group:

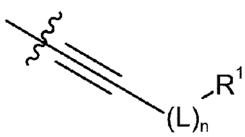 , 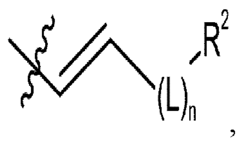 , 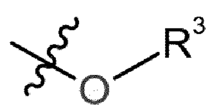 or 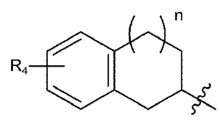 ;

L is $-CH_2-$ or $-CH_2O-$;
a numerical value of n is an integer between 0 and 2; and
$R^1$, $R^2$ and $R^3$ are independently substituted or unsubstituted alkyl, cycloalkyl, aryl or heterocyclyl.

Preferably, the aryl comprises a heteroaromatic ring.

In some embodiments,

L is $-CH_2-$ or $-CH_2O-$;
the numerical value of n is 0 or 1;
$R^1$ is substituted or unsubstituted $C_{1-4}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;
$R^2$ is substituted or unsubstituted $C_{1-4}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;
$R^3$ is substituted or unsubstituted $C_{1-10}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl; and
$R^4$ is at least one of hydrogen, substituted or unsubstituted alkyl, cycloalkyl, alkoxy, aryl and heterocyclyl.

In some embodiments,

L is $-CH_2-$ or $-CH_2O-$;
the numerical value of n is 0 or 1;
$R^1$ is substituted or unsubstituted phenyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen-

and/or nitrogen-containing 5-6 membered heterocyclyl;

$R^2$ is substituted or unsubstituted phenyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;

$R^3$ is substituted or unsubstituted $C_{1-10}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, halogen, oxygen- and/or nitrogen-containing 5-6 membered aromatic heterocyclyl, and oxygen- and/or nitrogen-containing 5-6 membered heterocyclyl; and

$R^4$ is at least one of hydrogen, substituted or unsubstituted alkyl, cycloalkyl, alkoxy, aryl and heterocyclyl,

or, $R^4$ is hydrogen, substituted or unsubstituted $C_{1-10}$ alkyl, $C_{1-10}$ alkoxy, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl,

or, $R^4$ is hydrogen, substituted or unsubstituted $C_{1-10}$ alkyl, $C_{1-10}$ alkoxy, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, halogen, oxygen- and/or nitrogen-containing 5-6 membered aromatic heterocyclyl, and oxygen- and/or nitrogen-containing 5-6 membered heterocyclyl.

In some embodiments,

the numerical value of n is 0 or 1;

$R^1$ is substituted or unsubstituted phenyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^2$ is substituted or unsubstituted phenyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^3$ is substituted or unsubstituted $C_{1-5}$ alkyl, phenyl, oxygen- and/or nitrogen-containing 5-6 membered aromatic heterocyclyl, or oxygen- and/or nitrogen-containing 5-6 membered heterocyclyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure; and

$R^4$ is hydrogen, substituted or unsubstituted $C_{1-5}$ alkyl, $C_{1-5}$ alkoxy, phenyl, oxygen- and/or nitrogen-containing 5-6 membered aromatic heterocyclyl, or oxygen- and/or nitrogen-containing 5-6 membered heterocyclyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure.

In some embodiments,

the numerical value of n is 0 or 1;

$R^1$ is substituted or unsubstituted phenyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^2$ is substituted or unsubstituted phenyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^3$ is substituted or unsubstituted $C_{1-5}$ alkyl, phenyl, oxygen- and/or nitrogen-containing 5-6 membered aromatic heterocyclyl, or oxygen- and/or nitrogen-containing 5-6 membered heterocyclyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure; and

$R^4$ is hydrogen, substituted or unsubstituted $C_{1-5}$ alkyl, $C_{1-5}$ alkoxy, phenyl, oxygen- and/or nitrogen-containing 5-6 membered aromatic heterocyclyl, or oxygen- and/or nitrogen-containing 5-6 membered heterocyclyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure.

In some embodiments,

the numerical value of n is 0 or 1;

$R^1$ is substituted or unsubstituted phenyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the F, Cl, Br or I is ortho-substituted, meta-substituted or para-substituted relative to the parent nucleus structure;

$R^2$ is substituted or unsubstituted phenyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the F, Cl, Br or I is ortho-substituted, meta-substituted or para-substituted relative to the parent nucleus structure;

$R^3$ is substituted or unsubstituted $C_{1-4}$ alkyl, phenyl, oxygen- and/or nitrogen-containing 5-6 membered aromatic heterocyclyl, or oxygen- and/or nitrogen-containing 5-6 membered heterocyclyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure; and

$R^4$ is hydrogen, substituted or unsubstituted $C_{1-4}$ alkyl, $C_{1-4}$ alkoxy, phenyl, oxygen- and/or nitrogen-containing 5-6 membered aromatic heterocyclyl, or oxygen- and/or nitrogen-containing 5-6 membered heterocyclyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure.

In some embodiments,

the numerical value of n is 0 or 1;

$R^1$ is substituted or unsubstituted phenyl, and the substituent is methyl, methoxy, F, Cl, oxygen- and/or nitrogen-containing 5-6 membered aromatic heterocyclyl, or oxygen- and/or nitrogen-containing 5-6 membered heterocyclyl; and preferably, the methyl, methoxy, or oxygen- and/or nitrogen-containing 5-6 membered aromatic heterocyclyl is para-substituted relative to a parent nucleus structure, and the F or Cl is ortho-substituted, meta-substituted or para-substituted relative to the parent nucleus structure;

$R^2$ is substituted or unsubstituted phenyl, and the substituent is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the For Cl is ortho-substituted, meta-substituted or para-substituted relative to the parent nucleus structure;

$R^3$ is substituted or unsubstituted $C_{1-3}$ alkyl, phenyl, or oxygen- and/or nitrogen-containing 5-6 membered aromatic heterocyclyl, and the substituent of the substituted phenyl is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure; and

$R^4$ is hydrogen, substituted or unsubstituted $C_{1-3}$ alkyl, $C_{1-3}$ alkoxy, phenyl, or oxygen- and/or nitrogen-containing 5-6 membered aromatic heterocyclyl, and the substituent of the substituted phenyl is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure.

In some embodiments,

the numerical value of n is 0 or 1;

$R^1$ is substituted or unsubstituted phenyl, and the substituent is methyl or oxygen- and/or nitrogen-containing 5-6 membered aromatic heterocyclyl; and preferably, the methyl or oxygen- and/or nitrogen-containing 5-6 membered aromatic heterocyclyl is para-substituted relative to a parent nucleus structure;

$R^2$ is substituted or unsubstituted phenyl, and the substituent is methyl or oxygen- and/or nitrogen-containing 5-6 membered aromatic heterocyclyl; and preferably, the methyl or oxygen- and/or nitrogen-containing 5-6 membered aromatic heterocyclyl is para-substituted relative to a parent nucleus structure;

$R^3$ is substituted or unsubstituted $C_{1-3}$ alkyl, phenyl, or oxygen- and/or nitrogen-containing 5-6 membered aromatic heterocyclyl, and the substituent is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure; and

$R^4$ is hydrogen, substituted or unsubstituted $C_{1-3}$ alkyl, $C_{1-3}$ alkoxy, phenyl, or oxygen- and/or nitrogen-containing 5-6 membered aromatic heterocyclyl, and the substituent is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure.

In some embodiments,

the numerical value of n is 0 or 1;

$R^1$ is phenyl;

$R^2$ is phenyl;

$R^3$ is substituted or unsubstituted $C_{1-3}$ alkyl, phenyl, or oxygen- and/or nitrogen-containing 5-6 membered aromatic heterocyclyl, and the substituent is methyl, methoxy, halogen, or oxygen- and/or nitrogen-containing 5-6 membered aromatic heterocyclyl; and preferably, the methyl, methoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure; and

$R^4$ is hydrogen.

The term "parent nucleus structure" refers to the remaining main structure except an R group in the compound structure as shown in formula (I).

In some embodiments, the compound is selected from the following structures:

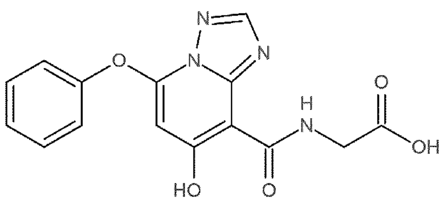 , 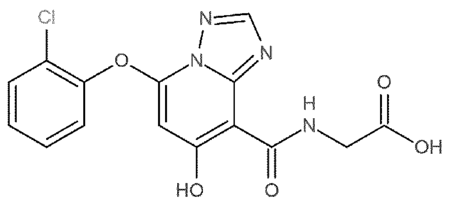 ,

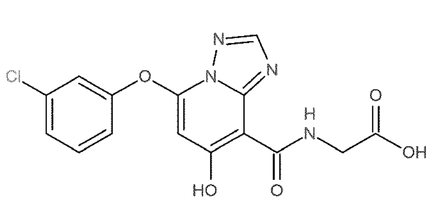 , 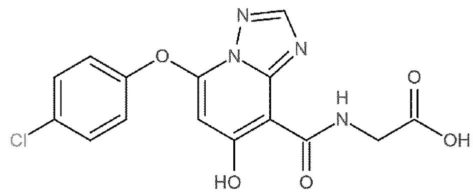 ,

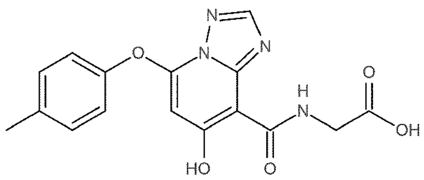 , 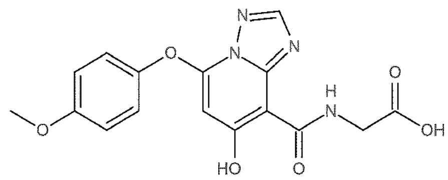 ,

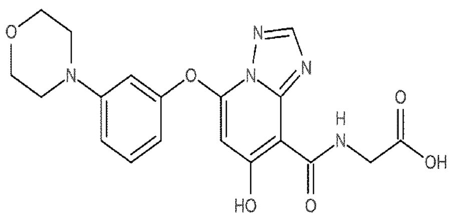 , 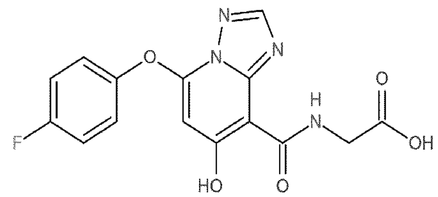 ,

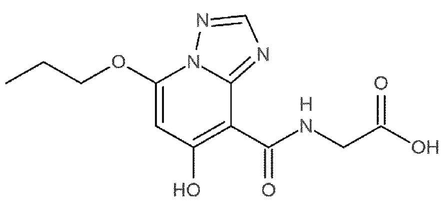 , 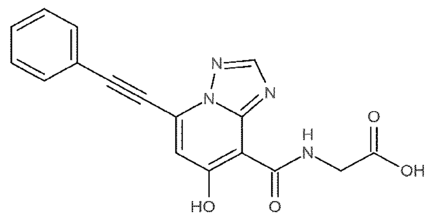 ,

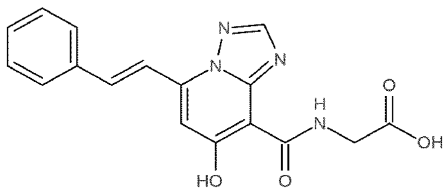 and 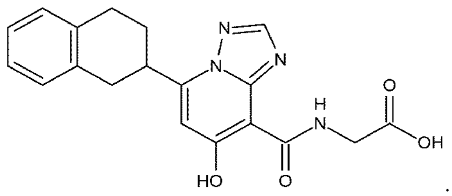 .

In some embodiments, the compound is selected from the following structures:

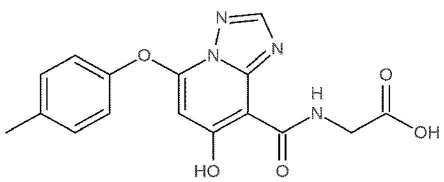 , 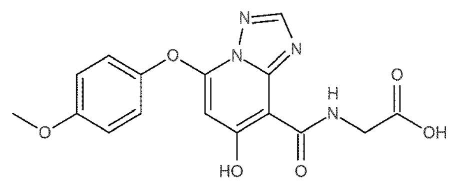 ,

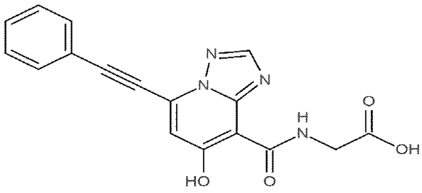,
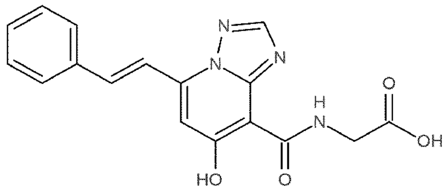

and

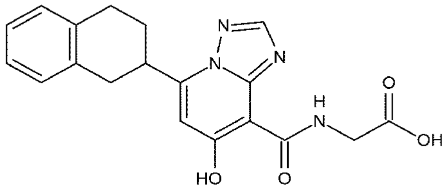.

In some embodiments, the compound is selected from the following structures:

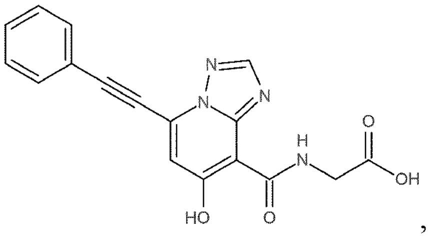,
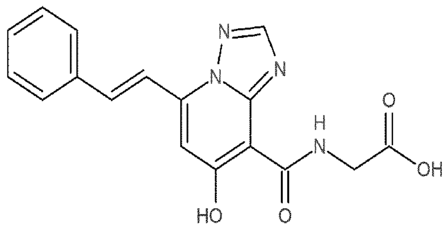

and

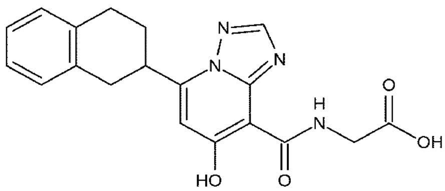.

In some embodiments, the compound is selected from the following structures:

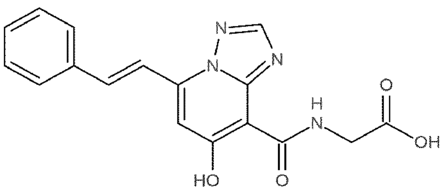 and 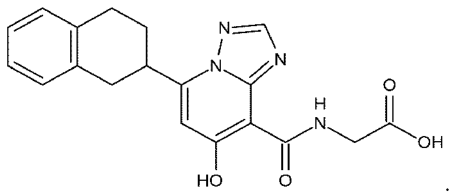.

In some embodiments, the compound is selected from the following structure:

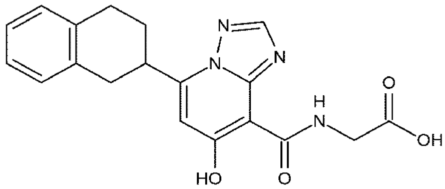.

In some embodiments, the present invention provides a compound as shown in formula (II) or a stereoisomer, geometric isomer, tautomer, nitrogen oxide, hydrate, solvate, pharmaceutically acceptable salt or prodrug thereof;

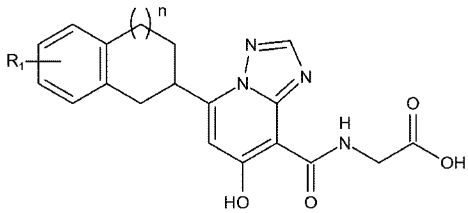

(II)

wherein,

a numerical value of n is an integer between 0 and 3; and

$R_1$ is monosubstituted or polysubstituted hydrogen or halogen, substituted or unsubstituted alkyl, cycloalkyl, alkoxy, aryl or heterocyclyl on an aromatic ring.

In some embodiments, the compound as shown in formula (II) or the stereoisomer, geometric isomer, tautomer, nitrogen oxide, hydrate, solvate, pharmaceutically acceptable salt or prodrug thereof comprises a heteroaromatic ring.

In some embodiments, a numercial value of n of the compound as shown in formula (II) or the stereoisomer, geometric isomer, tautomer, nitrogen oxide, hydrate, solvate, pharmaceutically acceptable salt or prodrug thereof is an integer between 0 and 2; and $R_1$ is hydrogen, halogen, substituted or unsubstituted alkyl, cycloalkyl, alkoxy, aryl or heterocyclyl.

In some embodiments, the numercial value of n of the compound as shown in formula (II) or the stereoisomer, geometric isomer, tautomer, nitrogen oxide, hydrate, solvate, pharmaceutically acceptable salt or prodrug thereof is 0 or 1; and $R_1$ is hydrogen, halogen, substituted or unsubstituted alkyl, cycloalkyl, alkoxy, aryl or heterocyclyl.

In some embodiments, the numercial value of n of the compound as shown in formula (II) or the stereoisomer, geometric isomer, tautomer, nitrogen oxide, hydrate, solvate, pharmaceutically acceptable salt or prodrug thereof is 0 or 1; $R_1$ is hydrogen, substituted or unsubstituted $C_{1-10}$ alkyl, $C_{1-10}$ alkoxy, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl.

In some embodiments, the numercial value of n of the compound as shown in formula (II) or the stereoisomer, geometric isomer, tautomer, nitrogen oxide, hydrate, solvate, pharmaceutically acceptable salt or prodrug thereof is 1; and $R_1$ is hydrogen.

In some embodiments, the compound as shown in formula (II) or the stereoisomer, geometric isomer, tautomer, nitrogen oxide, hydrate, solvate, pharmaceutically acceptable salt or prodrug thereof is selected from the following structure:

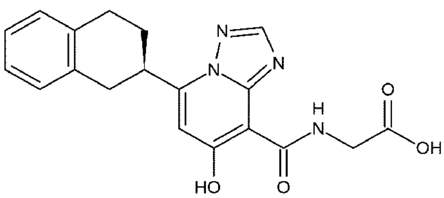 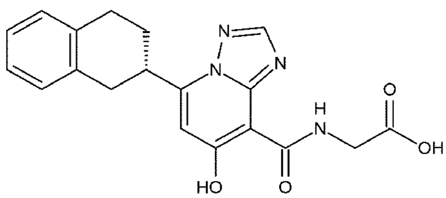

In some embodiments, a preparation method of the compound as shown in formula (II) is provided, which adopts the following synthetic route:

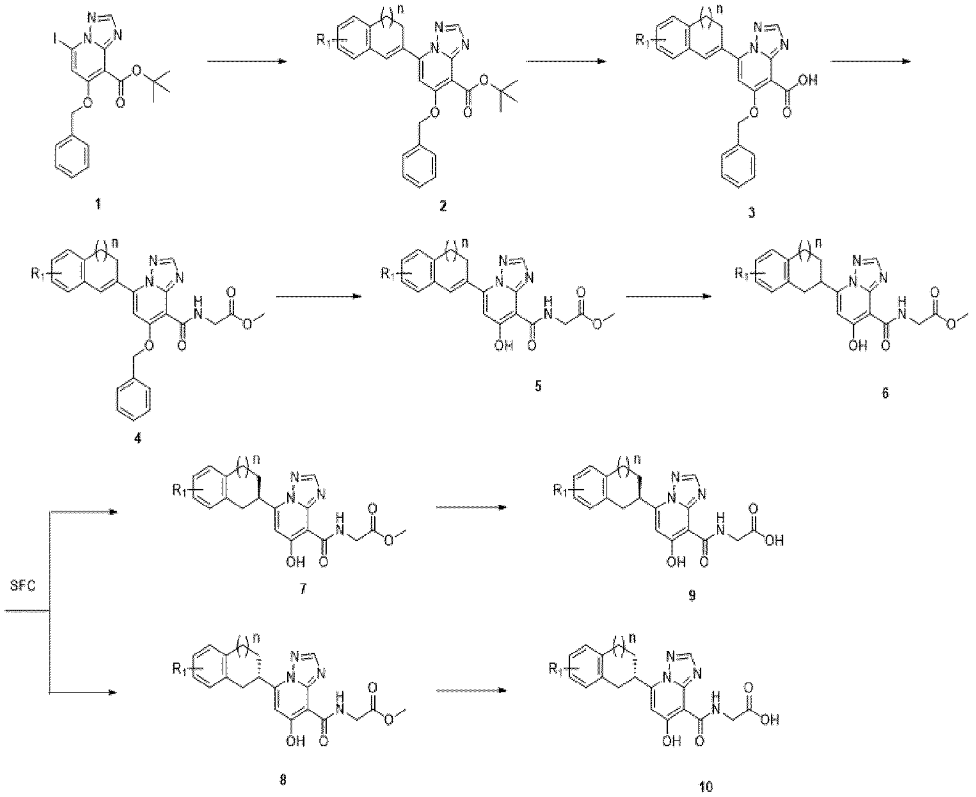


1 2 3 4 5 6 7 9 8 10

In some embodiments, a pharmaceutical composition is provided, which contains the compound as shown in formula (II) or the stereoisomer, geometric isomer, tautomer, nitrogen oxide, hydrate, solvate, pharmaceutically acceptable salt or prodrug thereof, and one or more of a medicinal carrier, a diluent and an excipient.

In some embodiments, a use of the compound as shown in formula (II) or the stereoisomer, geometric isomer, tautomer, nitrogen oxide, hydrate, solvate, pharmaceutically acceptable salt or prodrug thereof, or the pharmaceutical composition above in preparation of a drug for treating a prolyl hydroxylase domain inhibition-mediated disease is provided.

In some embodiments, the prolyl hydroxylase domain inhibition-mediated disease comprises anemia, ischemia and hypoxia.

In some embodiments, the prolyl hydroxylase domain inhibition-mediated disease is renal anemia.

In the present invention, FG-4592 is Roxadustat.

[(7-hydroxy-5-(1,2,3,4-tetrahydronaphthalene-2-yl)-[1,2,4]triazolo[1,5-a]pyridine-8-carbonyl]amino]acetic acid, in continuous chronic administration, shows an excellent pharmacological effect, is significant in data, can significantly increase a hemoglobin level and an EPO level in an SD rat, and has an overall effect significantly better than that of a positive drug Enarodustat.

Preferably, a purity of an intermediate prepared in each step in the embodiments of the present invention can be above 95%.

Unless otherwise specified, the term "alkyl" used herein comprises branched-chain and straight-chain saturated aliphatic hydrocarbon groups with a specified number of carbon atoms, comprising all isomers. Commonly used abbreviations of alkyl comprise, for example, methyl which may be represented by "Me" or $CH_3$, ethyl which may be represented by "Et" or $CH_2CH_3$, propyl which may be represented by "Pr" or $CH_2CH_2CH_3$, butyl which may be represented by "Bu" or $CH_2CH_2CH_2CH_3$, and the like. For example, "$C_{1-4}$ alkyl" (or "$C_1$-$C_4$ alkyl") refers to straight-chain or branched-chain alkyl with a specified number of carbon atoms, comprising all isomers. $C_{1-4}$ alkyl comprises n-, iso-, secondary- and tertiary-butyl, n- and iso-propyl, ethyl and methyl. The terms "$C_{1-10}$ alkyl" and the like have similar meanings.

The term "alkoxy" refers to straight-chain and branched-chain alkyl with a specified number of carbon atoms connected by an oxygen bridge.

The term "halogen" (or "halo") refers to fluorine, chlorine, bromine and iodine (or fluoro (F), chloro (Cl), bromo (Br) and iodo (I)).

The term "aryl" refers to an aromatic mono- and multi-carbon ring system, wherein each carbon ring is fused or interconnected by a single bond in the multi-carbon ring system. Aryl generally comprises phenyl, naphthyl and biphenyl.

The term "heterocyclic ring" refers to a ring structure composed of carbon atoms and non-carbon atoms, and the

non-carbon atoms in the ring are, for example, nitrogen, oxygen and sulfur. Heterocyclyl generally comprises pyridine, quinoline, tropane, phenothiazine, benzodiazepine, furan, pyrazolone and pyrimidine.

The term "heteroaromatic ring" refers to a 5 or 6-membered monocyclic aromatic ring or a 7 to 12-membered bicyclic ring, which is composed of carbon atoms and one or more heteroatoms selected from N, O and S. The heteroaromatic ring comprises, for example, pyridyl, pyrrolyl, pyrazinyl, pyrimidinyl, pyridazinyl, thienyl (or thiophenyl), thiazolyl, furyl, imidazolyl, pyrazolyl, triazolyl, tetrazolyl, oxazolyl, isozolyl, diazolyl, thiazolyl, isthiazolyl and thiadiazolyl, benzotriazolyl, indolyl, isoindolyl, indazolyl, dihydroindolyl, isodihydroindolyl, quinoxalinyl, quinazolinyl, cinnolinly, chromanly, ischromanly, tetrahydroquinolinly, quinolinyl, tetrahydroisoquinolinyl, isoquinolinyl, 2,3-dihydrobenzofuryl, 2,3-dihydrobenzo-1,4-dienyl, imidazo(2,1-b)(1,3)thiazole and benzo-1,3-m-dioxacyclopentenyl.

The aryl in the term "substituted aryl" is as defined above, and when the substituent of the substituted aryl is not specified, the substituent may be selected from the following groups, comprising but being not limited to: halogen, $C_1$-$C_{20}$ alkyl, $CF_3$, $NH_2$, N($C_1$-$C_6$ alkyl)$_2$, $NO_2$, oxo, CN, $N_3$, -OH, -O($C_1$-$C_6$ alkyl), $C_3$-$C_{10}$ cycloalkyl, $C_2$-$C_6$ alkenyl, $C_2$-$C_6$ alkynyl, ($C_0$-$C_6$ alkyl)S(O)$_{0-2}$-, aryl -S(O)$_{0-2}$-, ($C_0$-$C_6$ alkyl)S(O)$_{0-2}$($C_0$-$C_6$ alkyl)-, ($C_0$-$C_6$ alkyl)C(O)NH-, $H_2$N-C(NH)-, -O($C_1$-$C_6$ alkyl)$CF_3$, ($C_0$-$C_6$ alkyl)C(O)-, ($C_0$-$C_6$ alkyl)OC(O)-, ($C_0$-$C_6$ alkyl)$_2$NC(O)-$C_0$-$C_6$ alkyl)O($C_1$-$C_6$ alkyl)-, ($C_0$-$C_6$ alkyl)C(O)$_{1-2}$ ($C_0$-$C_6$ alkyl)-, ($C_0$-$C_6$ alkyl)OC(O)NH-, aryl, aralkyl, heteroaryl, heterocyclyl alkyl, halogen-aryl, halogen-aralkyl, halogen-heterocycle, halogen-heterocyclyl alkyl, cyano-aryl, cyano-aralkyl, cyano-heterocycle and cyano-heterocyclyl alkyl. The term "substituted phenyl" has a similar definition.

Unless explicitly stated, all ranges listed herein are inclusive. For example, "a numerical value of n is an integer between 0 and 2" means that n may be 0, 1 or 2.

The term "pharmaceutically acceptable salt" refers to a salt prepared with a pharmaceutically acceptable non-toxic base or acid. When the compound of the present invention is acidic, a corresponding salt may be easily prepared with an inorganic base or an organic base. The salt derived from the inorganic base comprises aluminum, ammonium, calcium, copper (copper and cuprous), iron, ferrous, lithium, magnesium, manganese (manganese and manganous), potassium, sodium, zinc and other salt. The salt is preferably ammonium, calcium, magnesium, potassium and sodium. The salt prepared with the organic base comprises primary amine, secondary amine and tertiary amine from natural and synthetic sources. A pharmaceutically acceptable organic nontoxic base capable of forming the salt comprises arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucosamine, glucosamine, histidine, hydrabamine, isopropylamine, dicyclohexylamine, lysine, methylglucosamine, morpholine, piperazine, piperidine, polyamine resin, procaine, purines, theobromine, triethylamine, trimethylamine, tripropylamine, tromethamine, and the like. When the compound of the present invention is alkaline, a corresponding salt may be easily prepared with an inorganic acid or an organic acid. The acid comprises, for example, acetic acid, benzenesulfonic acid, benzoic acid, camphorsulfonic acid, citric acid, ethanesulfonic acid, fumaric acid, gluconic acid, glutamic acid, hydrobromic acid, hydrochloric acid, hydroxyethanesulphonic acid, lactic acid, maleic acid, malic acid, mandelic acid, methanesulfonic acid, mucic acid, nitric acid, pamoic acid, pantothenic acid, phosphoric acid, succinic acid, sulfuric acid, tartaric acid, p-toluenesulfonic acid, and the like.

The term "solvate" refers to a variable stoichiometric complex formed by a solute (i.e., the compound of formula (I)) or a pharmaceutically acceptable salt thereof and a solvent that does not interfere with the biological activity of the solute. Examples of the solvent comprise, but are not limited to, water, ethanol and acetic acid. When the solvent is water, the solvate is called a hydrate. The hydrate comprises, but is not limited to, semi-, mono-, sesqui-, di- and tri-hydrates.

The term "prodrug" is a functional derivative of the compound of the present invention, which may be easily converted into a desired compound in vivo.

In another aspect, the present invention provides a preparation method of the compound above, which comprises the following four synthetic routes:

route 1:

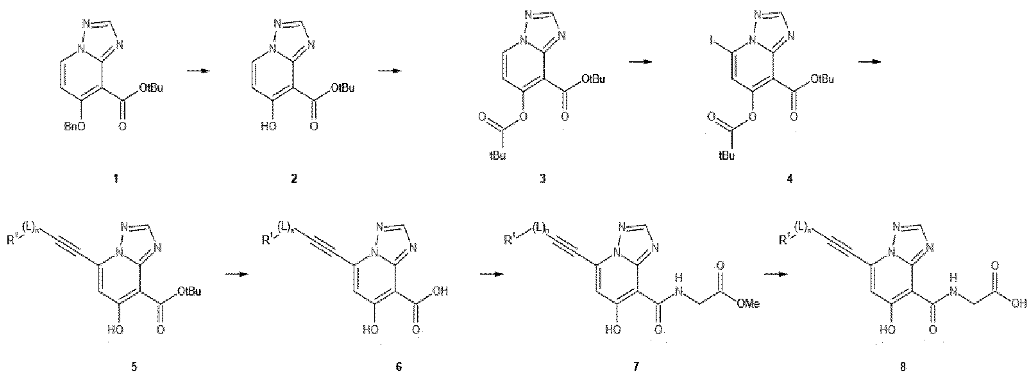

1 2 3 4

5 6 7 8

route 2:

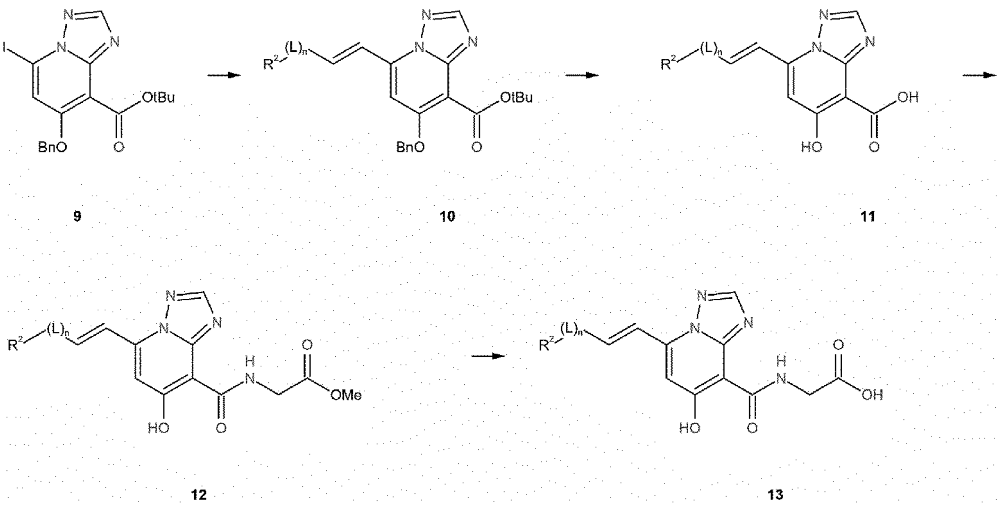

9 10 11

12 13

route 3:

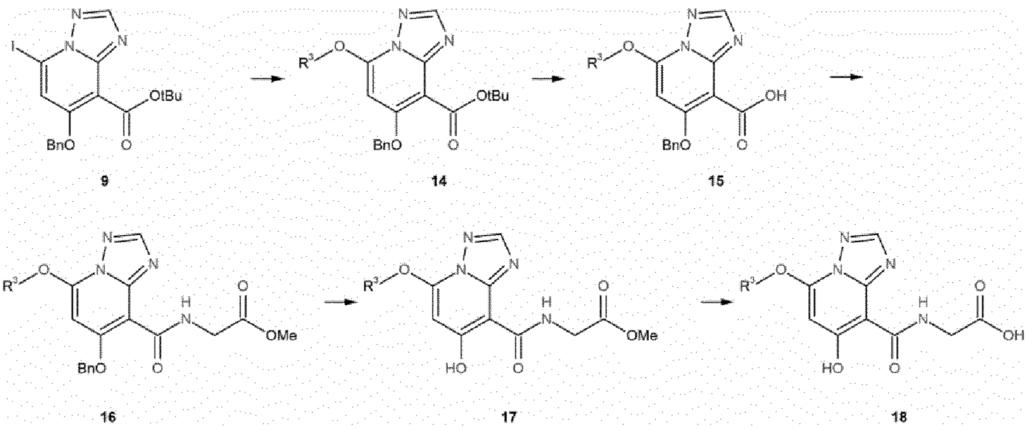

9 14 15

16 17 18

route 4:

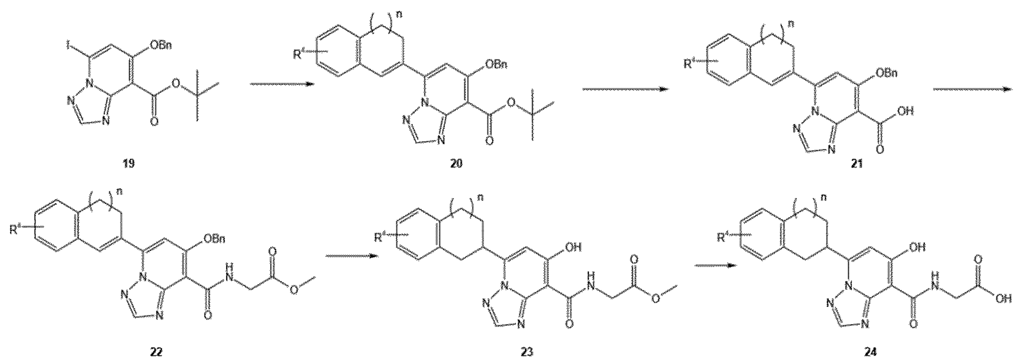

wherein, $R^1$, $R^2$, $R^3$ and $R^4$ have the same definitions as those mentioned above.

In yet another aspect, the present invention provides a pharmaceutical composition, which contains a compound as shown in formula (I) or a stereoisomer, geometric isomer, tautomer, nitrogen oxide, hydrate, solvate, pharmaceutically acceptable salt or prodrug thereof, and one or more of a medicinal carrier, a diluent and an excipient.

In the pharmaceutical composition, the term "composition" comprises a product containing an active ingredient and an inert ingredient (pharmaceutically acceptable excipient) constituting a carrier, and any product directly or indirectly obtained by the combination, complexation or aggregation of two or more ingredients, or the decomposition of one or more ingredients, or other types of reactions or interactions of one or more ingredients. Therefore, the pharmaceutical composition of the present invention comprises any composition prepared by mixing the compounds of formula (I) and/or formula (II), other active ingredient and the pharmaceutically acceptable excipient.

The pharmaceutical composition of the present invention contains the compounds (or the pharmaceutically acceptable salts or solvates thereof) as shown in formula (I) and/or formula (II) as active ingredients, a pharmaceutically acceptable carrier and other optional therapeutic ingredient or adjuvant. The pharmaceutical composition comprises compositions suitable for oral, rectal, local and parenteral (comprising subcutaneous, intramuscular and intravenous) administration, but the most suitable route in any particular case depends on the specific subject and the nature and severity of disease to which the active ingredient is administered. The pharmaceutical composition may be prepared by any method commonly known in the field of pharmacy.

The active ingredient may be administered orally in a solid dosage form or a liquid dosage form, wherein the solid dosage form comprises, for example, a capsule, a tablet, a pastille, a sugar pastille, a granule and a powder, and the liquid dosage form comprises, for example, an elixir, a syrup, an emulsion, a dispersion and a suspension. The active ingredient may also be administered parenterally in a sterile liquid dosage form such as a dispersion, a suspension or a solution. Other dosage forms that may be used for the administration of the active ingredient comprise an ointment, a cream, a drop, a transdermal patch or a powder for local administration; an ophthalmic solution or suspension for ophthalmic administration, i.e., an eye drop; and a spray or a powder composition for inhalation or intranasal administration, or a cream, an ointment, a spray or a suppository for rectal or vaginal administration. A gelatin capsule contains the active ingredient and a powdery carrier, such as lactose, starch, a cellulose derivative, magnesium stearate and stearic acid. A similar diluent may be used to prepare a compressed tablet. The tablet and the capsule may both be prepared into a sustained-release product to provide sustained release of drug within hours. The compressed tablet may be sugar-coated or film-coated to mask any unpleasant taste and protect the tablet from air, or may be enteric-coated for selective disintegration in the gastrointestinal tract. The liquid dosage form for oral administration may contain a coloring agent and a flavoring agent to increase the acceptability of patients. Generally speaking, water, suitable oil, saline, dextrose (glucose) aqueous solution, and related sugar solutions and glycols such as propylene glycol or polyethylene glycol are suitable carriers for a parenteral solution. The solution for parenteral administration preferably comprises a water-soluble salt containing the active ingredient, a suitable stabilizer and a buffer substance used as needed. An antioxidant, such as sodium bisulfite, sodium sulfite or ascorbic acid used alone or in combination, is the suitable stabilizer. Citric acid and a salt thereof, and sodium EDTA may also be used. In addition, the parenteral solution may also contain a preservative, such as benzalkonium chloride, methylparaben or propylparaben and chlorobutanol. For inhalation administration, the compound of the present invention may be conveniently delivered in a form of spray from a pressurized package or a sprayer. The compound may also be delivered in a form of formulated powder, and the powder composition may be inhaled with the help of a blown powder inhaler device. A preferred delivery system for inhalation is a metered-dose inhalation (MDI) aerosol, which can be prepared into suspensions or solutions of the compounds of formula (I) and/or formula (II) in a suitable propellant, such as fluorocarbon or hydrocarbon. For ophthalmic administration, ophthalmic preparations may be prepared from solutions or suspensions of the compounds of formula (I) and/or formula (II) with appropriate weight

percentages in a suitable ophthalmic carrier, so as to keep the compounds in contact with surfaces of eyes for a sufficient time to allow the compounds to penetrate into cornea and internal areas of the eyes.

Useful pharmaceutical dosage forms for administering the compound of the present invention comprise, but are not limited to, hard and soft gelatin capsules, a tablet, a parenteral injection and an oral suspension.

When the compound of the present invention is administered step by step or in combination with other therapeutic agent, the same dosage form mentioned above may be used. When drugs are administered in physical combination, the dosage form and the route of administration should be selected according to the compatibility of the combined drugs. The compound of the present invention may be administered as the only active ingredient or in combination with a second active ingredient, and the second active ingredient comprises an active ingredient known to be useful for increasing a level of erythropoietin in patients.

In yet another aspect, the present invention provides a use of the compound as shown in formula (I) or (II) or the stereoisomer, geometric isomer, tautomer, nitrogen oxide, hydrate, solvate, pharmaceutically acceptable salt or prodrug thereof, or the pharmaceutical composition above in preparation of a drug for treating a prolyl hydroxylase domain inhibition-mediated disease.

In some embodiments, the prolyl hydroxylase domain inhibition-mediated disease comprises anemia, ischemia and hypoxia.

In some embodiments, the anemia is renal anemia.

The present invention has the beneficial effects as follows:

a new compound is provided, which may be used as a prolyl hydroxylase domain inhibor, has higher prolyl hydroxylase domain inhibitory activity and EPO inducing activity, effectively treats and prevents HIF-related and/or EPO-related diseases, and provides a new thought for anemia, ischemia, hypoxia and other diseases.

## BRIEF DESCRIPTION OF THE DRAWINGS

FIG. 1 is a microphotograph of a single crystal sample of a chiral isomer A of methyl acetate;
FIG. 2 is an asymmetric unit diagram of a crystal structure of the chiral isomer A of methyl acetate;
FIG. 3 is a unit cell diagram of the crystal structure of the chiral isomer A of methyl acetate;
FIG. 4 is a stacking diagram of the crystal structure of the chiral isomer A of methyl acetate;
FIG. 5 is a comparison diagram of XRPD of the single crystal sample of the chiral isomer A of methyl acetate and XRPD obtained by calculation;
FIG. 6 is a diagram of a chemical structure of the chiral isomer A of methyl acetate;
FIG. 7 is a microphotograph of a single crystal sample of a chiral isomer B of methyl acetate;
FIG. 8 is an asymmetric unit diagram of a crystal structure of the chiral isomer B of methyl acetate;
FIG. 9 is a unit cell diagram of the crystal structure of the chiral isomer B of methyl acetate;
FIG. 10 is a stacking diagram of the crystal structure of the chiral isomer B of methyl acetate;
FIG. 11 is a comparison diagram of XRPD of the single crystal sample of the chiral isomer B of methyl acetate and XRPD obtained by calculation; and
FIG. 12 is a diagram of a chemical structure of the chiral isomer B of methyl acetate.

## DETAILED DESCRIPTION

The following non-restrictive embodiments may help those of ordinary skills in the art to understand the present invention more comprehensively, but the embodiments are not intended to limit the present invention in any way. The following contents are only exemplary explanations of the scope of protection claimed in the present application, and those skilled in the art can make various changes and modifications to the invention of the present application according to the disclosed contents, which should also belong to the scope of protection claimed in the present application.

The present invention is further described hereinafter with reference to specific embodiments. Unless otherwise specified, various chemical reagents used in the embodiments of the present invention are all obtained through conventional commercial channels.

### Embodiment 1

Preparation of (7-hydroxy-5-(phenoxy)-[1,2,4]triazolo[1,5-a]pyridine-8-carbonyl)amino]acetic acid

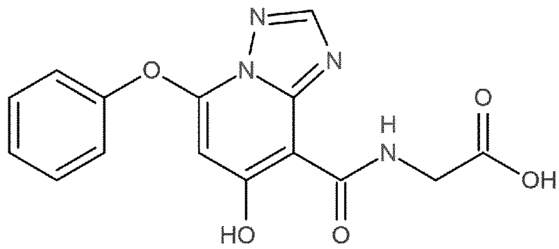

Step 1 Preparation of tert-butyl 5-(phenoxy)-7-(benzyloxy)-[1,2,4]triazolo[1,5-a]pyridine-8-carboxylate

Tert-butyl 5-iodo-7-(benzyloxy)-[1,2,4]triazolo[1,5-a]pyridine-8-carboxylate (1.0 g), phenol (310 mg), 2-pyridinecarboxylic acid (55 mg), cuprous iodide (42 mg), potassium phosphate (942 mg) and dimethyl sulfoxide (5.0 mL) were sequentially added into a reaction bottle, and nitrogen was replaced thrice. The mixture was heated to 65°C and reacted overnight until complete reaction of raw materials was monitored by TLC. The product was cooled to room temperature, extracted with ethyl acetate, and washed with a saturated salt solution. An organic phase was separated, dried with anhydrous sodium sulfate, concentrated under a reduced pressure, and subjected to column chromatography to obtain 450 mg of white solid.
MS m/z (ESI)$[M+H]^+$: 418.30

Step 2 Preparation of 5-(phenoxy)-7-(benzyloxy)-[1,2,4]triazolo[1,5-a]pyridine-8-carboxylic acid

The tert-butyl 5-(phenoxy)-7-(benzyloxy)-[1,2,4]triazolo[1,5-a]pyridine-8-carboxylate (450 mg) was dissolved in a mixed solution of toluene and tetrahydrofuran (10 mL) in a volume ratio of 1: 1, and then added with methanesulfonic acid (0.5 mL) and stirred to react at a temperature kept between 60°C and 70°C. After complete reaction of raw materials was monitored by TLC, the reaction was stopped. The product was cooled to room temperature, added with a small amount of ethyl acetate, and filtered to obtain 440 mg of white solid.
MS m/z (ESI) $[M+H]^+$: 362.22

Step 3 Preparation of [(7-(benzyloxy)-5-(phenoxy)-[1,2,4]triazolo[1,5-a]pyridine-8-carbonyl)amino]methyl acetate

The 5-(phenoxy)-7-(benzyloxy)-[1,2,4]triazolo[1,5-a]pyridine-8-carboxylic acid (440 mg), EDCI (280 mg), HoBt (197 mg), N,N-dimethylformamide (5.0) and triethylamine (247 mg) were sequentially added into a reaction bottle, and stirred at room temperature for 10 minutes, and then, glycine methyl ester hydrochloride (306 mg) was added into the system, heated to 60°C, and reacted for 1.5 hours. After stopping the reaction, the product was cooled to room temperature, added with a saturated sodium bicarbonate aqueous solution for a quenching reaction, and filtered by suction to obtain 300 mg of yellow solid.
MS m/z (ESI) $[M+H]^+$: 433.31

Step 4 Preparation of [(7-hydroxy-5-(phenoxy)-[1,2,4]triazolo[1,5-a]pyridine-8-carbonyl)amino]methyl acetate

The [(7-(benzyloxy)-5-(phenoxy)-[1,2,4]triazolo[1,5-a]pyridine-8-carbonyl)amino]methyl acetate (300 mg) and palladium carbon (30 mg) were dissolved in a mixed solvent (4.0 mL) of methanol and tetrahydrofuran in a volume ratio of 1: 1, and stirred at room temperature in a hydrogen environment. After complete reaction of raw materials was monitored by TLC, the reaction was stopped. The solvent was removed by concentration under a reduced pressure, and column chromatography separation was carried out to obtain 170 mg of product.
MS m/z (ESI) $[M+H]^+$: 343.22

Step 5 Preparation of [(7-hydroxy-5-(phenoxy)-[1,2,4]triazolo[1,5-a]pyridine-8-carbonyl)amino]acetic acid

The [(7-hydroxy-5-(phenoxy)-[1,2,4]triazolo[1,5-a]pyridine-8-carbonyl)amino]methyl acetate (170 mg) was dissolved in methanol (2.0 mL), then added with 30% sodium hydroxide aqueous solution (1.0 mL), and stirred at room temperature until complete reaction of raw materials was monitored by TLC, and the mixture was added with dilute hydrochloric acid to adjust pH to be 1 to 2, and filtered to obtain 150 mg of product.
$^1$H NMR (400 MHz, d-DMSO) δ: 14.52 (s, 1H), 12.95 (brs, 1H), 9.71 (s, 1H), 8.59 (s, 1H), 7.59-7.53 (m, 2H), 7.44-7.41 (m, 3H), 5.83 (s, 1H), 4.20 (d, J = 5.6 Hz, 2H)
MS m/z (ESI): 329.09

**Embodiment 2**

Preparation of (5-(2-chlorophenoxy)-7-hydroxy-[1,2,4]triazolyl[1,5-a]pyridine-8-carbonyl)glycine

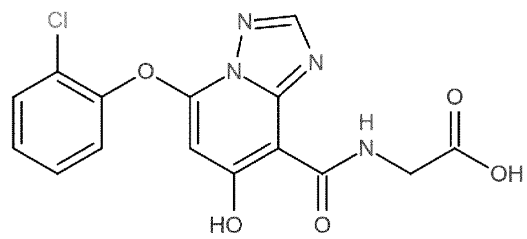

According to the method of Embodiment 1, 428.1 mg of 2-chlorophenol was used instead of phenol to obtain 108.9 mg of product.

$^1$H NMR (400 MHz, d-DMSO) δ: 14.58 (s, 1H), 12.97 (brs, 1H), 9.71 (t, J = 5.6 Hz, 1H), 8.63 (s, 1H), 7.75 (d, J = 7.6 Hz, 1H), 7.61 (d, J = 8.0 Hz, 1H), 7.54 (t, J = 8.0 Hz, 1H), 7.47 (t, J = 7.2 Hz, 1H), 5.89 (s, 1H), 4.21 (d, J = 5.6 Hz, 2H)
MS m/z (ESI) $[M+H]^+$: 363.17

**Embodiment 3**

Preparation of (5-(3-chlorophenoxy)-7-hydroxy-[1,2,4]triazolyl[1,5-a]pyridine-8-carbonyl)glycine

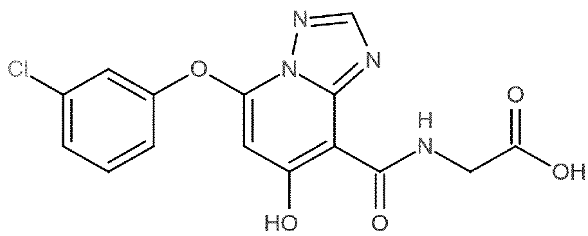

According to the method of Embodiment 1, 428.1 mg of 3-chlorophenol was used instead of phenol to obtain 201.6 mg of product.

$^1$H NMR (400 MHz, d-DMSO)14.54 (s, 1H), 12.97 (brs, 1H), 9.72 (s, 1H), 8.58 (s, 1H), 7.62 (s, 1H), 7.57 (t, J = 8.4 Hz, 1H), 7.47 (d, J = 8.4 Hz, 1H), 7.41 (d, J = 8.4 Hz, 1H), 6.10 (s, 1H), 4.21 (d, J = 5.6 Hz, 2H)
MS m/z (ESI) $[M+H]^+$: 363.14

**Embodiment 4**

Preparation of (5-(4-chlorophenoxy)-7-hydroxy-[1,2,4]triazolyl[1,5-a]pyridine-8-carbonyl)glycine

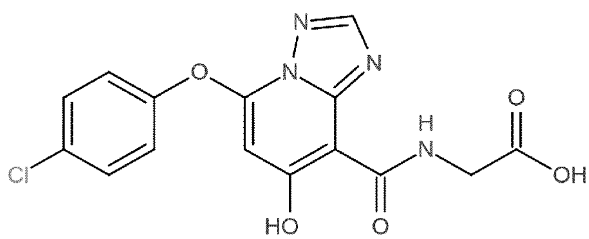

According to the method of Embodiment 1, 428.1 mg of 4-chlorophenol was used instead of phenol to obtain 182.1 mg of product.

$^1$H NMR (400 MHz, d-DMSO) δ: 14.54 (s, 1H), 12.96 (s, 1H), 9.72 (t, J = 5.6 Hz, 1H), 8.60 (s, 1H), 7.62 (d, J = 8.4 Hz, 2H), 7.48 (d, J = 8.4 Hz, 2H), 6.02 (s, 1H), 4.21 (d, J = 5.6 Hz, 2H)
MS m/z (ESI) $[M+H]^+$: 363.14

**Embodiment 5**

Preparation of (5-(p-tolyloxy)-7-hydroxy-[1,2,4]triazolyl[1,5-a]pyridine-8-carbonyl)glycine

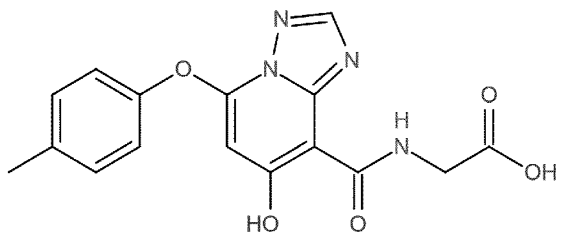

According to the method of Embodiment 1, 356.9 mg of 4-methylphenol was used instead of phenol to obtain 87.7 mg of product.

$^1$H NMR (400 MHz, d-DMSO) δ: 14.51 (s, 1H), 12.95 (s, 1H), 9.69 (t, J = 5.6 Hz, 1H), 8.59 (s, 1H), 7.37 (d, J = 8.4 Hz, 2H), 7.31 (d, J = 8.4 Hz, 2H), 5.77 (s, 1H), 4.20 (d, J = 5.6 Hz, 2H), 2.37 (s, 3H)
MS m/z (ESI) $[M+H]^+$: 343.16

**Embodiment 6**

Preparation of (5-(p-methoxyphenoxy)-7-hydroxy-[1,2,4]triazolyl[1,5-a]pyridine-8-carbonyl)glycine

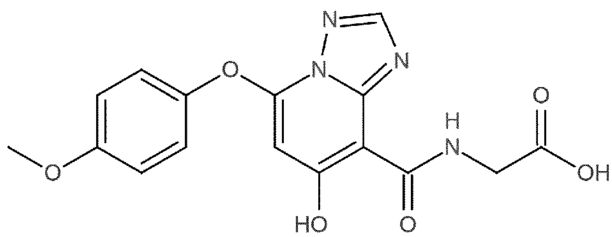

According to the method of Embodiment 1, 409.2 mg of 4-methoxyphenol was used instead of phenol to obtain 223.7 mg of product.

$^1$H NMR (400 MHz, d-DMSO) δ: 14.51 (s, 1H), 12.96 (s, 1H), 9.70 (t, J = 5.6 Hz, 1H), 8.60 (s, 1H), 7.39 (d, J = 8.8 Hz, 2H), 7.11(d, J = 8.8 Hz, 2H), 5.72 (s, 1H), 4.21 (d, J = 5.6 Hz, 2H), 3.82 (s, 3H)
MS m/z (ESI) $[M+H]^+$: 359.22

**Embodiment 7**

Preparation of (5-(3-morpholinphenoxy)-7-hydroxy-[1,2,4]triazolyl[1,5-a]pyridine-8-carbonyl)glycine

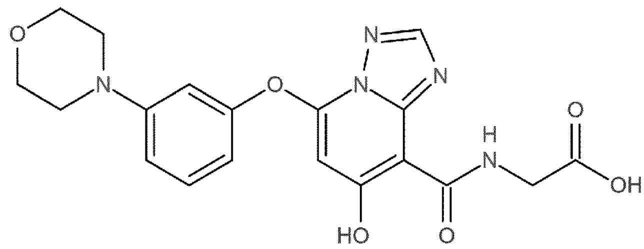

According to the method of Embodiment 1, 590 mg of 3-morpholine phenol was used instead of phenol to obtain 110.7 mg of product.

$^1$H NMR (400 MHz, d-DMSO) δ: 14.51 (s, 1H), 12.95 (brs, 1H), 9.70 (t, J = 5.2 Hz, 1H), 8.59 (s, 1H), 7.39 (d, J = 8.0 Hz, 2H), 6.99 (d, J = 12.0 Hz, 2H), 6.80 (d, J = 7.6 Hz, 2H), 5.82 (s, 1H), 4.21 (d, J = 5.6 Hz, 2H), 3.73 (t, J = 4.8 Hz, 4H), 3.18 (t, J = 4.8 Hz, 4H)
MS m/z (ESI) $[M+H]^+$: 414.23

**Embodiment 8**

Preparation of (5-(4-fluorophenoxy)-7-hydroxy-[1,2,4]triazolyl[1,5-a]pyridine-8-carbonyl)glycine

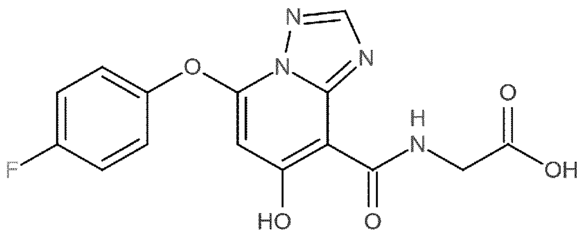

According to the method of Embodiment 1, 369.6 mg of 4-fluorophenol was used instead of phenol to obtain 239.3 mg of product.

$^1$H NMR (400 MHz, d-DMSO) 14.53 (s, 1H), 12.98 (brs, 1H), 9.71 (s, 1H), 8.60 (s, 1H), 7.53-7.49 (m, 2H), 7.43-7.38 (m, 2H), 5.86 (s, 1H), 4.20 (d, J = 5.6 Hz, 2H)
MS m/z (ESI) $[M+H]^+$: 347.14

**Embodiment 9**

Preparation of (5-(propoxy)-7-hydroxy-[1,2,4]triazolyl[1,5-a]pyridine-8-carbonyl)glycine

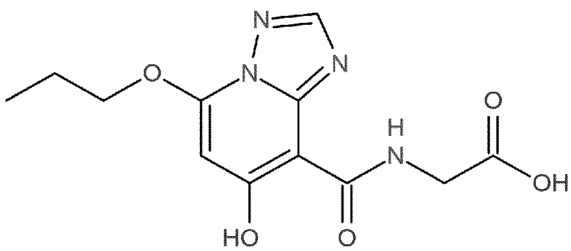

According to the method of Embodiment 1, 2 ml of n-propanol was used instead of phenol to obtain 20.1 mg of product.

$^1$H NMR (400 MHz, d-DMSO) 14.51 (s, 1H), 12.92 (brs, 1H), 9.65 (t, J = 5.6 Hz, 1H), 8.47 (s, 1H), 6.44 (s, 1H), 4.39 (t, J = 6.4 Hz, 2H), 4.18 (d, J = 5.6 Hz, 2H), 1.91-1.82 (m, 2H), 1.03 (t, J = 7.2 Hz, 3H)

MS m/z (ESI) [M+H]+: 295.12

**Embodiment 10**

Preparation of [(7-hydroxy-5-(phenylethynyl)-[1,2,4]triazolo[1,5-a]pyridine-8-carbonyl)amino]acetic acid

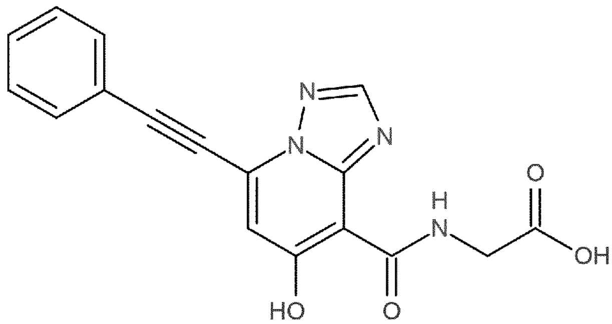

Step 1 Preparation of tert-butyl 7-hydroxy-[1,2,4]triazolo[1,5-a]pyridine-8-carboxylate

In a reaction bottle, tert-butyl 7-(benzyloxy)-[1,2,4]triazolo[1,5-a]pyridine-8-carboxylate (15 g) was dissolved in a mixed solvent (25 mL/50 mL) of methanol and tetrahydrofuran, and stirred at room temperature in a hydrogen environment. After complete reaction of raw materials was monitored by TLC, the reaction was stopped. Palladium carbon in the solution was removed by filtration, and then the filtrate was concentrated under a reduced pressure to obtain 11.7 g of crude product.
MS m/z (ESI): [M+H]+: 236.24

Step 2 Preparation of tert-butyl 7-(pivaloyloxy)-[1,2,4]triazolo[1,5-a]pyridine-8-carboxylate

The tert-butyl 7-hydroxy-[1,2,4]triazolo[1,5-a]pyridine-8-carboxylate (10.7 g) was placed in a reaction bottle, added with tetrahydrofuran (65 mL), and then sequentially added with triethylamine (8.0 mL) and pivaloyl chloride (9.1 g). Nitrogen was replaced thrice, and the reaction bottle was sealed. The reaction mixture was heated to 60°C, and stirred overnight. After complete reaction of raw materials was monitored by TLC, the reaction was stopped. After the reaction solution was cooled to room temperature, the reaction solution was extracted with ethyl acetate, sequentially washed with a saturated sodium bicarbonate solution, a saturated ammonium chloride solution and a saturated salt solution, and then concentrated under a reduced pressure to obtain 12.0 g of yellow solid.
MS m/z (ESI): [M+H]+: 320.36

Step 3 Preparation of tert-butyl 5-iodo-7-(pivaloyloxy)-[1,2,4]triazolo[1,5-a]pyridine-8-carboxylate

The tert-butyl 7-(pivaloyloxy)-[1,2,4]triazolo[1,5-a]pyridine-8-carboxylate (10.0 g) was dissolved in a tetrahydrofuran (50 mL) solution, and then added with elemental iodine (8.75 g) to react at a temperature kept below -60°C. Lithium hexamethyldisilicate (62.7 mL, 1.0 M) was added in batches 5 minutes later, and stirred until complete reaction of raw materials was monitored by TLC. Subsequently, the product was added with an ethyl acetate hydrochloride solution for a quenching reaction, extracted with ethyl acetate, and washed with a sodium sulfite aqueous solution. An organic phase was separated, and sequentially washed with a saturated ammonium chloride aqueous solution, a saturated sodium bicarbonate aqueous solution and a saturated salt solution. An organic phase was separated and concentrated under a reduced pressure, and a crude product was subjected to column chromatography to obtain 5.9 g of product.

$^1$H NMR (400 MHz, $CDCl_3$) δ: 8.36 (d, J = 5.2 Hz, 1H), 7.45 (d, J = 5.2 Hz, 1H), 2.31 (s, 6H)
MS m/z (ESI): [M+H]+: 446.26

Step 4 Preparation of tert-butyl 5-(phenylethynyl)-7-hydroxy-[1,2,4]triazolo[1,5-a]pyridine-8-carboxylate

The tert-butyl 5-iodo-7-(pivaloyloxy)-[1,2,4]triazolo[1,5-a]pyridine-8-carboxylate (5.9 g), cuprous iodide (70 mg), Pd(dppf)$Cl_2$ (285 mg), tetrahydrofuran (30 mL), triethylamine (6.6 g) and phenylacetylene (2.1 g) were sequentially added

into a reaction bottle. Nitrogen was replaced thrice, the mixture was heated to 50°C and reacted until complete reaction of raw materials was monitored by TLC, and the mixture was added with ammonia water for a quenching reaction, and extracted with ethyl acetate. An organic phase was separated. An ammonia water layer was washed with hydrochloric acid and extracted with ethyl acetate, and then the organic phases were combined, concentrated under a reduced pressure, and purified by column chromatography to obtain 1.9 g of red solid.
MS m/z (ESI): $[M+H]^+$: 336.36

Step 5 Preparation of 5-(phenylethynyl)-7-hydroxy-[1,2,4]triazolo[1,5-a]pyridine-8-carboxylic acid

The tert-butyl 5-(phenylethynyl)-7-hydroxy-[1,2,4]triazolo[1,5-a]pyridine-8-carboxylate (1.8 g) was dissolved in a mixed solution of toluene and ethyl acetate (24 mL) in a volume ratio of 2: 1, and then added with methanesulfonic acid (2.1 g) and stirred to react at a temperature kept between 60°C and 70°C. After complete reaction of raw materials was monitored by TLC, the reaction was stopped. The reaction solution was cooled to room temperature and then added with ethyl acetate (10 mL), and a large number of solids were precipitated and filtered to obtain green solids. Subsequently, the solids were dissolved with a small amount of N,N-dimethylacetamide, ethyl acetate was added into the system, and solids were precipitated and filtered to obtain 1.0 g of crude product.
MS m/z (ESI): $[M+H]^+$: 280.26

Step 6 Preparation of [(7-hydroxy-5-(phenylethynyl)-[1,2,4]triazolo[1,5-a]pyridine-8-carbonyl)amino]methyl acetate

In a reaction bottle A, the 5-(phenylethynyl)-7-hydroxy-[1,2,4]triazolo[1,5-a]pyridine-8-carboxylic acid (0.8 g) was dissolved in dichloromethane (20 mL), dropwise added with oxalyl chloride (0.76 g), and stirred at room temperature. In a reaction bottle B, methyl glycinate hydrochloride (0.68 g), dichloromethane (20 mL) and triethylamine (3.0 mL) were sequentially added, and stirred at 0°C. After complete reaction of raw materials in the reaction bottle A was monitored by TLC, the stirring in the reaction bottle A was stopped, the material in the reaction bottle A was added into the reaction bottle B, and stirred at 0°C until complete reaction of raw materials was monitored by TLC, and the mixture was added with water for a quenching reaction, and extracted with dichloromethane. An organic phase was sequentially washed with a saturated ammonium chloride aqueous solution and a saturated sodium bicarbonate aqueous solution, and the combined organic phase was separated, concentrated under a reduced pressure, and separated by column chromatography to obtain 0.8 g of yellow solid.
MS m/z (ESI): $[M+H]^+$: 351.33

Step 7 Preparation of [(7-hydroxy-5-(phenylethynyl)-[1,2,4]triazolo[1,5-a]pyridine-8-carbonyl)amino]acetic acid

The [(7-hydroxy-5-phenylethynyl[1,2,4]triazolo[1,5-a]pyridine-8-carbonyl)amino]methyl acetate (0.8 g) was dissolved in ethanol (10 mL), then added with a sodium hydroxide aqueous solution (18 mL, 2.0 M), and stirred at room temperature until complete reaction of raw materials was monitored by TLC, and the mixture was added with dilute hydrochloric acid to adjust pH to be 4 to 5, and filtered to obtain 0.42 g of pale yellow solid.

$^1H$ NMR (400 MHz, d-DMSO) δ: 14.33 (s, 1H), 13.04 (s, 1H), 9.90 (s, 1H), 8.64 (s, 1H), 7.74-7.72 (m, 2H), 7.61-7.53 (m, 3H), 7.37 (s, 1H), 4.24 (d, J = 5.6 Hz, 2H)
MS m/z (ESI): $[M+H]^+$: 337.31

**Embodiment 11**

Preparation of [(7-hydroxy-5-(styryl)-[1,2,4]triazolo[1,5-a]pyridine-8-carbonyl)amino]acetic acid

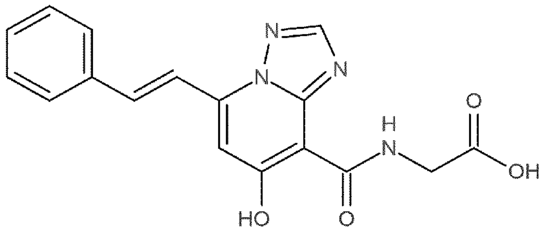

Step 1 Preparation of tert-butyl 5-(styryl)-7-(benzyloxy)-[1,2,4]triazolo[1,5-a]pyridine-8-carboxylate

Tert-butyl 5-iodo-7-(benzyloxy)-[1,2,4]triazolo[1,5-a]pyridine-8-carboxylate (500 mg), $Pd(PPh_3)_2Cl_2$ (70.2 mg), sodium carbonate (360 mg) and styrene boric acid (200 mg) were dissolved in a mixed solvent (30 mL) of ethylene glycol dimethyl ether and de-aerated water in a volume ratio of 1: 1. Nitrogen was replaced thrice. Subsequently, the mixture was stirred at 80°C, and after complete reaction of raw materials was monitored by TLC, the heating was stopped. The reaction solution was cooled to room temperature, and then extracted with ethyl acetate. An organic phase was washed with a saturated salt solution. The organic phase was separated, concentrated under a reduced pressure, and separated by column chromatography to obtain 340 mg of crude product.

Step 2 Preparation of 5-(styryl)-7-hydroxy-[1,2,4]triazolo[1,5-a]pyridine-8-carboxylic acid

The tert-butyl 5-(styryl)-7-(benzyloxy)-[1,2,4]triazolo[1,5-a]pyridine-8-carboxylate (290 mg) was dissolved in a dichloromethane (10 mL) solution, and cooled to -65°C, and then a boron tribromide dichloromethane solution (3.4) was added into the reaction system and stirred. After complete reaction of raw materials was monitored by TLC, the reaction was stopped. The solution was removed by concentration under a reduced pressure, and then the residue was added into a mixed solvent of water (10 mL) and methanol (3.0 mL), stirred at room temperature for 1 hour, and filtered to obtain 250 mg of solid.
MS m/z (ESI): $[M+H]^+$: 282.04

Step 3 Preparation of [(7-hydroxy-5-(styryl)-[1,2,4]triazolo[1,5-a]pyridine-8-carbonyl)amino]methyl acetate

The 5-(styryl)-7-hydroxy-[1,2,4]triazolo[1,5-a]pyridine-8-carboxylic acid (143 mg), N,N-dimethylformamide (10 mL), DIPEA (329 mg), PyBOP (395 mg) and methyl glycinate hydrochloride (178 mg) were sequentially added into a reaction bottle, stirred overnight at room temperature, and filtered to collect a solid, so as to obtain 50 mg of crude product.
MS m/z (ESI): $[M+H]^+$: 353.28

Step 4 Preparation of [(7-hydroxy-5-(styryl)-[1,2,4]triazolo[1,5-a]pyridine-8-carbonyl)amino]acetic acid

The [(7-hydroxy-5-(styryl)-[1,2,4]triazolo[1,5-a]pyridine-8-carbonyl)amino]methyl acetate (50 mg) was dissolved in methanol (2.0 mL), then added with 30% sodium hydroxide aqueous solution (1.0 mL), and stirred at room temperature until complete reaction of raw materials was monitored by TLC, and the mixture was added with dilute hydrochloric acid to adjust pH to be 1 to 2, and filtered to obtain 10 mg of product.

$^1$H NMR (400 MHz, d-DMSO) δ: 14.26 (s, 1H), 12.98 (s, 1H), 9.90 (t, J = 16.4 Hz, 1H), 8.63 (s, 1H), 8.22 (d, J = 16.4 Hz, 1H), 7.76-7.71 (m, 3H), 7.50-7.40 (m, 4H), 4.23 (d, J = 5.6 Hz, 2H)
MS m/z (ESI): $[M+H]^+$: 339.21

**Embodiment 12**

Preparation of [(7-hydroxy-5-(1,2,3,4-tetrahydronaphthalene-2-yl)-[1,2,4]triazolo[1,5-a]pyridine-8-carbonyl)amino] acetic acid.

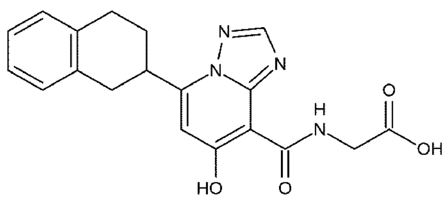

Step 1 Preparation of tert-butyl 5-(3,4-dihydronaphthalene-2- yl)-7-(benzyloxy)-[1,2,4]triazolo[1,5-a]pyridine-8-carboxylate

Tert-butyl 5-iodo-7-(benzyloxy)-[1,2,4]triazolo[1,5-a]pyridine-8-carboxylate (5.40 g), $Pd(PPh_3)_2Cl_2$ (800 mg), potassium carbonate (4.20 g) and 3,4-dihydronaphthyl-2-boronic acid pinacol ester (3.10 g) were dissolved in a mixed

solvent (30 mL) of ethylene glycol dimethyl ether and de-aerated water in a volume ratio of 2: 1. Nitrogen was replaced thrice. Subsequently, the mixture was stirred at 80°C, and after complete reaction of raw materials was monitored by TLC, the heating was stopped. The reaction solution was cooled to room temperature, and then extracted with ethyl acetate. An organic phase was washed with a saturated salt solution. The organic phase was separated, concentrated under a reduced pressure, and separated by column chromatography to obtain 3.30 g of crude product.

Step 2 Preparation of 5-(3,4-dihydronaphthalene-2-yl)-7-(benzyloxy)-[1,2,4]triazolo[1,5-a]pyridine-8-carboxylic acid

The tert-butyl 5-(3,4-dihydronaphthalene-2-yl)-7-(benzyloxy)-[1,2,4]triazolo[1,5-a]pyridine-8-carboxylate (3.30 g) was dissolved in a mixed solution (28 mL) of toluene and ethyl acetate in a volume ratio of 3: 1, then methanesulfonic acid (2.80 g) was added into the reaction system and stirred until complete reaction of raw materials was monitored by TLC, and ethyl acetate (20.0 mL) was added into the reaction system and stirred for crystallization, and filtered by suction to collect a solid. Subsequently, the solid was pulped with DMF/$H_2O$ (20.0 mL /40.0 mL), stirred for crystallization, and filtered by suction to collect a solid, so as to obtain 2.70 g of pale cyan solid.

Step 3 Preparation of [5-(3,4-dihydronaphthalene-2-yl)-7-(benzyloxy)-[1,2,4]triazolo[1,5-a]pyridine-8-carbonyl)amino] methyl acetate.

The tert-butyl 5-(3,4-dihydronaphthalene-2-yl)-7-(benzyloxy)-[1,2,4]triazolo[1,5-a]pyridine-8-carboxylate (2.70 g), N,N-dimethylformamide (20.0 mL), HOBt (1.10 g), EDCI (1.40 g), glycine methyl ester hydrochloride (0.90 g) and triethylamine (1.50 g) were sequentially added into a reaction bottle, and stirred at room temperature until complete reaction of raw materials was monitored by TLC, and water (30.0 mL) was added into the reaction system, stirred for crystallization, filtered by suction to collect a solid, and dried to a white-like solid (2.2 g).

Step 4 Preparation of [(7-hydroxy-5-(1,2,3,4-tetrahydronaphthalene-2-yl)-[1,2,4]triazolo[1,5-a]pyridine-8-carbonyl)amino]methyl acetate

[5-(3,4-dihydronaphthalene-2-yl)-7-(benzyloxy)-[1,2,4]triazolo[1,5-a]pyridine-8-carbonyl]amino]acetic acid (1.0 g) was dissolved in a tetrahydrofuran solution (8.0 mL), and then added with palladium carbon (300 mg). Hydrogen was replaced, and the mixture was stirred at room temperature until complete reaction of raw materials was monitored by TLC, filtered with diatomite, concentrated under a reduced pressure, then pulped with isopropyl ether/n-hexane (1.5 mL/1.5 mL), and filtered to obtain about 500 mg of white solid.

Step 5 Preparation of [(7-hydroxy-5-(1,2,3,4-tetrahydronaphthalene-2-yl)-[1,2,4]triazolo[1,5-a]pyridine-8-carbonyl)amino]acetic acid

[(7-hydroxy-5-(1,2,3,4-tetrahydronaphthalene-2-yl)-[1,2,4]triazolo[1,5-a]pyridine-8-carbonyl)amino]methyl acetate (0.20 g) was dissolved in an ethanol solution (2.0 mL), then added with a sodium hydroxide aqueous solution (2.0 mL, 4.0 M), and stirred at room temperature until complete reaction of raw materials was monitored by TLC, and the mixture was added with dilute hydrochloric acid to adjust pH to be 6 to 7, and filtered to obtain 200 mg of grayish-white solid.

$^1$H NMR (400 MHz, d-DMSO) $\delta$: 14.37 (brs, 1H), 9.93 (s, 1H), 8.56 (s, 1H), 7.13 (s, 4H), 6.78 (s, 1H), 4.12 (d, J = 4.0 Hz, 2H), 3.81-3.76 (m, 1H), 3.26-2.86 (m, 4H), 2.26-2.05 (m, 2H),
MS m/z (ESI): $[M+H]^+$: 367.32

**Embodiment 13 Preparation of chiral isomer A of [(7-hydroxy-5-(1,2,3,4-tetrahydronaphthalene-2-yl)-[1,2,4] triazolo[1,5-a]pyridine-8-carbonyl)amino]acetic acid**

Step 1 Preparation of tert-butyl 5-(3,4-dihydronaphthalene-2- yl)-7-(benzyloxy)-[1,2,4]triazolo[1,5-a]pyridine-8-carboxylate

Tert-butyl 5-iodo-7-(benzyloxy)-[1,2,4]triazolo[1,5-a]pyridine-8-carboxylate (5.40 g), $Pd(PPh_3)_2Cl_2$ (800 mg), sodium carbonate (4.20 g) and 3,4-dihydronaphthyl-2-boronic acid pinacol ester (3.10 g) were dissolved in a mixed solvent (30 mL) of ethylene glycol dimethyl ether and de-aerated water in a volume ratio of 2: 1. Nitrogen was replaced thrice. Subsequently, the mixture was stirred at 80°C, and after complete reaction of raw materials was monitored by TLC, the heating was stopped. The reaction solution was cooled to room temperature, and then extracted with ethyl acetate. An organic phase was washed with a saturated salt solution. The organic phase was separated, concentrated under a reduced pressure, and separated by column chromatography to obtain 3.30 g of crude product.

Step 2 Preparation of 5-(3,4-dihydronaphthalene-2-yl)-7-(benzyloxy)-[1,2,4]triazolo[1,5-a]pyridine-8-carboxylic acid

The tert-butyl 5-(3,4-dihydronaphthalene-2-yl)-7-(benzyloxy)-[1,2,4]triazolo[1,5-a]pyridine-8-carboxylate (3.30 g) was dissolved in a mixed solution (28 mL) of toluene and ethyl acetate in a volume ratio of 3: 1, then methanesulfonic acid (2.80 g) was added into the reaction system and stirred until complete reaction of raw materials was monitored by TLC, and ethyl acetate (20.0 mL) was added into the reaction system and stirred for crystallization, and filtered by suction to collect a solid. Subsequently, the solid was pulped with DMF/$H_2O$ (20.0 mL /40.0 mL), stirred for crystallization, and filtered by suction to collect a solid, so as to obtain 2.70 g of pale cyan solid.

Step 3 Preparation of [5-(3,4-dihydronaphthalene-2-yl)-7-(benzyloxy)-[1,2,4]triazolo[1,5-a]pyridine-8-carbonyl)amino] methyl acetate.

The tert-butyl 5-(3,4-dihydronaphthalene-2-yl)-7-(benzyloxy)-[1,2,4]triazolo[1,5-a]pyridine-8-carboxylate (2.70 g), N,N-dimethylformamide (20.0 mL), HoBt (1.10 g), EDCI (1.40 g) and methyl glycinate hydrochloride (0.90 g) were sequentially added into a reaction bottle, and stirred at room temperature until complete reaction of raw materials was monitored by TLC, and water (30.0 mL) was added into the reaction system, stirred for crystallization, filtered by suction to collect a solid, and dried to a white-like solid (2.2 g).

Step 4 Preparation of [(7-hydroxy-5-(1,2,3,4-tetrahydronaphthalene-2-yl)-[1,2,4]triazolo[1,5-a]pyridine-8-carbonyl)amino]methyl acetate

[5-(3,4-dihydronaphthalene-2-yl)-7-(benzyloxy)-[1,2,4]triazolo[1,5-a]pyridine-8-carbonyl]amino]acetic acid (1.0 g) was dissolved in a tetrahydrofuran solution (8.0 mL), and then added with palladium carbon (300 mg). The mixture was stirred at room temperature until complete reaction of raw materials was monitored by TLC, filtered with diatomite, concentrated under a reduced pressure, then pulped with isopropyl ether/n-hexane (1.5 mL/1.5 mL), and filtered to obtain about 500 mg of white solid.

Step 5 Preparation of chiral isomer A of [(7-hydroxy-5-(1,2,3,4-tetrahydronaphthalene-2-yl)-[1,2,4]triazolo[1,5-a]pyridine-8-carbonyl)amino]methyl acetate (chiral isomer A of methyl acetate)

The product synthesized in the previous step was subjected to chiral resolution to resolve 5 g of [(7-hydroxy-5-(1,2,3,4-tetrahydronaphthalene-2-yl)-[1,2,4]triazolo[1,5-a]pyridine-8-carbonyl)amino]methyl acetate under resolution conditions: (chiral column: (S, S) Whelk-O1, 5*25 cm, 10 $\mu$m; mobile phase A: $CO_2$, mobile phase B: ACN: IPA=1: 1; flow rate: 200 ml/min; gradient: 50% to 50%; B travel for 12 minutes; wavelength: 220 nm; peak appearance time: 8.50 minutes; sample solution: methanol: dichloromethane = 1: 1; sample volume: 2 ML; number of syringes: 50), so as to obtain the chiral isomer A: white solid (2.060 g, yield 41.2%).

Step 6 Preparation of chiral isomer A of [(7-hydroxy-5-(1,2,3,4-tetrahydronaphthalene-2-yl)-[1,2,4]triazolo[1,5-a]pyridine-8-carbonyl)amino]acetic acid

The chiral isomer A of [(7-hydroxy-5-(1,2,3,4-tetrahydronaphthalene-2-yl)-[1,2,4]triazolo[1,5-a]pyridine-8-carbonyl)amino]methyl acetate (0.20 g) was dissolved in an ethanol solution (2.0 mL), then added with a sodium hydroxide aqueous solution (1.0 mL, 4.0 M), and stirred at room temperature until complete reaction of raw materials was monitored by TLC, and the mixture was added with dilute hydrochloric acid to adjust pH to be 1 to 2, and filtered to obtain 200 mg of grayish-white solid.

1H NMR (400 MHz, d-DMSO) $\delta$: 14.37 (brs, 1H), 9.93 (s, 1H), 8.56 (s, 1H), 7.13 (s, 4H), 6.78 (s, 1H), 4.12 (d, J = 4.0 Hz, 2H), 3.81-3.76 (m, 1H), 3.26-2.86 (m, 4H), 2.26-2.05 (m, 2H),
MS m/z (ESI): $[M+H]^+$: 367.32
$[\alpha]D^{20}$ = +37.384 (c 1.01, DMF).

**Embodiment 14 Preparation of chiral isomer B of [(7-hydroxy-5-(1,2,3,4-tetrahydronaphthalene-2-yl)-[1,2,4] triazolo[1,5-a]pyridine-8-carbonyl)amino]acetic acid**

Step 1 Preparation of chiral isomer B of [(7-hydroxy-5-(1,2,3,4-tetrahydronaphthalene-2-yl)-[1,2,4]triazolo[1,5-a]pyridine-8-carbonyl)amino]methyl acetate

5 g of the [(7-hydroxy-5-(1,2,3,4-tetrahydronaphthalene-2-yl)-[1,2,4]triazolo[1,5-a]pyridine-8-carbonyl)amino]methyl acetate synthesized was subjected to chiral resolution under resolution conditions: (chiral column: (S, S) Whelk-O1, 5*25 cm, 10 μm; mobile phase A: $CO_2$, mobile phase B: ACN: IPA=1: 1; flow rate: 200 ml/min; gradient: 50% to 50%; B travel for 12 minutes; wavelength: 220 nm; OB peak appearance time: 10.09 minutes; sample solution: methanol: dichloromethane = 1: 1; sample volume: 2 ML; number of syringes: 50), so as to obtain the chiral isomer B (chiral isomer B of methyl acetate): white solid (1.997 g, yield 39.94%).

Step 2 Preparation of chiral isomer B of [(7-hydroxy-5-(1,2,3,4-tetrahydronaphthalene-2-yl)-[1,2,4]triazolo[1,5-a]pyridine-8-carbonyl)amino]acetic acid

The chiral isomer B of [(7-hydroxy-5-(1,2,3,4-tetrahydronaphthalene-2-yl)-[1,2,4]triazolo[1,5-a]pyridine-8-carbonyl)amino]methyl acetate (0.20 g) was dissolved in an ethanol solution (2.0 mL), then added with a sodium hydroxide aqueous solution (1.0 mL, 4.0 M), and stirred at room temperature until complete reaction of raw materials was monitored by TLC, and the mixture was added with dilute hydrochloric acid to adjust pH to be 1 to 2, and filtered to obtain 200 mg of grayish-white solid.

$^1$H NMR (400 MHz, d-DMSO) δ: 14.37 (brs, 1H), 9.93 (s, 1H), 8.56 (s, 1H), 7.13 (s, 4H), 6.78 (s, 1H), 4.12 (d, J = 4.0 Hz, 2H), 3.81-3.76 (m, 1H), 3.26-2.86 (m, 4H), 2.26-2.05 (m, 2H),
MS m/z (ESI): $[M+H]^+$: 367.32
$[\alpha]D^{20}$ = -36.601 (c 1.01, DMF).

**Embodiment 15 Biological test**

**1. Detection of PHD2 enzyme activity inhibition**

1.1 Reagents and consumables

PHD2 enzyme: purchased from Active motif;
α-ketoglutaric acid sodium salt: purchased from Sigma;
FITC-HIF1α: purchased from GL; and
porous plate Nunc™ 384: purchased from Thermo Scientific.

1.2 Experimental method

(1) A 1×Assay buffer was prepared.
(2) Preparation of concentration gradient of compound: a test concentration of a tested compound started from 10 μM, and the tested compound was diluted 3 times into 10 concentrations for a parallel well test; and the tested compound was diluted into a solution with 100 times final concentration in a 384 well plate, and then 100 nL of the tested compound was transferred to a 384 reaction plate by a non-contact acoustic pipetting system Echo550. 100 nL of 100% DMSO was added into a negative control well and a positive control well respectively.
(3) An enzyme solution with 2 times final concentration was prepared with the 1×Assay buffer.
(4) 5 μL of the enzyme solution with 2 times final concentration was added into a compound well and the positive control well respectively.
(5) 5 μL of the 1 ×Assay buffer was added into the negative control well.
(6) The 384-well plate was centrifuged at 1000 rpm for 30 seconds, oscillated and mixed evenly, and then incubated for 15 minutes.
(7) A tracer solution with 2 times final concentration was prepared with the 1×Assay buffer, and 5.0 μL of the tracer solution with 2 times final concentration was added to start the reaction.
(8) The 384-well plate was centrifuged at 1000 rpm for 30 seconds, and oscillated and mixed evenly for 60 minutes, an mP value was read from an Envision Multimode PlateReader (Perkin Elmer), and data were exported and processed to obtain an inhibition rate of a compound to be tested. Serial numbers 1 to 12 of compounds corresponded to the compounds prepared in Embodiments 1 to 12 respectively, and results were shown in the following table:

Table 1

| Serial number of compound | $IC_{50}$ (nM) |
|---|---|
| FG-4592 | 89.2 |
| Enarodustat | 21.9 |
| Compound in Embodiment 1 | 19.8 |
| Compound in Embodiment 2 | 34.8 |
| Compound in Embodiment 3 | 30.2 |
| Compound in Embodiment 4 | 33.3 |
| Compound in Embodiment 5 | 44.1 |
| Compound in Embodiment 6 | 47.6 |
| Compound in Embodiment 7 | 34.1 |
| Compound in Embodiment 8 | 35.4 |
| Compound in Embodiment 9 | 34.0 |
| Compound in Embodiment 10 | 46.7 |
| Compound in Embodiment 11 | 31.3 |
| Compound in Embodiment 12 | 34.3 |

It could be seen from the above table that the compounds in the embodiments of the present invention had good HIF-prolyl hydroxylase domain inhibitory activity, so that the compounds 1 to 12 had good inhibitory activity.

**2. In-vitro erythropoietin (EPO)-inducing activity experiment of compounds of the present invention**

In-vitro erythropoietin (EPO)-inducing activities of the compounds in Embodiments 10 to 12 were evaluated by using a human hepatocarcinoma cell line Hep3B (ATCC). Hep3B cells were cultured in an EMEM medium (Eagle's Minimum Essential Medium) at 37°C in the presence of 10% fetal bovine serum (FBS). Experimental steps were as follows:

(1) Cell preparation: the cells were inoculated in a 96-well plate, and cell plating was carried out according to an amount of 100 μL per well based on 2*10~4 per well.
(2) Preparation of compound concentration gradient: 100 μM, 20 μM, 2-parallel well detection. A solution with 200 times final concentration was prepared in a 96-well plate, the compound was diluted 200/3 times by using a cell culture medium, and then 50 μL of the compound was sucked for drug administration to the cells. 50 μL of DMSO-containing culture solution was added into a negative control well to make a final concentration contain 5 ‰ DMSO, and 50 μL of positive compound with the highest concentration was added into a positive control well to incubate the cells at 37°C for 24 hours.
(3) The reaction plate was washed twice with 1X Wash Buffer according to about 400 μL per well.
(4) 100 μL of diluted standard (comprising a standard blank control) was added into an appropriate well.
(5) 50 μL of sample and 50 μL of Sample Diluent were added into a sample well.
(6) 50 μL of 1X Biotin Conjugated Antibody were added into all wells to incubate the cells at room temperature for 1 hour.
(7) The reaction plate was washed with 1X Wash Buffer for 6 times according to about 400 μL per well.
(8) 100 μL of 1X Streptavidin-HRP was added in to each well to incubate the cells at room temperature for 15 minutes.
(9) The reaction plate was washed with 1X Wash Buffer for 6 times according to about 400 μL per well.
(10) 100 μL of TMB Substrate Solution was added into each well to incubate the cells at room temperature for 10 minutes.
(11) 100 μL of Stop Solution was added into each well.
(12) OD450 was read by EnSight.

The EPO-inducing activities of the compounds were expressed as the concentration for 50% of maximal effect (EC50). Experimental results were shown in the following Table:

Table 2

| Serial number | $EC_{50}$ (μM) |
|---|---|
| FG-4592 | 13.79 |
| Enarodustat | 15.06 |
| Compound in Embodiment 10 | 6.46 |
| Compound in Embodiment 11 | 1.79 |
| Compound in Embodiment 12 | 6.79 |
| Compound in Embodiment 13 | 5.05 |
| Compound in Embodiment 14 | 4.16 |

It could be seen from the above table that the compounds of the present invention had high EPO-inducing activity, so that the compounds in Embodiments 10 to 14 had good inducing activity.

**Embodiment 16 Pharmacokinetic evaluation of compounds**

1. Experimental purpose

After the compounds were given to SD rats by intravenous injection or intragastric administration, concentrations of related compounds in plasma were detected by an LC-MS/MS method, and pharmacokinetics and absolute bioavailability of the compounds prepared in Embodiments 13 and 14 in the rats were preliminarily evaluated.

2. Line: Sprague Dawley rats, sex: male, weight: 200 g to 250 g

Source: Zhejiang Vital River Laboratory Animal Technology Co., Ltd.

3. Experimental operation

Pharmacokinetic characteristics of rodents after intravenous injection and oral administration of the compounds were tested by a standard scheme. In the experiment, a candidate compound was prepared into a clear solution, and given to the rats by single intravenous injection and intragastric administration. A solvent for intravenous injection was a certain proportion of N,N-dimethylacetamide (DMA) and 10% Solutol HS15 solution, and a solvent for oral administration was a certain proportion of sodium carboxymethyl cellulose (CMC) suspension. 5 minutes, 15 minutes, 30 minutes, 1 hour, 2 hours, 4 hours, 6 hours, 8 hours, 10 hours and 24 hours after drug administration, blood samples of the animals were respectively collected into K2EDTA anticoagulation tubes, which were temporarily stored on ice until centrifugation.

Experimental results:

| Test sample | Half-life period $T_{1/2}$ (h) | Concentration integral AUC (hr*ng/mL) | Bioavailability F (%) |
|---|---|---|---|
| Enarodustat | 1.12 | 11440 | 30.1 |
| Compound in Embodiment 13 | 2.02 | 19130 | 29.4 |
| Compound in Embodiment 14 | 2.43 | 15740 | 31.3 |

It could be seen from the above table that the compounds prepared in Embodiments 13 and 14 of the present invention had good PK property, so that half-lives of the compounds prepared in Embodiments 13 and 14 were obviously longer, and in-vivo exposure was higher.

**Embodiment 17 Pharmacological evaluation of compounds**

1. Experimental purpose

This experiment was intended to evaluate an influence of continuous administration of the compounds prepared in Embodiments 12, 13 and 14 on EPO (erythropoietin) in SD rats.

2. Animals

Species: rats

Line: SD (Sprague Dawley)
Weeks of age and weight: 8 weeks, weight 200 g to 220 g
Sex: male
Animal source: Beijing Vital River Laboratory Animal Technology Co., Ltd.

3. Tested compound and positive control drug information

Positive drug: Enarodustat
Description: commercialized drugs approved for marketing in Japan
Specification and content: 2 mg JTZ-951(Enarodustat), 140 capsules/box

4. Experimental scheme:

After a week of adaptive feeding, SD rats were randomly divided into 3 groups, 6 rats in each group, which were namely a blank group, a positive drug group (Enarodustat) and a group of compound in Embodiment 12. An Enarodustat tablet was crushed by a mortar, then quantitatively added with 0.5% methyl cellulose, and magnetically stirred and mixed evenly; and the compound prepared in Embodiment 12 was weighed, then quantitatively added with 0.5% methyl cellulose, and magnetically stirred and mixed evenly. All the compounds had a final concentration of 0.3 mg/mL and an administration volume of 10 mL/kg. The drug was given once a day for 14 consecutive days, and blood was taken to measure an EPO level 4 hours after the drug was given on the $14^{th}$ day.

5. Results and analysis

The results showed that EPO levels in serum of the rats in the groups of positive drug Enarodustat and compounds prepared in Embodiments 12, 13 and 14 were increased significantly after 14 days of administration, and EPO levels in plasma of the rats in the groups of compounds prepared in Embodiments 12, 13 and 14 were significantly higher than that of the rats in the group of positive drug Enarodustat after administration.

Table 3 EPO level (pg/mL) after 14 days of administration

| | Vehicle | Enarodustat | Compound in Embodiment 12 | Compound in Embodiment 13 | Compound in Embodiment 14 |
|---|---|---|---|---|---|
| 1 | 36.7 | 452.8 | 2649.3 | 2904.6 | 2279.1 |
| 2 | 30.9 | 396.3 | 2904.6 | 2697.4 | 2095.6 |
| 3 | 31.8 | 483.2 | 2882.6 | 2904.6 | 1982.7 |
| 4 | 45.4 | 404.5 | 2904.6 | 2904.6 | 1943.6 |
| 5 | 49.6 | 418.4 | 2904.6 | 2658.5 | 2038.9 |
| 6 | 38.1 | 442.3 | 2904.6 | 2772.1 | 2137.6 |
| Mean | 38.8 | 432.9 | 2858.4 | 2807.0 | 2079.6 |

Remark: 2904.6 pg/mL was an upper limit of measurement.

To sum up, the compounds prepared in Embodiments 12, 13 and 14 could significantly increase the EPO level of

the SD rats, and had an overall effect significantly better than that of the positive drug Enarodustat, showing excellent pharmacological effect.

**Embodiment 18 Determination of chiral configuration of compound of chiral isomer A of methyl acetate in Embodiment 13**

1. Preparation of compound

With reference to the preparation method of the chiral isomer A of [(7-hydroxy-5-(1,2,3,4-tetrahydronaphthalene-2-yl)-[1,2,4 ]triazolo[1,5-a]pyridine-8-carbonyl)amino]methyl acetate (step 5) in Embodiment 13, the chiral isomer A of methyl acetate was obtained by chiral resolution, and the isomer was the previous compound of the final product in Embodiment 13 (product of step 5).

2. Culture of single crystal

About 3 mg to 7 mg of the chiral isomer A of methyl acetate was added into a bottle, and gradually added with acetonitrile while stirring at 50°C until a medicine liquid was just clear or nearly clear. The mixture was filtered while the mixture was hot, and a filtrate was placed into a clean bottle, and cooled by a Crystal 16 instrument, which was cooled from 50°C to 30°C at a rate of 0.01°C/min, and then kept at 30°C for about 2 days.

A needle-shaped single crystal was obtained from acetonitrile by cooling and crystallization, as shown in FIG. 1, and used for X-ray single crystal diffraction analysis.

3. Instruments and parameters

Single crystal data of the chiral isomer A of methyl acetate were collected by a Rigaku XtaLAB Synergy DW diffractometer at 180 K by using a Cu K$\alpha$ ray ($\lambda$=1.54184 Å). Diffraction data were subjected to data restoration and absorption correction by using a CrysAlisPro program, and the structure was analyzed through a dual space algorithm by using a SHELXT program. All non-hydrogen atoms were directly located from a Fourier diagram of difference, and hydrogen atoms were filled on parent atoms. The final structure was refined based on an F2 full-matrix least square method by using the SHELXL program.

4. X-ray diffraction analysis

The single crystal structure of the chiral isomer A of methyl acetate belonged to a monoclinic system and a P21 space group, and a molecular formula of single crystal was $C_{20}H_{20}N_4O_4$. There was one molecule of chiral isomer A of methyl acetate in each asymmetric unit, and each unit cell contained two asymmetric units. An absolute configuration of chiral carbon was "R".

The refined single crystal structure was as shown in FIG. 2, FIG. 3 and FIG. 4. Crystal structure parameters were summarized in Table 4, and details of atomic coordinates, anisotropic displacement parameters, torsion angle, hydrogen bond, bond length and bond angle data were shown in Table 5 to Table 11. An XRPD pattern calculated according to the single crystal structure was basically consistent with test results of the single crystal sample, but a peak position was offset to some extent, which could be due to the fact that the single crystal data were collected at 180 K, while the XRPD pattern determined by the experiment was made at room temperature (FIG. 5).

Table 4. Crystal structure parameters of chiral isomer A of methyl acetate

| | |
|---|---|
| Molecular formula | $C_{20}H_{20}N_4O_4$ |
| Molecular weight | 380.40 |
| Temperature/K | 180.00(10) |
| Crystal system | Monoclinic system |
| Space group | $P2_1$ |
| *a*/Å | 13.2296(2) |
| *b*/Å | 5.10250(10) |
| *c*/Å | 13.7423(2) |
| $\alpha$/° | 90 |

(continued)

| | |
|---|---|
| $\beta/°$ | 104.357(2) |
| $\gamma/°$ | 90 |
| Unit cell volume/$Å^3$ | 898.69(3) |
| $Z$ | 2 |
| $\rho_{calc}g/cm^3$ | 1.406 |
| $\mu/mm^{-1}$ | 0.828 |
| $F(000)$ | 400.0 |
| Crystal size/$mm^3$ | 0.48 × 0.04 × 0.02 |
| Diffraction light source | Cu Kα ($\lambda$ = 1.54184 Å) |
| 2θscope/° of data collection | 6.64 to 145.488 |
| Scope of diffraction index | $-16 \leq h \leq 15$, $-6 \leq k \leq 6$, $-16 \leq l \leq 16$ |
| Collection number of diffraction points | 15082 |
| Number of independent diffraction points | 3389 [$R_{int}$ = 0.0370, $R_{sigma}$ = 0.0282] |
| Data/restriction/parameter | 3389/3/260 |
| Goodness of fit based on $F^2$ | 1.063 |
| Final $R$ value [$I$>=2$\sigma$ ($I$)] | $R_1$= 0.0389, $wR_2$= 0.1045 |
| Final $R$ value [all data] | $R_1$ = 0.0407, $wR_2$ = 0.1067 |
| Peak/valley/e $Å^{-3}$ of maximum residual electron density | 0.53/-0.21 |
| Flack parameter | 0.04(9) |

Table 5. Atomic coordinates (×$10^4$) and equivalent isotropic shift parameters ($Å^2$×$10^3$) of chiral isomer A of methyl acetate

| Atom | x | y | z | U(eq) |
|---|---|---|---|---|
| O1 | 3838.8(15) | 12688(4) | -49.0(13) | 46.2(5) |
| O2 | 3791.7(17) | 9024(6) | 837.1(17) | 65.1(7) |
| O3 | 7203.8(14) | 12162(4) | 2559.3(12) | 40.2(4) |
| O4 | 8666.9(13) | 10360(4) | 3967.4(13) | 39.3(4) |
| N1 | 5615.5(16) | 10253(5) | 2171.4(14) | 36.6(4) |
| N2 | 5282.0(14) | 6067(4) | 3404.3(14) | 35.3(4) |
| N3 | 5897.1(15) | 3302(4) | 4724.3(14) | 35.4(4) |
| N4 | 6661.4(14) | 5018(4) | 4612.7(14) | 30.5(4) |
| C1 | 2834(2) | 12039(7) | -710(2) | 49.8(7) |
| C2 | 4200.2(19) | 11068(6) | 711.2(17) | 39.1(5) |
| C3 | 5227(2) | 12043(5) | 1345.5(17) | 39.9(5) |
| C4 | 6593.7(18) | 10453(5) | 2731.0(16) | 32.7(5) |
| C5 | 6942.0(17) | 8563(5) | 3556.8(16) | 30.8(4) |
| C6 | 6278.8(17) | 6657(5) | 3811.2(16) | 31.0(5) |
| C7 | 5107.2(17) | 4063(5) | 3986.1(17) | 36.7(5) |
| C8 | 7674.1(17) | 5054(5) | 5193.2(16) | 30.9(4) |
| C9 | 8322.9(17) | 6845(5) | 4945.1(16) | 32.4(5) |
| C10 | 7971.4(17) | 8634(5) | 4133.5(16) | 31.7(5) |

(continued)

| Atom | x | y | z | U(eq) |
|---|---|---|---|---|
| C11 | 7945.5(16) | 3092(4) | 6037.9(16) | 30.5(5) |
| C12 | 7426.2(17) | 3712(5) | 6898.2(17) | 35.4(5) |
| C13 | 7585.6(18) | 1429(6) | 7632.6(18) | 39.6(5) |
| C14 | 8675.6(18) | 307(5) | 7883.5(16) | 34.4(5) |
| C15 | 8965(2) | -1499(6) | 8668.7(18) | 43.1(6) |
| C16 | 9942(2) | -2652(6) | 8904.0(19) | 47.7(6) |
| C17 | 10655(2) | -2009(6) | 8356(2) | 46.1(6) |
| C18 | 10377.8(19) | -210(5) | 7578.0(19) | 40.6(5) |
| C19 | 9391.3(17) | 938(5) | 7326.8(16) | 32.4(5) |
| C20 | 9122.4(17) | 2870(5) | 6468.3(17) | 34.1(5) |

Table 6. Anisotropic shift parameters ($Å^2 \times 10^3$) of chiral isomer A of methyl acetate

| Atom | $U_{11}$ | $U_{22}$ | $U_{33}$ | $U_{23}$ | $U_{13}$ | $U_{12}$ |
|---|---|---|---|---|---|---|
| O1 | 49.2(10) | 48.8(11) | 33.1(8) | 8.3(8) | -4.2(7) | 1.8(8) |
| O2 | 51.3(11) | 77.9(16) | 56.9(12) | 25.9(12) | -4.4(9) | -19.0(11) |
| O3 | 42.9(9) | 43.1(9) | 32.3(8) | 6.1(7) | 5.0(7) | -2.9(8) |
| O4 | 34.1(8) | 43.9(10) | 37.8(8) | 6.8(8) | 5.0(7) | -4.3(7) |
| N1 | 39.9(10) | 41.0(11) | 25.9(9) | 6.4(8) | 2.2(8) | -0.6(9) |
| N2 | 29.6(9) | 46.1(11) | 28.3(9) | 1.7(8) | 3.4(7) | 1.2(8) |
| N3 | 31.5(9) | 41.9(11) | 31.2(9) | 2.3(8) | 4.9(7) | -3.8(8) |
| N4 | 29.5(9) | 34.4(9) | 26.2(8) | 1.0(7) | 4.5(7) | 1.6(7) |
| C1 | 39.1(12) | 69.3(18) | 35.4(12) | 6.1(13) | -1.4(10) | 9.9(13) |
| C2 | 37.1(11) | 51.1(14) | 28.8(10) | 7.9(10) | 7.5(9) | 2.3(11) |
| C3 | 45.6(13) | 40.6(13) | 29.1(10) | 5.8(10) | 0.9(9) | -0.2(10) |
| C4 | 38.6(11) | 34.5(11) | 25.3(9) | -0.5(9) | 8.4(8) | 1.5(9) |
| C5 | 33.0(10) | 33.8(11) | 25.1(9) | -1.6(8) | 5.9(8) | 4.0(9) |
| C6 | 31.1(10) | 37.0(12) | 24.1(9) | -0.3(9) | 5.3(8) | 5.6(8) |
| C7 | 30.0(10) | 46.0(13) | 32.0(10) | 1.3(10) | 3.9(9) | -3.5(10) |
| C8 | 29.4(10) | 34.3(11) | 28.0(10) | -2.7(9) | 5.1(8) | 3.3(9) |
| C9 | 29.1(10) | 36.9(11) | 28.9(10) | -1.7(9) | 2.7(8) | 3.2(9) |
| C10 | 31.8(10) | 34.4(11) | 29.8(10) | -1.0(9) | 9.2(8) | 1.1(9) |
| C11 | 29.1(10) | 32.7(11) | 27.8(10) | -0.5(8) | 3.4(8) | 2.7(8) |
| C12 | 32.8(10) | 42.5(12) | 29.5(10) | -1.0(9) | 5.0(8) | 6.6(10) |
| C13 | 33.1(11) | 54.0(14) | 32.0(11) | 4.8(10) | 8.3(9) | 4.7(10) |
| C14 | 35.1(11) | 40.1(12) | 24.2(10) | -2.1(9) | 0.1(8) | 0.5(9) |
| C15 | 43.4(12) | 54.1(15) | 28.9(11) | 2.3(10) | 3.2(9) | -3.8(11) |
| C16 | 51.6(14) | 49.7(15) | 32.9(11) | 7.9(11) | -6.1(10) | 4.4(12) |
| C17 | 38.4(12) | 49.8(14) | 43.0(13) | 6.4(11) | -3.3(10) | 10.4(11) |
| C18 | 34.5(11) | 46.1(13) | 37.9(12) | 1.6(10) | 2.4(9) | 3.7(10) |

(continued)

| Atom | $U_{11}$ | $U_{22}$ | $U_{33}$ | $U_{23}$ | $U_{13}$ | $U_{12}$ |
|---|---|---|---|---|---|---|
| C19 | 31.0(10) | 34.3(11) | 28.3(10) | -2.4(8) | 0.4(8) | 0.1(9) |
| C20 | 29.3(10) | 38.6(12) | 32.5(11) | 3.8(9) | 4.0(8) | 0.7(9) |

Table 7. Bond length of chiral isomer A of methyl acetate

| Atom | Atom | Bond length/Å | | Atom | Atom | Bond length/Å |
|---|---|---|---|---|---|---|
| O1 | C1 | 1.450(3) | | C5 | C10 | 1.396(3) |
| O1 | C2 | 1.325(3) | | C8 | C9 | 1.354(3) |
| O2 | C2 | 1.206(4) | | C8 | C11 | 1.507(3) |
| O3 | C4 | 1.250(3) | | C9 | C10 | 1.427(3) |
| O4 | C10 | 1.334(3) | | C11 | C12 | 1.540(3) |
| N1 | C3 | 1.448(3) | | C11 | C20 | 1.526(3) |
| N1 | C4 | 1.335(3) | | C12 | C13 | 1.521(3) |
| N2 | C6 | 1.333(3) | | C13 | C14 | 1.510(3) |
| N2 | C7 | 1.353(3) | | C14 | C15 | 1.398(4) |
| N3 | N4 | 1.375(3) | | C14 | C19 | 1.395(3) |
| N3 | C7 | 1.321(3) | | C15 | C16 | 1.383(4) |
| N4 | C6 | 1.376(3) | | C16 | C17 | 1.385(4) |
| N4 | C8 | 1.379(3) | | C17 | C18 | 1.387(4) |
| C2 | C3 | 1.505(3) | | C18 | C19 | 1.394(3) |
| C4 | C5 | 1.473(3) | | C19 | C20 | 1.511(3) |
| C5 | C6 | 1.411(3) | | | | |

Table 8. Bond angle of chiral isomer A of methyl acetate

| Atom | Atom | Atom | Bond angle/° | | Atom | Atom | Atom | Bond angle/° |
|---|---|---|---|---|---|---|---|---|
| C2 | O1 | C1 | 116.5(2) | | C9 | C8 | N4 | 116.7(2) |
| C4 | N1 | C3 | 120.6(2) | | C9 | C8 | C11 | 126.69(19) |
| C6 | N2 | C7 | 102.66(19) | | C8 | C9 | C10 | 121.63(19) |
| C7 | N3 | N4 | 101.00(19) | | O4 | C10 | C5 | 122.5(2) |
| N3 | N4 | C6 | 109.98(18) | | O4 | C10 | C9 | 116.8(2) |
| N3 | N4 | C8 | 125.45(19) | | C5 | C10 | C9 | 120.7(2) |
| C6 | N4 | C8 | 124.54(19) | | C8 | C11 | C12 | 112.85(18) |
| O1 | C2 | C3 | 110.4(2) | | C8 | C11 | C20 | 111.90(19) |
| O2 | C2 | O1 | 124.4(2) | | C20 | C11 | C12 | 108.91(17) |
| O2 | C2 | C3 | 125.1(2) | | C13 | C12 | C11 | 109.70(19) |
| N1 | C3 | C2 | 109.9(2) | | C14 | C13 | C12 | 114.16(19) |
| O3 | C4 | N1 | 121.6(2) | | C15 | C14 | C13 | 119.3(2) |
| O3 | C4 | C5 | 120.6(2) | | C19 | C14 | C13 | 121.8(2) |
| N1 | C4 | C5 | 117.8(2) | | C19 | C14 | C15 | 118.8(2) |
| C6 | C5 | C4 | 123.2(2) | | C16 | C15 | C14 | 121.5(2) |
| C10 | C5 | C4 | 119.7(2) | | C15 | C16 | C17 | 119.7(2) |

(continued)

| Atom | Atom | Atom | Bond angle/° | | Atom | Atom | Atom | Bond angle/° |
|---|---|---|---|---|---|---|---|---|
| C10 | C5 | C6 | 117.1(2) | | C16 | C17 | C18 | 119.3(2) |
| N2 | C6 | N4 | 109.1(2) | | C17 | C18 | C19 | 121.4(2) |
| N2 | C6 | C5 | 131.5(2) | | C14 | C19 | C20 | 121.2(2) |
| N4 | C6 | C5 | 119.3(2) | | C18 | C19 | C14 | 119.2(2) |
| N3 | C7 | N2 | 117.2(2) | | C18 | C19 | C20 | 119.5(2) |
| N4 | C8 | C11 | 116.64(19) | | C19 | C20 | C11 | 111.78(19) |

Table 9. Torsion angle of chiral isomer A of methyl acetate

| A | B | C | D | Torsion angle/° | | A | B | C | D | Torsion angle/° |
|---|---|---|---|---|---|---|---|---|---|---|
| O1 | C2 | C3 | N1 | -179.8(2) | | C7 | N3 | N4 | C8 | -178.7(2) |
| O2 | C2 | C3 | N1 | -4.0(4) | | C8 | N4 | C6 | N2 | 178.6(2) |
| O3 | C4 | C5 | C6 | 177.5(2) | | C8 | N4 | C6 | C5 | -1.3(3) |
| O3 | C4 | C5 | C10 | -1.9(3) | | C8 | C9 | C10 | O4 | 178.4(2) |
| N1 | C4 | C5 | C6 | -3.3(3) | | C8 | C9 | C10 | C5 | -0.9(3) |
| N1 | C4 | C5 | C10 | 177.3(2) | | C8 | C11 | C12 | C13 | 170.60(19) |
| N3 | N4 | C6 | N2 | 0.6(2) | | C8 | C11 | C20 | C19 | 178.99(18) |
| N3 | N4 | C6 | C5 | -179.36(19) | | C9 | C8 | C11 | C12 | 110.1(2) |
| N3 | N4 | C8 | C9 | 177.9(2) | | C9 | C8 | C11 | C20 | -13.2(3) |
| N3 | N4 | C8 | C11 | -2.0(3) | | C10 | C5 | C6 | N2 | -178.6(2) |
| N4 | N3 | C7 | N2 | 0.6(3) | | C10 | C5 | C6 | N4 | 1.3(3) |
| N4 | C8 | C9 | C10 | 1.0(3) | | C11 | C8 | C9 | C10 | -179.1(2) |
| N4 | C8 | C11 | C12 | -70.0(3) | | C11 | C12 | C13 | C14 | 43.5(3) |
| N4 | C8 | C11 | C20 | 166.74(19) | | C12 | C11 | C20 | C19 | 53.5(3) |
| C1 | O1 | C2 | O2 | 6.0(4) | | C12 | C13 | C14 | C15 | 169.6(2) |
| C1 | O1 | C2 | C3 | -178.1(2) | | C12 | C13 | C14 | C19 | -13.1(3) |
| C3 | N1 | C4 | O3 | -0.5(4) | | C13 | C14 | C15 | C16 | 177.7(3) |
| C3 | N1 | C4 | C5 | -179.7(2) | | C13 | C14 | C19 | C18 | -178.4(2) |
| C4 | N1 | C3 | C2 | 168.2(2) | | C13 | C14 | C19 | C20 | 2.5(3) |
| C4 | C5 | C6 | N2 | 2.0(4) | | C14 | C15 | C16 | C17 | 0.2(4) |
| C4 | C5 | C6 | N4 | -178.12(19) | | C14 | C19 | C20 | C11 | -23.4(3) |
| C4 | C5 | C10 | O4 | -0.1(3) | | C15 | C14 | C19 | C18 | -1.1(3) |
| C4 | C5 | C10 | C9 | 179.18(19) | | C15 | C14 | C19 | C20 | 179.9(2) |
| C6 | N2 | C7 | N3 | -0.3(3) | | C15 | C16 | C17 | C18 | 0.1(4) |
| C6 | N4 | C8 | C9 | 0.1(3) | | C16 | C17 | C18 | C19 | -0.9(4) |
| C6 | N4 | C8 | C11 | -179.77(19) | | C17 | C18 | C19 | C14 | 1.4(4) |
| C6 | C5 | C10 | O4 | -179.6(2) | | C17 | C18 | C19 | C20 | -179.5(2) |
| C6 | C5 | C10 | C9 | -0.2(3) | | C18 | C19 | C20 | C11 | 157.6(2) |
| C7 | N2 | C6 | N4 | -0.2(2) | | C19 | C14 | C15 | C16 | 0.3(4) |
| C7 | N2 | C6 | C5 | 179.7(2) | | C20 | C11 | C12 | C13 | -64.5(2) |

(continued)

| A | B | C | D | Torsion angle/° | | A | B | C | D | Torsion angle/° |
|---|---|---|---|---|---|---|---|---|---|---|
| C7 | N3 | N4 | C6 | -0.7(2) | | | | | | |

Table 10. Hydrogen bond of chiral isomer A of methyl acetate

| D | H | A | d(D-H)/Å | d(H-A)/Å | d(D-A)/Å | D-H-A/° |
|---|---|---|---|---|---|---|
| O4 | H4 | O3 | 0.81(2) | 1.83(3) | 2.545(2) | 146(3) |
| N1 | H1 | N2 | 0.88(2) | 2.09(3) | 2.828(3) | 141(3) |

Table 11. Atomic coordinates (× $10^4$) and isotropic shift parameters (Å$^2$×$10^3$) of chiral isomer A of methyl acetate

| Atom | *x* | *y* | *z* | U(eq) |
|---|---|---|---|---|
| H4 | 8390(20) | 11350(60) | 3510(20) | 47 |
| H1 | 5220(20) | 9070(60) | 2360(20) | 44 |
| H1A | 2928 | 10715 | -1197 | 75 |
| H1B | 2522 | 13618 | -1067 | 75 |
| H1C | 2373 | 11346 | -312 | 75 |
| H3A | 5738 | 12183 | 930 | 48 |
| H3B | 5133 | 13806 | 1611 | 48 |
| H7 | 4444 | 3237 | 3869 | 44 |
| H9 | 9030 | 6913 | 5321 | 39 |
| H11 | 7686 | 1340 | 5757 | 37 |
| H12A | 7736 | 5322 | 7252 | 42 |
| H12B | 6671 | 4021 | 6621 | 42 |
| H13A | 7421 | 2023 | 8262 | 48 |
| H13B | 7086 | 21 | 7344 | 48 |
| H15 | 8480 | -1944 | 9049 | 52 |
| H16 | 10123 | -3879 | 9439 | 57 |
| H17 | 11328 | -2792 | 8511 | 55 |
| H18 | 10871 | 248 | 7209 | 49 |
| H20A | 9453 | 2305 | 5931 | 41 |
| H20B | 9407 | 4613 | 6708 | 41 |

**5. Analysis method**

5.1 Polarizing microscope (PLM)

PLM analysis was carried out by using an ECLIPSE LV100POL(Nikon, JPN) polarizing microscope. A small amount of sample was spread on a glass slide, and dropwise added with an cedar oil, and the sample was dispersed, and then covered by a cover slip. Subsequently, the sample was placed under the microscope and observed with a 10x objective lens.

5.2 X-ray powder diffraction (XRPD)

An X-ray diffractometer was used to detect the solid sample. The sample was spread on a single crystal silicon sample disc with zero background, and after the sample was gently pressed and spread, the analysis was carried out

according to parameters in Table 12.

Table 12. XRPD test parameters

| | |
|---|---|
| Equipment | Bruker, D8 Advance |
| Light source | Cu Kα ($\lambda$ = 1.5418 Å) |
| Detector | LynxEye XE-T |
| Scanning angle | 3-40° (2θ) |
| Scanning step size | 0.02° (2θ) |
| Scanning speed | 0.1 s/step |
| Light tube voltage/current | 40 KV/40 mA |
| Divergence slit | 0.6 mm |
| Rotation | 30 rpm |
| Sample disc | Sample disc with zero background |

According to the above single crystal structure analysis, the chiral configuration of the chiral isomer A of methyl acetate was the "R" configuration, as shown in FIG. 6. Therefore, in Embodiment 13, when methyl ester was removed by using a sodium hydroxide aqueous solution to obtain a carboxylic acid product, the generated chiral configuration was not changed, so that it could be inferred that the chiral configuration of the chiral isomer A of the product [(7-hydroxy-5-(1,2,3,4-tetrahydronaphthalene-2-yl)-[1,2,4]triazolo[1,5-a]pyridine-8-carbonyl]amino]acetic acid in Embodiment 13 was the "R" configuration.

**Embodiment 19 Determination of chiral configuration of compound of chiral isomer B of methyl acetate in Embodiment 14**

1. Preparation of compound

With reference to the preparation method of the chiral isomer B of [(7-hydroxy-5-(1,2,3,4-tetrahydronaphthalene-2-yl)-[1,2,4]triazolo[1,5-a]pyridine-8-carbonyl)amino]methyl acetate (step 1) in Embodiment 14, the chiral isomer B of methyl acetate was obtained by chiral resolution, and the isomer was the previous compound of the final product in Embodiment 14.

2. Culture of single crystal

A proper amount of sample of the chiral isomer B of methyl acetate was added into a bottle, and gradually added with dimethyl sulfoxide while stirring at room temperature until a medicine liquid was just clear or nearly clear. The mixture was filtered, a filtrate was placed in a clean bottle, and the bottle was added with a cover which was provided with holes, and allowed to stand at room temperature for slow volatilization.

A needle-shaped single crystal was obtained from dimethyl sulfoxide by slow volatilization, as shown in FIG. 7, and used for X-ray single crystal diffraction analysis.

3. Instruments and parameters

Single crystal data of the chiral isomer B of methyl acetate were collected by a Rigaku XtaLAB Synergy DW diffractometer at 180 K by using a Cu Kα ray (λ=1.54184 Å). Diffraction data were subjected to data restoration and absorption correction by using a CrysAlisPro program, and the structure was analyzed through a dual space algorithm by using a SHELXT program. All non-hydrogen atoms were directly located from a Fourier diagram of difference, and hydrogen atoms were filled on parent atoms. The final structure was refined based on an F2 full-matrix least square method by using the SHELXL program.

4. X-ray diffraction analysis

The single crystal structure of the chiral isomer B of methyl acetate belonged to a monoclinic system and a P21 space group, and a molecular formula of single crystal was $C_{20}H_{20}N_4O_4$. There was one molecule of chiral isomer B of

methyl acetate in each asymmetric unit, and each unit cell contained two asymmetric units. An absolute configuration of chiral carbon was "S".

The refined single crystal structure was as shown in FIG. 8, FIG. 9 and FIG. 10. Crystal structure parameters were summarized in Table 13, and details of atomic coordinates, anisotropic displacement parameters, torsion angle, hydrogen bond, bond length and bond angle parameters were summarized in Table 14 to Table 20. An XRPD pattern obtained by experimental determination of the single crystal sample had more peaks than that obtained by calculation, which could be caused by the situation that the solvent was volatilized to be nearly dry after the single crystal test, and other crystal forms were precipitated in the process of solvent volatilization (FIG. 11).

Table 13. Crystal structure parameters of chiral isomer B of methyl acetate

| | |
|---|---|
| Molecular formula | $C_{20}H_{20}N_4O_4$ |
| Molecular weight | 380.40 |
| Temperature/K | 179.99(10) |
| Crystal system | Monocline |
| Space group | $P2_1$ |
| $a$/Å | 13.2287(2) |
| $b$/Å | 5.10690(10) |
| $c$/Å | 13.7391(2) |
| $\alpha$/° | 90 |
| $\beta$/° | 104.3640(10) |
| $\gamma$/° | 90 |
| Unit cell volume/Å$^3$ | 899.17(3) |
| $Z$ | 2 |
| $\rho_{calc}$g/cm$^3$ | 1.405 |
| $\mu$/mm$^{-1}$ | 0.827 |
| $F(000)$ | 400.0 |
| Crystal size/mm$^3$ | 0.29 × 0.03 × 0.02 |
| Diffraction light source | Cu Kα ($\lambda$ = 1.54184 Å) |
| 2$\theta$scope/° of data collection | 6.64 to 145.034 |
| Scope of diffraction index | $-16 \leq h \leq 16$, $-6 \leq k \leq 6$, $-16 \leq l \leq 16$ |
| Collection number of diffraction points | 15381 |
| Number of independent diffraction points | 3395 [$R_{int}$ = 0.0243, $R_{sigma}$= 0.0168] |
| Data/restriction/parameter | 3395/1/255 |
| Goodness of fit based on $F^2$ | 1.067 |
| Final R value [$I$>=2$\sigma$ ($I$)] | $R_1$ = 0.0320, $wR_2$ = 0.0881 |
| Final R value [all data] | $R_1$ = 0.0326, $wR_2$ = 0.0889 |
| Peak/valley/e Å$^{-3}$ of maximum residual electron density | 0.47/-0.22 |
| Flack parameter | -0.02(5) |

Table 14. Atomic coordinates (×10$^4$) and equivalent isotropic shift parameters (Å$^2$×10$^3$) of chiral isomer B of methyl acetate

| Atom | $x$ | $y$ | $z$ | U(eq) |
|---|---|---|---|---|
| O1 | 3838.3(13) | -2686(4) | -49.0(12) | 45.2(4) |
| O2 | 3791.2(15) | 970(5) | 839.5(15) | 65.5(6) |

(continued)

| Atom | x | y | z | U(eq) |
|---|---|---|---|---|
| O3 | 7202.8(12) | -2163(3) | 2559.1(11) | 39.2(4) |
| O4 | 8666.7(11) | -359(3) | 3967.6(11) | 38.4(4) |
| N1 | 5615.2(14) | -252(4) | 2170.6(12) | 35.2(4) |
| N2 | 5282.3(12) | 3932(4) | 3404.3(12) | 33.9(4) |
| N3 | 5896.3(12) | 6691(4) | 4724.6(13) | 33.9(4) |
| N4 | 6661.4(12) | 4979(3) | 4613.1(12) | 29.0(3) |
| C1 | 2832.0(18) | -2032(6) | -709.2(17) | 48.1(6) |
| C2 | 4200.6(17) | -1071(5) | 712.5(15) | 37.8(5) |
| C3 | 5225.7(18) | -2041(5) | 1345.7(15) | 38.4(5) |
| C4 | 6592.5(16) | -444(4) | 2732.7(15) | 31.8(4) |
| C5 | 6943.4(15) | 1429(4) | 3557.1(14) | 29.6(4) |
| C6 | 6280.4(15) | 3347(4) | 3813.0(14) | 29.2(4) |
| C7 | 5108.6(15) | 5935(5) | 3986.9(15) | 35.2(4) |
| C8 | 7676.4(15) | 4946(4) | 5195.7(14) | 29.2(4) |
| C9 | 8322.4(15) | 3151(4) | 4943.3(15) | 31.3(4) |
| C10 | 7968.9(15) | 1369(4) | 4133.4(14) | 30.8(4) |
| C11 | 7944.4(14) | 6904(4) | 6038.2(14) | 29.0(4) |
| C12 | 9122.1(15) | 7132(4) | 6468.9(15) | 32.7(4) |
| C13 | 9390.5(15) | 9063(4) | 7327.3(14) | 30.8(4) |
| C14 | 10381.0(16) | 10213(5) | 7578.5(17) | 39.3(5) |
| C15 | 10653.4(18) | 12001(5) | 8353.9(18) | 45.1(5) |
| C16 | 9942.0(19) | 12652(5) | 8904.1(17) | 46.5(6) |
| C17 | 8963.3(18) | 11500(5) | 8666.0(16) | 41.5(5) |
| C18 | 8675.7(15) | 9699(4) | 7885.1(14) | 32.6(4) |
| C19 | 7585.8(16) | 8562(5) | 7634.9(16) | 38.2(5) |
| C20 | 7425.0(15) | 6285(4) | 6897.7(15) | 34.6(4) |

Table 15. Anisotropic shift parameters (Å$^2$×10$^3$) of chiral isomer B of methyl acetate

| Atom | $U_{11}$ | $U_{22}$ | $U_{33}$ | $U_{23}$ | $U_{13}$ | $U_{12}$ |
|---|---|---|---|---|---|---|
| O1 | 47.6(9) | 45.2(9) | 35.6(7) | -8.7(7) | -3.4(7) | -1.2(7) |
| O2 | 48.8(9) | 76.2(14) | 62.7(11) | -29.4(11) | -2.9(8) | 18.2(10) |
| O3 | 41.9(8) | 38.5(8) | 35.3(7) | -6.4(7) | 6.0(6) | 3.2(7) |
| O4 | 33.2(7) | 40.1(8) | 40.4(8) | -7.0(7) | 6.4(6) | 4.9(7) |
| N1 | 38.0(9) | 36.3(9) | 28.9(8) | -5.3(7) | 3.6(7) | 1.2(8) |
| N2 | 27.7(8) | 41.3(10) | 30.8(8) | -1.1(7) | 3.7(6) | -0.7(7) |
| N3 | 28.7(8) | 37.0(10) | 35.0(8) | -2.1(7) | 5.9(7) | 4.4(7) |
| N4 | 26.7(7) | 30.2(8) | 29.4(8) | -1.2(7) | 5.7(6) | -1.3(7) |
| C1 | 37.7(11) | 63.6(16) | 37.9(11) | -6.0(11) | -0.3(9) | -10.1(11) |
| C2 | 36.5(10) | 45.6(12) | 31.5(10) | -7.5(9) | 8.7(8) | -2.2(9) |

(continued)

| Atom | $U_{11}$ | $U_{22}$ | $U_{33}$ | $U_{23}$ | $U_{13}$ | $U_{12}$ |
|---|---|---|---|---|---|---|
| C3 | 44.5(11) | 35.4(11) | 31.6(9) | -5.4(9) | 2.7(8) | -0.2(9) |
| C4 | 37.1(10) | 30.8(10) | 28.4(9) | 1.6(8) | 9.8(8) | -1.0(8) |
| C5 | 31.2(9) | 29.5(9) | 28.1(8) | 3.0(8) | 7.3(7) | -2.7(8) |
| C6 | 29.0(9) | 32.4(10) | 25.9(8) | 0.7(8) | 6.0(7) | -4.4(7) |
| C7 | 26.9(9) | 41.6(11) | 35.6(10) | -1.8(9) | 5.0(8) | 2.8(8) |
| C8 | 27.3(9) | 29.6(9) | 29.6(9) | 3.0(8) | 4.8(7) | -2.8(8) |
| C9 | 26.7(9) | 32.6(10) | 32.3(9) | 2.0(8) | 3.3(7) | -2.6(8) |
| C10 | 31.2(9) | 29.2(10) | 33.3(9) | 2.5(8) | 10.6(7) | -0.1(8) |
| C11 | 26.0(9) | 28.5(10) | 30.7(9) | 0.2(8) | 3.6(7) | -2.8(7) |
| C12 | 26.1(9) | 34.6(10) | 35.5(10) | -3.6(8) | 4.0(7) | -1.4(8) |
| C13 | 28.5(9) | 29.7(9) | 30.6(9) | 3.5(8) | 0.6(7) | -0.2(8) |
| C14 | 32.1(10) | 41.3(12) | 41.3(11) | -0.9(9) | 2.8(8) | -4.1(9) |
| C15 | 36.3(11) | 46.4(13) | 45.9(12) | -4.9(10) | -2.4(9) | -9.4(10) |
| C16 | 49.5(13) | 45.6(13) | 35.7(10) | -9.7(10) | -6.0(9) | -4.8(11) |
| C17 | 41.5(11) | 49.2(13) | 31.3(10) | -1.8(9) | 4.4(8) | 3.7(10) |
| C18 | 31.6(9) | 35.2(10) | 27.5(9) | 3.3(8) | 0.9(7) | -0.7(8) |
| C19 | 30.8(10) | 49.7(13) | 34.3(10) | -5.2(10) | 8.6(8) | -5.1(9) |
| C20 | 30.4(9) | 38.8(11) | 33.1(9) | 1.8(9) | 5.3(7) | -7.1(9) |

Table 16. Bond length (Å) of chiral isomer B of methyl acetate

| Atom | Atom | Bond length/Å | | Atom | Atom | Bond length/Å |
|---|---|---|---|---|---|---|
| O1 | C1 | 1.452(3) | | C5 | C10 | 1.391(3) |
| O1 | C2 | 1.325(3) | | C8 | C9 | 1.356(3) |
| O2 | C2 | 1.207(3) | | C8 | C11 | 1.504(3) |
| O3 | C4 | 1.255(3) | | C9 | C10 | 1.424(3) |
| O4 | C10 | 1.337(3) | | C11 | C12 | 1.528(2) |
| N1 | C3 | 1.447(3) | | C11 | C20 | 1.539(3) |
| N1 | C4 | 1.335(3) | | C12 | C13 | 1.510(3) |
| N2 | C6 | 1.334(3) | | C13 | C14 | 1.399(3) |
| N2 | C7 | 1.353(3) | | C13 | C18 | 1.395(3) |
| N3 | N4 | 1.375(2) | | C14 | C15 | 1.381(3) |
| N3 | C7 | 1.318(3) | | C15 | C16 | 1.386(4) |
| N4 | C6 | 1.372(3) | | C16 | C17 | 1.386(4) |
| N4 | C8 | 1.382(2) | | C17 | C18 | 1.393(3) |
| C2 | C3 | 1.502(3) | | C18 | C19 | 1.513(3) |
| C4 | C5 | 1.467(3) | | C19 | C20 | 1.522(3) |
| C5 | C6 | 1.416(3) | | | | |

Table 17. Bond length of chiral isomer B of methyl acetate

| Atom | Atom | Atom | Bond angle/° | | Atom | Atom | Atom | Bond angle/° |
|---|---|---|---|---|---|---|---|---|
| C2 | O1 | C1 | 116.49(19) | | C9 | C8 | N4 | 116.31(17) |
| C4 | N1 | C3 | 120.83(18) | | C9 | C8 | C11 | 127.11(16) |
| C6 | N2 | C7 | 102.46(16) | | C8 | C9 | C10 | 121.77(17) |
| C7 | N3 | N4 | 101.04(17) | | O4 | C10 | C5 | 122.32(19) |
| N3 | N4 | C8 | 125.41(16) | | O4 | C10 | C9 | 116.64(17) |
| C6 | N4 | N3 | 109.96(16) | | C5 | C10 | C9 | 121.04(18) |
| C6 | N4 | C8 | 124.60(17) | | C8 | C11 | C12 | 111.84(16) |
| O1 | C2 | C3 | 110.47(19) | | C8 | C11 | C20 | 112.89(16) |
| O2 | C2 | O1 | 124.4(2) | | C12 | C11 | C20 | 108.96(15) |
| O2 | C2 | C3 | 125.0(2) | | C13 | C12 | C11 | 111.83(16) |
| N1 | C3 | C2 | 109.90(19) | | C14 | C13 | C12 | 119.42(18) |
| O3 | C4 | N1 | 121.17(19) | | C18 | C13 | C12 | 121.38(17) |
| O3 | C4 | C5 | 120.50(18) | | C18 | C13 | C14 | 119.19(19) |
| N1 | C4 | C5 | 118.33(18) | | C15 | C14 | C13 | 121.1(2) |
| C6 | C5 | C4 | 123.08(17) | | C14 | C15 | C16 | 119.8(2) |
| C10 | C5 | C4 | 120.14(18) | | C17 | C16 | C15 | 119.4(2) |
| C10 | C5 | C6 | 116.78(18) | | C16 | C17 | C18 | 121.5(2) |
| N2 | C6 | N4 | 109.27(17) | | C13 | C18 | C19 | 121.57(18) |
| N2 | C6 | C5 | 131.26(18) | | C17 | C18 | C13 | 118.95(19) |
| N4 | C6 | C5 | 119.47(17) | | C17 | C18 | C19 | 119.43(19) |
| N3 | C7 | N2 | 117.27(18) | | C18 | C19 | C20 | 114.36(17) |
| N4 | C8 | C11 | 116.58(17) | | C19 | C20 | C11 | 109.77(17) |

Table 18. Torsion angle of chiral isomer B of methyl acetate

| A | B | C | D | Torsion angle/° | | A | B | C | D | Torsion angle/° |
|---|---|---|---|---|---|---|---|---|---|---|
| O1 | C2 | C3 | N1 | 179.68(18) | | C7 | N3 | N4 | C8 | 178.65(18) |
| O2 | C2 | C3 | N1 | 3.8(3) | | C8 | N4 | C6 | N2 | -178.64(18) |
| O3 | C4 | C5 | C6 | -177.55(18) | | C8 | N4 | C6 | C5 | 1.5(3) |
| O3 | C4 | C5 | C10 | 2.2(3) | | C8 | C9 | C10 | O4 | -178.39(19) |
| N1 | C4 | C5 | C6 | 3.0(3) | | C8 | C9 | C10 | C5 | 0.9(3) |
| N1 | C4 | C5 | C10 | -177.25(18) | | C8 | C11 | C12 | C13 | -178.98(16) |
| N3 | N4 | C6 | N2 | -0.6(2) | | C8 | C11 | C20 | C19 | -170.86(17) |
| N3 | N4 | C6 | C5 | 179.56(17) | | C9 | C8 | C11 | C12 | 13.2(3) |
| N3 | N4 | C8 | C9 | -177.93(18) | | C9 | C8 | C11 | C20 | -110.1(2) |
| N3 | N4 | C8 | C11 | 2.0(3) | | C10 | C5 | C6 | N2 | 178.7(2) |
| N4 | N3 | C7 | N2 | -0.5(2) | | C10 | C5 | C6 | N4 | -1.5(3) |
| N4 | C8 | C9 | C10 | -1.0(3) | | C11 | C8 | C9 | C10 | 179.05(18) |
| N4 | C8 | C11 | C12 | -166.68(17) | | C11 | C12 | C13 | C14 | -157.64(19) |
| N4 | C8 | C11 | C20 | 70.0(2) | | C11 | C12 | C13 | C18 | 23.2(3) |

(continued)

| A | B | C | D | Torsion angle/° | | A | B | C | D | Torsion angle/° |
|---|---|---|---|---|---|---|---|---|---|---|
| C1 | O1 | C2 | O2 | -6.0(3) | | C12 | C11 | C20 | C19 | 64.2(2) |
| C1 | O1 | C2 | C3 | 178.15(19) | | C12 | C13 | C14 | C15 | 179.6(2) |
| C3 | N1 | C4 | O3 | 0.5(3) | | C12 | C13 | C18 | C17 | -179.7(2) |
| C3 | N1 | C4 | C5 | 179.86(18) | | C12 | C13 | C18 | C19 | -2.2(3) |
| C4 | N1 | C3 | C2 | -168.23(19) | | C13 | C14 | C15 | C16 | 0.8(4) |
| C4 | C5 | C6 | N2 | -1.6(3) | | C13 | C18 | C19 | C20 | 12.6(3) |
| C4 | C5 | C6 | N4 | 178.17(17) | | C14 | C13 | C18 | C17 | 1.1(3) |
| C4 | C5 | C10 | O4 | 0.0(3) | | C14 | C13 | C18 | C19 | 178.7(2) |
| C4 | C5 | C10 | C9 | -179.34(17) | | C14 | C15 | C16 | C17 | -0.1(4) |
| C6 | N2 | C7 | N3 | 0.2(2) | | C15 | C16 | C17 | C18 | 0.0(4) |
| C6 | N4 | C8 | C9 | -0.2(3) | | C16 | C17 | C18 | C13 | -0.5(3) |
| C6 | N4 | C8 | C11 | 179.72(17) | | C16 | C17 | C18 | C19 | -178.1(2) |
| C6 | C5 | C10 | O4 | 179.68(18) | | C17 | C18 | C19 | C20 | -169.87(19) |
| C6 | C5 | C10 | C9 | 0.4(3) | | C18 | C13 | C14 | C15 | -1.3(3) |
| C7 | N2 | C6 | N4 | 0.3(2) | | C18 | C19 | C20 | C11 | -43.1(2) |
| C7 | N2 | C6 | C5 | -179.9(2) | | C20 | C11 | C12 | C13 | -53.5(2) |
| C7 | N3 | N4 | C6 | 0.6(2) | | | | | | |

Table 19. Hydrogen bond of chiral isomer B of methyl acetate

| D | H | A | d(D-H)/Å | d(H-A)/Å | d(D-A)/Å | D-H-A/° |
|---|---|---|---|---|---|---|
| O4 | H4 | O3 | 0.84 | 1.80 | 2.546(2) | 146.7 |
| N1 | H1 | N2 | 0.88 | 2.12 | 2.829(3) | 137.0 |

Table 20. Hydrogen atom coordinates ($\times 10^4$) and isotropic shift parameters ($Å^2 \times 10^3$) of chiral isomer B of methyl acetate

| Atom | *x* | *y* | *z* | U(eq) |
|---|---|---|---|---|
| H4 | 8383 | -1329 | 3483 | 58 |
| H1 | 5203 | 976 | 2303 | 42 |
| H1A | 2925 | -694 | -1191 | 72 |
| H1B | 2370 | -1358 | -309 | 72 |
| H1C | 2522 | -3604 | -1073 | 72 |
| H3A | 5133 | -3802 | 1612 | 46 |
| H3B | 5736 | -2182 | 929 | 46 |
| H7 | 4446 | 6763 | 3869 | 42 |
| H9 | 9030 | 3079 | 5317 | 38 |
| H11 | 7683 | 8652 | 5757 | 35 |
| H12A | 9408 | 5392 | 6709 | 39 |
| H12B | 9452 | 7699 | 5931 | 39 |
| H14 | 10875 | 9758 | 7209 | 47 |
| H15 | 11326 | 12781 | 8510 | 54 |

(continued)

| Atom | *x* | *y* | *z* | U(eq) |
|---|---|---|---|---|
| H16 | 10124 | 13877 | 9440 | 56 |
| H17 | 8477 | 11949 | 9044 | 50 |
| H19A | 7084 | 9966 | 7349 | 46 |
| H19B | 7425 | 7961 | 8265 | 46 |
| H20A | 6669 | 5980 | 6620 | 41 |
| H20B | 7734 | 4673 | 7250 | 41 |

**5. Analysis method**

5.1 Polarizing microscope (PLM)

PLM analysis was carried out by using an ECLIPSE LV100POL (Nikon, JPN) polarizing microscope. A small amount of sample was spread on a glass slide, and dropwise added with an cedar oil, and the sample was dispersed, and then covered by a cover slip. Subsequently, the sample was placed under the microscope and observed with an objective lens. 5.2 X-ray powder diffraction (XRPD)

An X-ray diffractometer was used to detect the solid sample. The sample was spread on a single crystal silicon sample disc with zero background, and after the sample was gently pressed and spread, the analysis was carried out according to parameters in Table 21.

Table 21. XRPD test parameters

| Equipment | Bruker, D8 Advance |
|---|---|
| Light source | Cu Kα ($\lambda$ = 1.5418 Å) |
| Detector | LynxEye XE-T |
| Scanning angle | 3-40° (2θ) |
| Scanning step size | 0.02° (2θ) |
| Scanning speed | 0.1 s/step |
| Light tube voltage/current | 40 KV/40 mA |
| Divergence slit | 0.6 mm |
| Rotation | 30 rpm |
| Sample disc | Sample disc with zero background |

According to the above single crystal structure analysis, the chiral configuration of the chiral isomer B of methyl acetate was the "S" configuration, as shown in FIG. 12. Therefore, in Embodiment 14, when methyl ester was removed by using a sodium hydroxide aqueous solution to obtain a carboxylic acid product, the generated chiral configuration was not changed, so that it could be inferred that the chiral configuration of the chiral isomer B of the product [(7-hydroxy-5-(1,2,3,4-tetrahydronaphthalene-2-yl)-[1,2,4]triazolo[1,5-a]pyridine-8-carbonyl]amino]acetic acid in Embodiment 14 was the "S" configuration.

**Embodiment 20 Subacute toxicity test of compounds in Embodiments 13 and 14**

**1. Experimental materials**

**1.1 Tested sample**

**Table 22 Information of samples**

| Name of compound | Compound in Embodiment 13 | Compound in Embodiment 14 |
|---|---|---|

### 1.2 Preparation of formulation

A proper amount of the compound in Embodiment 13 and a proper amount of the compound in Embodiment 14 were weighed by a balance and respectively placed into proper glass bottles, then DMSO, Solutol HS-15 and 0.9% physiological saline (in a ratio of v: v: v = 5%: 10%: 85%) were sequentially added into the glass bottles, and vortexed and stirred, and a drug dissolution condition was continuously concerned in the stirring process until the compounds were completely dissolved/suspended. Finally, drug solutions of the compound in Embodiment 13 and the compound in Embodiment 14 with a concentration of 0.67 mg/mL were prepared for current use.

### 2. Experimental animals

The source and number of experimental animals were shown in Table 23. An animal room was well ventilated, equipped with an air-conditioner, kept at a temperature of 20°C and 25°C and a humidity of 40% to 70%, and in light and dark conditions for 12 hours respectively, and the animals could eat and drink freely. SD rats with good physical signs could be selected for this experiment after being adaptively fed for at least 3 days before administration.

**Table 23 Source and number of experimental animals**

| Species | Line, grade | Weeks of age | Number of animals selected |
|---|---|---|---|
| Rat | SD rat, SPF | 160 g to 200 g | 24 (half male and half female) |

### 3. Experimental scheme

### 3.1 Experimental period and dosage setting

Any operation on the experimental animals in this experiment met the requirements of the "Regulations for the Administration of Affairs Concerning Experimental Animals" issued by the Ministry of Science and Technology of the People's Republic of China. In this experiment, a solvent control group, an administration group of compound in Embodiment 13 and an administration group of compound in Embodiment 14 were set up. Administration dosages of the administration group of compound in Embodiment 13 and the administration group of compound in Embodiment 14 were (10 mg/kg) once a day for 14 consecutive days, while the same amount of blank solvent was given to the blank control group, and administration volumes were all 15 mL/kg. After each administration, symptoms and degrees of toxic reaction of the animals were observed. Recovery observation was carried out for 7 days after the last administration. The animals could drink freely during the experiment. Details were shown in Table 24.

**Table 24 Experimental dosage and grouping design**

| Group | Administration dosage (mg/kg) | Concentration (mg/mL) | Administration frequency | Administration volume (mL/kg) | Number of animals (animal) | |
|---|---|---|---|---|---|---|
| | | | | | ♀ | ♂ |
| Blank control group | / | / | Once a day for 14 consecutive days | 15 | 4 | 4 |
| Low-dosage group of compound in Embodiment 13 | 10 | 0.67 | Once a day for 14 consecutive days | 15 | 4 | 4 |
| Low-dosage group of compound in Embodiment 14 | 10 | 0.67 | Once a day for 14 consecutive days | 15 | 4 | 4 |

After weighing, a theoretical administration volume of each SD rat was calculated according to the following

formula. An actual administration dosage and sample collection time of each SD rat should be recorded in a corresponding form in detail.

$$\text{Actual drug administration volume}\,(\text{mL}) = \left(\frac{\text{Dosage}\,(\text{mg/kg})}{\text{Concentration of test solution}\,(\text{mg/mL})}\right) \times \text{Body weight of animal}\,(\text{kg})$$

### 3.2 Experimental observation

During the experiment, experimenters and veterinarians should observe physical signs and health conditions of the experimental animals continuously. Any abnormal manifestations of the animals, such as pain, depression and decreased activity should be recorded in original records of the experiment. If abnormal performances of the experimental animals exceeded the provisions of IACUC's animal welfare documents, the veterinarians could judge whether to stop the experiment and inform the person in charge of the experimental project.

### 3.3 Observation beside cage

During the experiment, all animals were observed beside cages at least twice a day, and observed continuously for 2 hours after the drug administration. During the drug administration, if the animals had serious side effects or died, observation results should be recorded in a corresponding form. Observation indexes comprised: animal appearance, behavior, secretion, excrement, diet, death (number of deaths, time of death, reaction before dying), and the like. If clinical symptoms appeared, it was necessary to increase a number of observations, so as to observe the symptoms, starting time, severity, duration and reversibility of toxic reaction of the animals in detail. If the animals were found dead or dying, the animals should be dissected and observed in time according to the regulations.

### 3.4 Body weight

The animals should be weighed before daily drug administration for 14 consecutive days, and the weights were recorded in a corresponding form. Food intakes of the experimental animals were detected during the experiment.

### 3.5 Clinicopathology

Before the first drug administration and 24 hours after the last drug administration, about 1.8 mL to 2.0 mL of whole blood was collected for hematology and blood biochemistry tests. In addition, a blood sample was collected for a coagulation test 24 hours after the last drug administration. If the animals were abnormal in state during the drug administration, time of hematology, coagulation and blood biochemistry tests was advanced. The animals were fasted for more than 12 hours before blood collection.

About 500μL of whole blood was placed in an EDTA- K2-containing anticoagulant tube for hematological analysis:

☐ White blood cell count (WBC)
☐ Neutrophil differential count (absolute count, NEUT; percentage, %NEUT)
☐ Lymphocyte differential count (absolute count, LYM; percentage, %LYM)
☐ Monocyte differential count (absolute count, MONO; percentage, %MONO)
☐ Eosinophil differential count (absolute count, EOS; percentage, %EOS)
☐ Basophil differential count (absolute count, BASO; percentage, %BASO)
☐ Red blood cell count (RBC)
☐ Hemoglobin (HGB)
☐ Hematocrit (HCT)
☐ Mean corpuscular volume (MCV)
☐ Mean corpusular hemoglobin (MCH)
☐ Mean corpusular hemoglobin concentration (MCHC)
☐ Red blood cell distribution width - coefficient of variation (RDW- CV)
☐ Red blood cell distribution width - standard deviation (RDW-SD)
☐ Platelet count (PLT)
☐ Mean platelet volume (MPV)

☐ Platelet distribution width (PDW)
☐ Plateletcrit (PCT)

About 1.5 mL of whole blood was placed in a separation gel blood collection tube (without anticoagulant) for blood biochemistry analysis:

☐ Alanine Aminotransferase (ALT)
☐ Aspartate Aminotransferase (AST)
☐ Alkaline Phosphatase (ALP)
☐ Albumin (ALB)
☐ Total Cholesterol (TC)
☐ Creatinine (CRE)
☐ UREA
☐ Creatine Phosphokinase (CK)
☐ Triglyceride (TG)
☐ Total Bilirubin (TBIL)
☐ Total Protein (TP)
☐ Aspartate Aminotransferase/Alanine Aminotransferase (AST/ALT)
☐ Globulin (Glo II)
☐ Albumin/Globulin (A/G II)
☐ Electrolytes (K+/ Na+/ Cl-/Ca2+)

**4. Conclusion**

Under the experimental conditions, the compounds in Embodiments 13 and 14 had no obvious gastrointestinal necrosis, and bleeding spots or flatulence in a black stomach part, and toxicities of the compounds were lower than that of Enarodustat, so that the compounds had good safety.
Those described above are only preferred embodiments of the present invention, and are not intended to limit the present invention. Any modifications, equivalent substitutions and improvements made without departing from the spirit and principle of the present invention shall fall within the scope of protection of the present invention.

## Claims

1. A compound as shown in formula (I) or a stereoisomer, geometric isomer, tautomer, nitrogen oxide, hydrate, solvate, pharmaceutically acceptable salt or prodrug thereof:

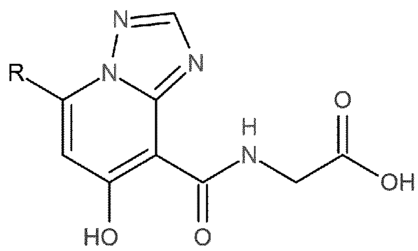

(I)

wherein,

R is a unit of the following atom or group:

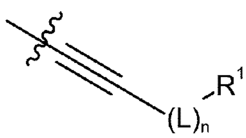 , 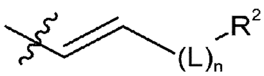 , 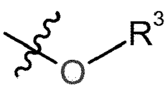 or 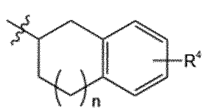 ;

L is $-CH_2-$ or $-CH_2O-$;
a numerical value of n is an integer between 0 and 2; and
$R^1$, $R^2$ and $R^3$ are independently substituted or unsubstituted alkyl, cycloalkyl, aryl or heterocyclyl; and $R^4$ is hydrogen, substituted or unsubstituted alkyl, cycloalkyl, alkoxy, aryl or heterocyclyl.

**2.** The compound or the stereoisomer, geometric isomer, tautomer, nitrogen oxide, hydrate, solvate, pharmaceutically acceptable salt or prodrug thereof according to claim 1, wherein the aryl comprises a heteroaromatic ring.

**3.** The compound or the stereoisomer, geometric isomer, tautomer, nitrogen oxide, hydrate, solvate, pharmaceutically acceptable salt or prodrug thereof according to claim 1, wherein,

L is $-CH_2-$ or $-CH_2O-$;
the numerical value of n is 0 or 1;
$R^1$ is substituted or unsubstituted $C_{1-4}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;
$R^2$ is substituted or unsubstituted $C_{1-4}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;
$R^3$ is substituted or unsubstituted $C_{1-10}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl; and
$R^4$ is hydrogen, substituted or unsubstituted $C_{1-10}$ alkyl, $C_{1-10}$ alkoxy, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;

**4.** The compound or the stereoisomer, geometric isomer, tautomer, nitrogen oxide, hydrate, solvate, pharmaceutically acceptable salt or prodrug thereof according to claim 2, wherein,

L is $-CH_2-$ or $-CH_2O-$;
the numerical value of n is 0 or 1;
$R^1$ is substituted or unsubstituted phenyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;
$R^2$ is substituted or unsubstituted phenyl, and the substituent is at least one of $C_{1-4}$ alkyl, $C_{1-4}$ alkoxy, $C_{1-4}$ halogenated alkyl, halogen, cyano, phenyl, oxygen- and/or nitrogen-containing 5-6 membered aromatic heterocyclyl, and oxygen- and/or nitrogen-containing 5-6 membered heterocyclyl;
$R^3$ is substituted or unsubstituted $C_{1-10}$ alkyl, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, halogen, oxygen- and/or nitrogen-containing 5-6 membered aromatic heterocyclyl, and oxygen- and/or nitrogen-containing 5-6 membered heterocyclyl; and
$R^4$ is hydrogen, substituted or unsubstituted $C_{1-10}$ alkyl, $C_{1-10}$ alkoxy, phenyl, oxygen- and/or nitrogen-containing 5-6 membered aromatic heterocyclyl, or oxygen- and/or nitrogen-containing 5-6 membered heterocyclyl, and the substituent is at least one of $C_{1-4}$ alkyl, $C_{1-4}$ alkoxy, halogen, oxygen- and/or nitrogen-containing 5-6 membered aromatic heterocyclyl, and oxygen- and/or nitrogen-containing 5-6 membered heterocyclyl.

**5.** The compound or the stereoisomer, geometric isomer, tautomer, nitrogen oxide, hydrate, solvate, pharmaceutically acceptable salt or prodrug thereof according to claim 3, wherein,

the numerical value of n is 0 or 1;
$R^1$ is substituted or unsubstituted phenyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered

aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^2$ is substituted or unsubstituted phenyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^3$ is substituted or unsubstituted $C_{1-5}$ alkyl, phenyl, oxygen- and/or nitrogen-containing 5-6 membered aromatic heterocyclyl, or oxygen- and/or nitrogen-containing 5-6 membered heterocyclyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^4$ is hydrogen, substituted or unsubstituted $C_{1-5}$ alkyl, $C_{1-5}$ alkoxy, phenyl, oxygen- and/or nitrogen-containing 5-6 membered aromatic heterocyclyl, or oxygen- and/or nitrogen-containing 5-6 membered heterocyclyl, and the substituent is $C_{1-4}$ alkyl, $C_{1-4}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-4}$ alkyl, $C_{1-4}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

preferably,

the numerical value of n is 0 or 1;

$R^1$ is substituted or unsubstituted phenyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted;

$R^2$ is substituted or unsubstituted phenyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^3$ is substituted or unsubstituted $C_{1-5}$ alkyl, phenyl, oxygen- and/or nitrogen-containing 5-6 membered aromatic heterocyclyl, or oxygen- and/or nitrogen-containing 5-6 membered heterocyclyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl;

and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^4$ is hydrogen, substituted or unsubstituted $C_{1-5}$ alkyl, $C_{1-5}$ alkoxy, phenyl, oxygen- and/or nitrogen-containing 5-6 membered aromatic heterocyclyl, or oxygen- and/or nitrogen-containing 5-6 membered heterocyclyl, and the substituent is $C_{1-3}$ alkyl, $C_{1-3}$ alkoxy, halogen, oxygen- and/or nitrogen-containing 5-6 membered aromatic heterocyclyl, or oxygen- and/or nitrogen-containing 5-6 membered heterocyclyl; and preferably, the $C_{1-3}$ alkyl, $C_{1-3}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure.

**6.** The compound or the stereoisomer, geometric isomer, tautomer, nitrogen oxide, hydrate, solvate, pharmaceutically acceptable salt or prodrug thereof according to claim 5, wherein,

the numerical value of n is 0 or 1;

$R^1$ is substituted or unsubstituted phenyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered

heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the F, Cl, Br or I is ortho-substituted, meta-substituted or para-substituted relative to the parent nucleus structure;

$R^2$ is substituted or unsubstituted phenyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the F, Cl, Br or I is ortho-substituted, meta-substituted or para-substituted relative to the parent nucleus structure;

$R^3$ is substituted or unsubstituted $C_{1-4}$ alkyl, phenyl, oxygen- and/or nitrogen-containing 5-6 membered aromatic heterocyclyl, or oxygen- and/or nitrogen-containing 5-6 membered heterocyclyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

$R^4$ is hydrogen, substituted or unsubstituted $C_{1-4}$ alkyl, $C_{1-4}$ alkoxy, phenyl, oxygen- and/or nitrogen-containing 5-6 membered aromatic heterocyclyl, or oxygen- and/or nitrogen-containing 5-6 membered heterocyclyl, and the substituent is $C_{1-2}$ alkyl, $C_{1-2}$ alkoxy, F, Cl, Br, I, oxygen- and/or nitrogen-containing 5-6 membered aromatic heterocyclyl, or oxygen- and/or nitrogen-containing 5-6 membered heterocyclyl; and preferably, the $C_{1-2}$ alkyl, $C_{1-2}$ alkoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure;

preferably,

the numerical value of n is 0 or 1;

$R^1$ is substituted or unsubstituted phenyl, and the substituent is methyl, methoxy, F, Cl, oxygen- and/or nitrogen-containing 5-6 membered aromatic heterocyclyl, or oxygen- and/or nitrogen-containing 5-6 membered heterocyclyl; and preferably, the methyl, methoxy, or oxygen- and/or nitrogen-containing 5-6 membered aromatic heterocyclyl is para-substituted relative to a parent nucleus structure, and the F or Cl is ortho-substituted, meta-substituted or para-substituted relative to the parent nucleus structure;

$R^2$ is substituted or unsubstituted phenyl, and the substituent is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the F or Cl is ortho-substituted, meta-substituted or para-substituted relative to the parent nucleus structure;

$R^3$ is substituted or unsubstituted $C_{1-3}$ alkyl, phenyl, or oxygen- and/or nitrogen-containing 5-6 membered aromatic heterocyclyl, and the substituent of the substituted phenyl is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure;

$R^4$ is hydrogen, substituted or unsubstituted $C_{1-3}$ alkyl, $C_{1-3}$ alkoxy, phenyl, or oxygen- and/or nitrogen-containing 5-6 membered aromatic heterocyclyl, and the substituent of the substituted phenyl is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure;

further preferably,

the numerical value of n is 0 or 1;

$R^1$ is substituted or unsubstituted phenyl, and the substituent is methyl or oxygen- and/or nitrogen-containing 5-6 membered aromatic heterocyclyl; and preferably, the methyl or oxygen- and/or nitrogen-containing 5-6 membered aromatic heterocyclyl is para-substituted relative to a parent nucleus structure;

$R^2$ is substituted or unsubstituted phenyl, and the substituent is methyl or oxygen- and/or nitrogen-containing 5-6 membered aromatic heterocyclyl; and preferably, the methyl or oxygen- and/or nitrogen-containing 5-6 membered aromatic heterocyclyl is para-substituted relative to a parent nucleus structure;

$R^3$ is substituted or unsubstituted $C_{1-3}$ alkyl, phenyl, or oxygen- and/or nitrogen-containing 5-6 membered aromatic heterocyclyl, and the substituent is methyl, methoxy, F, Cl, or oxygen- and/or nitrogen-containing 5-6

membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure;

$R^4$ is hydrogen, substituted or unsubstituted $C_{1-3}$ alkyl, $C_{1-3}$ alkoxy, phenyl, or oxygen- and/or nitrogen-containing 5-6 membered aromatic heterocyclyl, and the substituent is methyl, methoxy, F, Cl, or oxygen- and/or nitrogen-containing 5-6 membered aromatic heterocyclyl; and preferably, the methyl, methoxy, or oxygen- and/or nitrogen-containing 5-6 membered aromatic heterocyclyl is para-substituted relative to a parent nucleus structure, and the halogen is ortho-substituted, meta-substituted or para-substituted relative to the parent nucleus structure;

further preferably,

the numerical value of n is 0 or 1;

$R^1$ is phenyl;

$R^2$ is phenyl;

$R^3$ is substituted or unsubstituted $C_{1-3}$ alkyl, phenyl, or oxygen- and/or nitrogen-containing 5-6 membered aromatic heterocyclyl, and the substituent is methyl, methoxy, halogen, or oxygen- and/or nitrogen-containing 5-6 membered aromatic heterocyclyl; and preferably, the methyl, methoxy, oxygen- and/or nitrogen-containing 5-6 membered aromatic heterocyclyl, or oxygen- and/or nitrogen-containing 5-6 membered heterocyclyl is para-substituted relative to a parent nucleus structure, and the halogen is ortho-substituted, meta-substituted or para-substituted relative to the parent nucleus structure; and

$R^4$ is hydrogen.

**7.** The compound or the stereoisomer, geometric isomer, tautomer, nitrogen oxide, hydrate, solvate, pharmaceutically acceptable salt or prodrug thereof according to claim 6, wherein the compound is selected from the following structures:

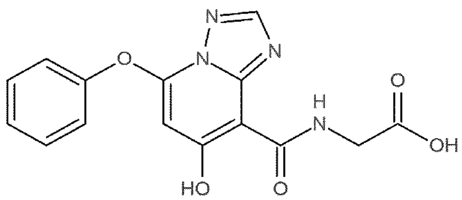
,
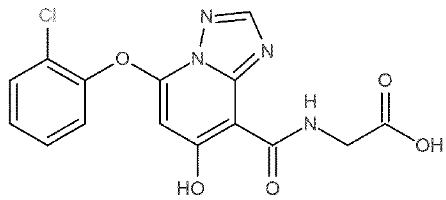
,
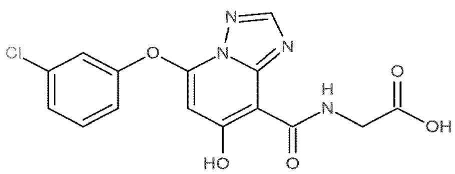
,
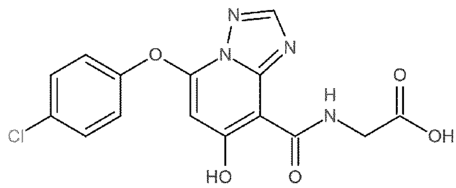
,
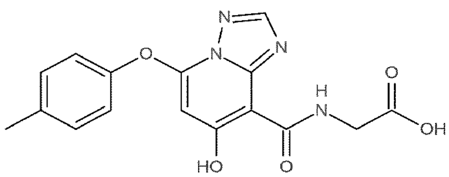
,
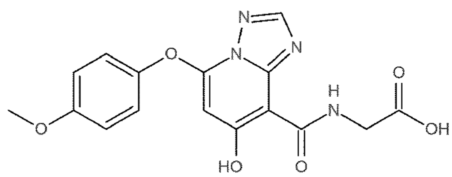
,
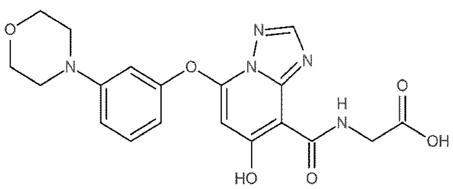
,
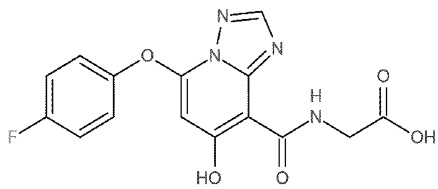
,

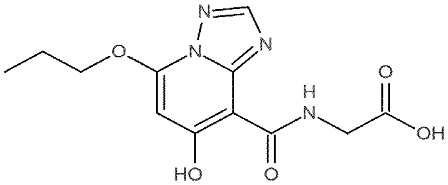,
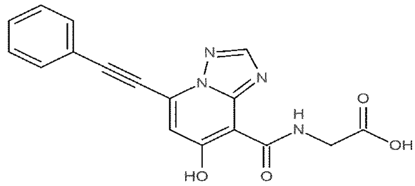,

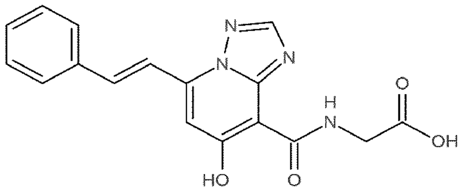 and 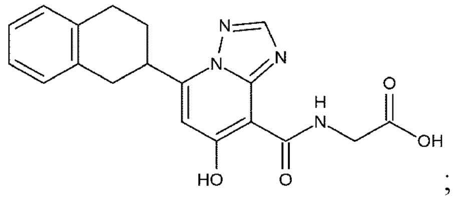;

preferably, the compound is selected from the following structures:

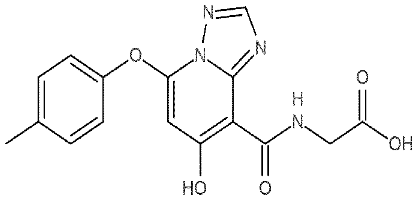,
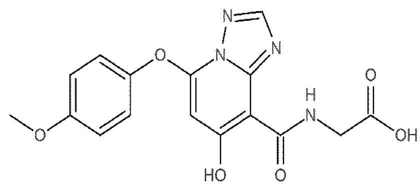,

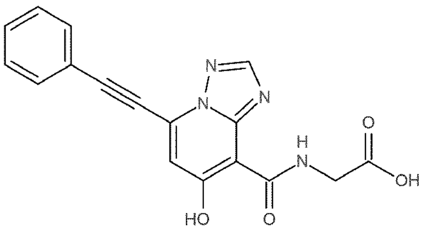,
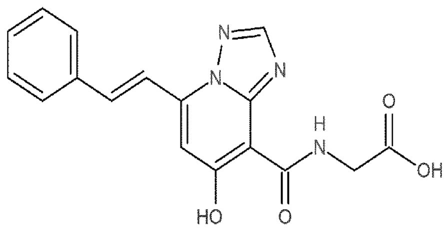

and

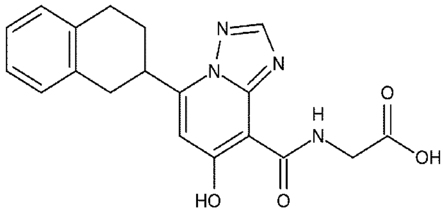;

further preferably, the compound is selected from the following structures:

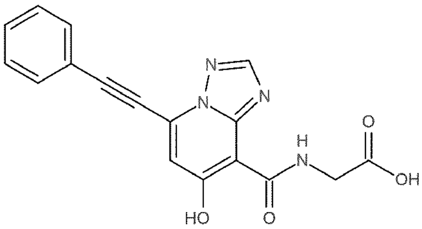,
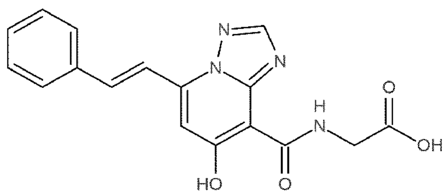

and

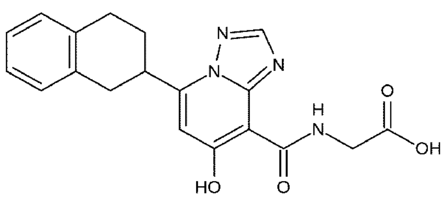
;

further preferably, the compound is selected from the following structures:

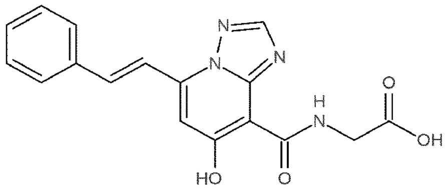
and
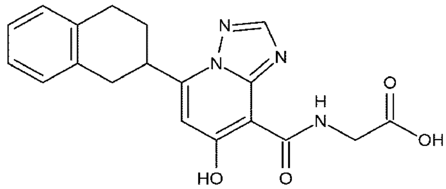
.

**8.** A preparation method of the compound according to any one of claims 1 to 7, comprising the following four synthetic routes:

route 1:

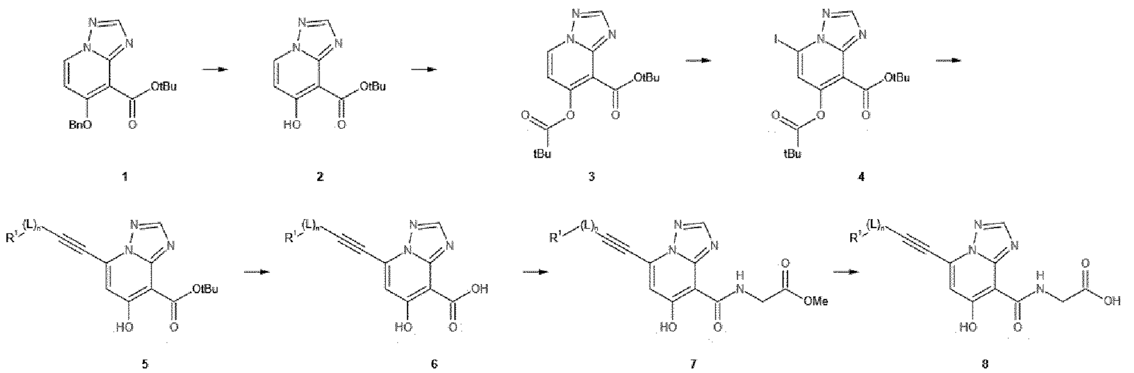
1 2 3 4
5 6 7 8

route 2:

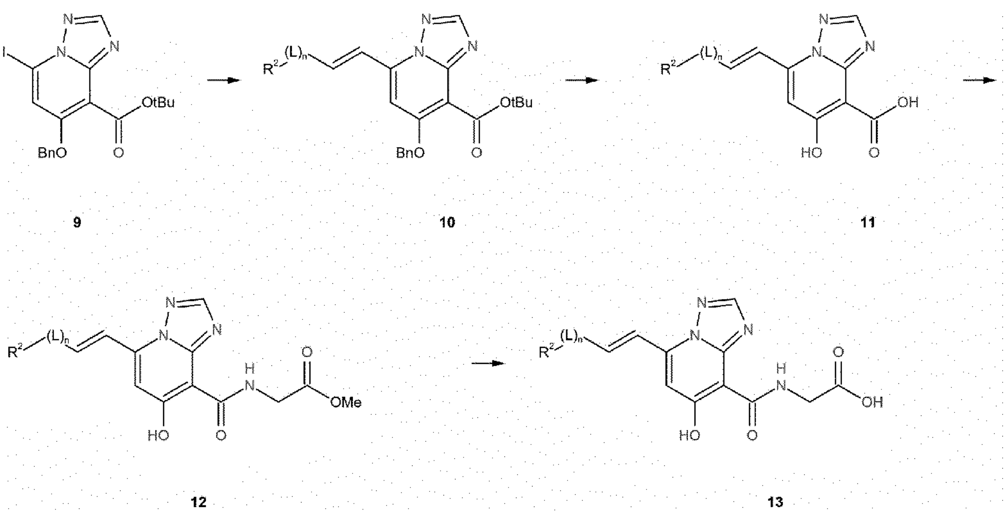
9 10 11
12 13

route 3:

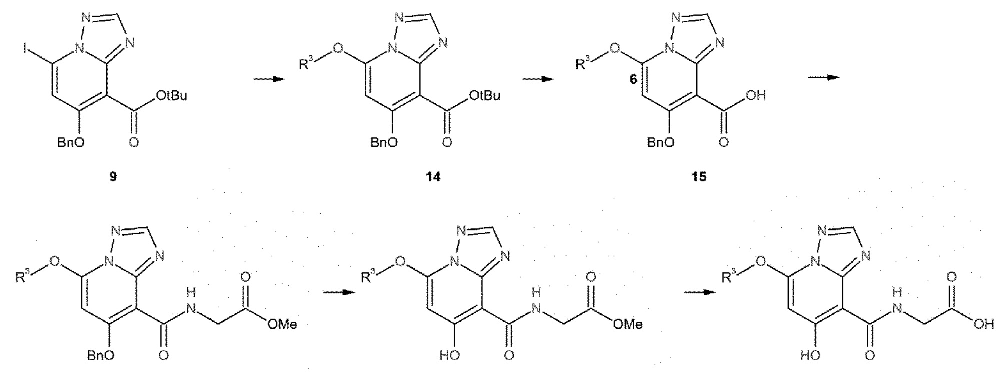

9 14 15

16 17 18

route 4:

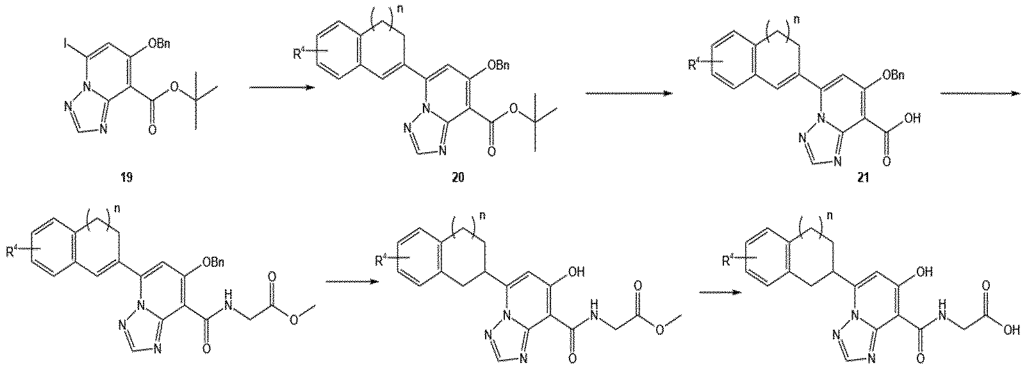

19 20 21

22 23 24

wherein, $R^1$, $R^2$ and $R^3$ have the same definitions as those of the cited claims.

**9.** A pharmaceutical composition, containing a compound as shown in formula (I) or a stereoisomer, geometric isomer, tautomer, nitrogen oxide, hydrate, solvate, pharmaceutically acceptable salt or prodrug thereof, and one or more of a medicinal carrier, a diluent and an excipient.

**10.** A use of the compound as shown in formula (I) or a stereoisomer, geometric isomer, tautomer, nitrogen oxide, hydrate, solvate, pharmaceutically acceptable salt or prodrug thereof, or the pharmaceutical composition above in preparation of a drug for treating a prolyl hydroxylase domain inhibition-mediated disease; preferably, the prolyl hydroxylase domain inhibition-mediated disease comprising anemia, ischemia and hypoxia; and further preferably, the prolyl hydroxylase domain inhibition-mediated disease being renal anemia.

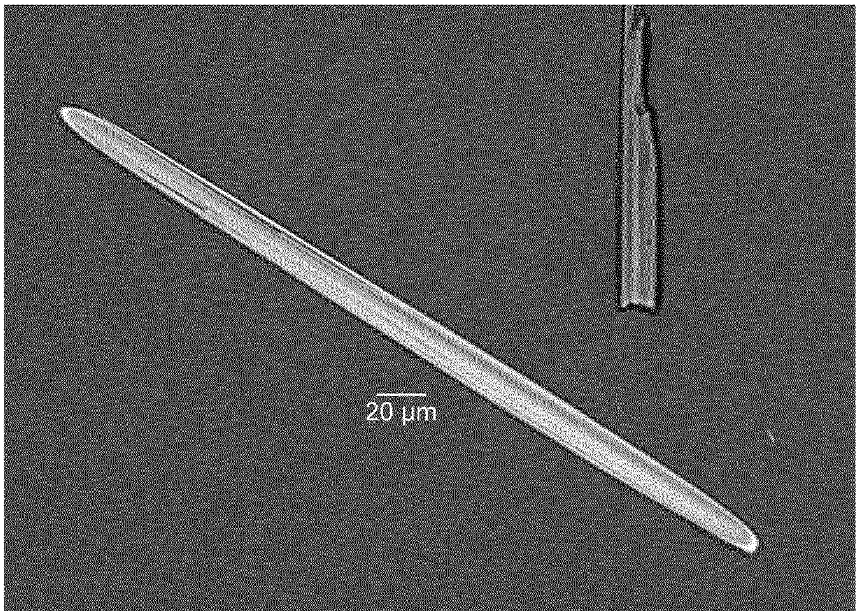


FIG. 1

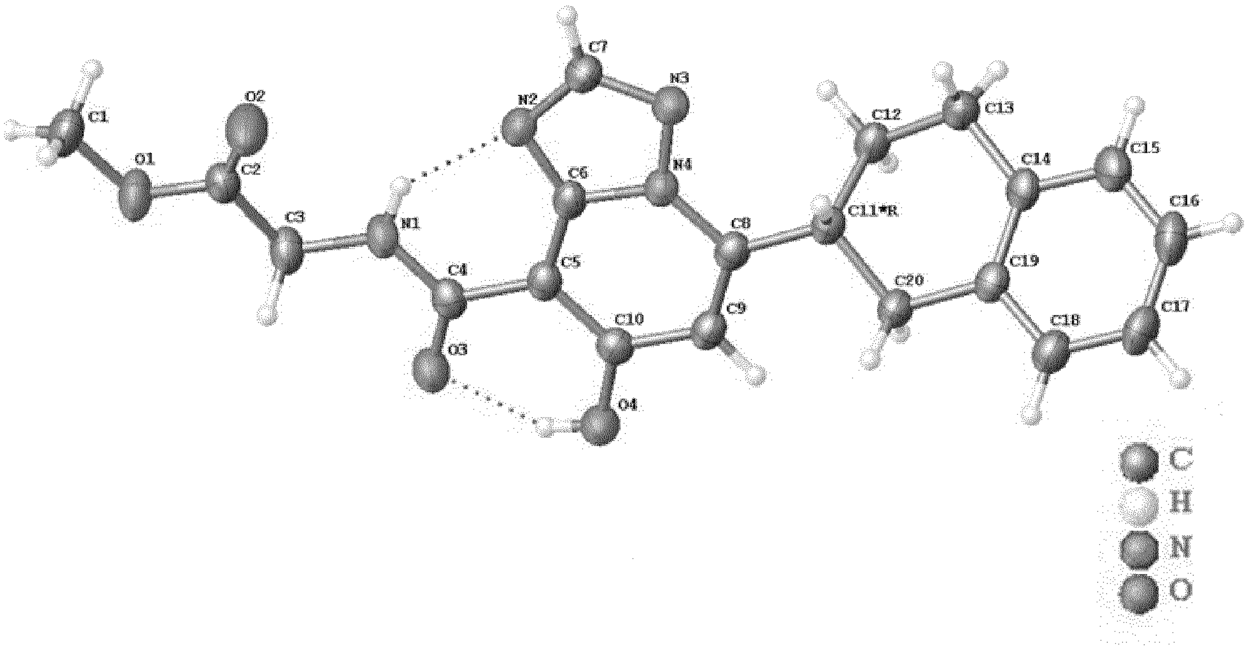

FIG. 2

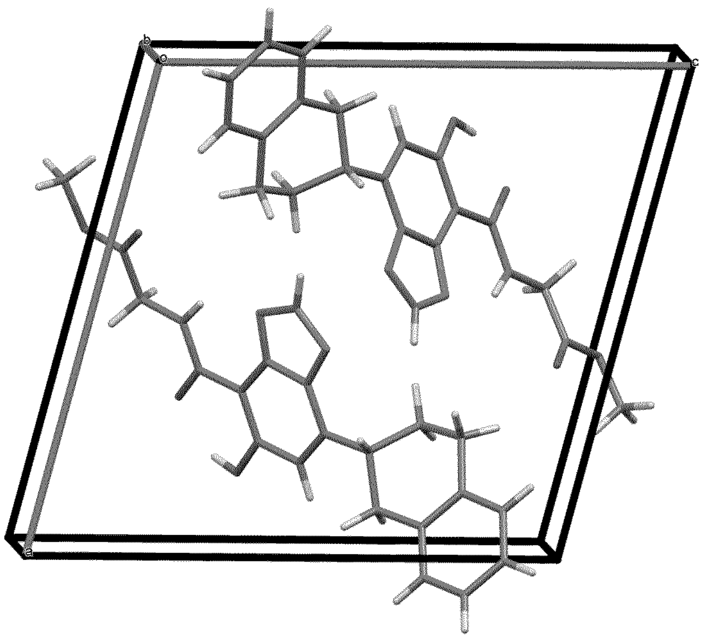

FIG. 3

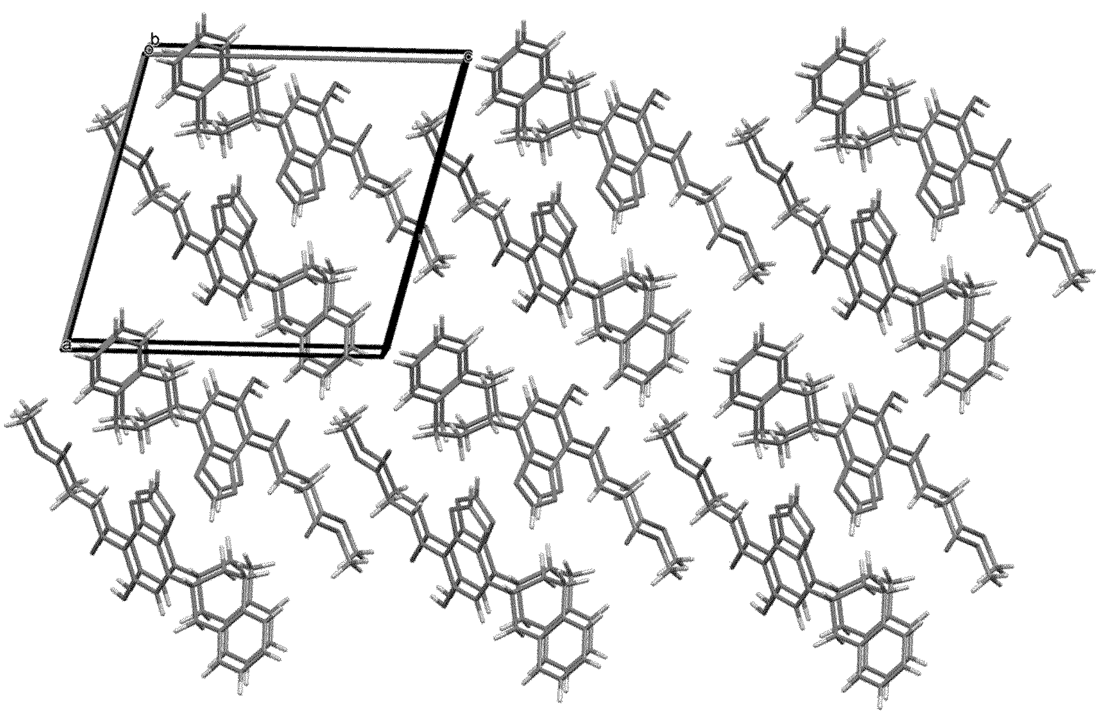

FIG. 4

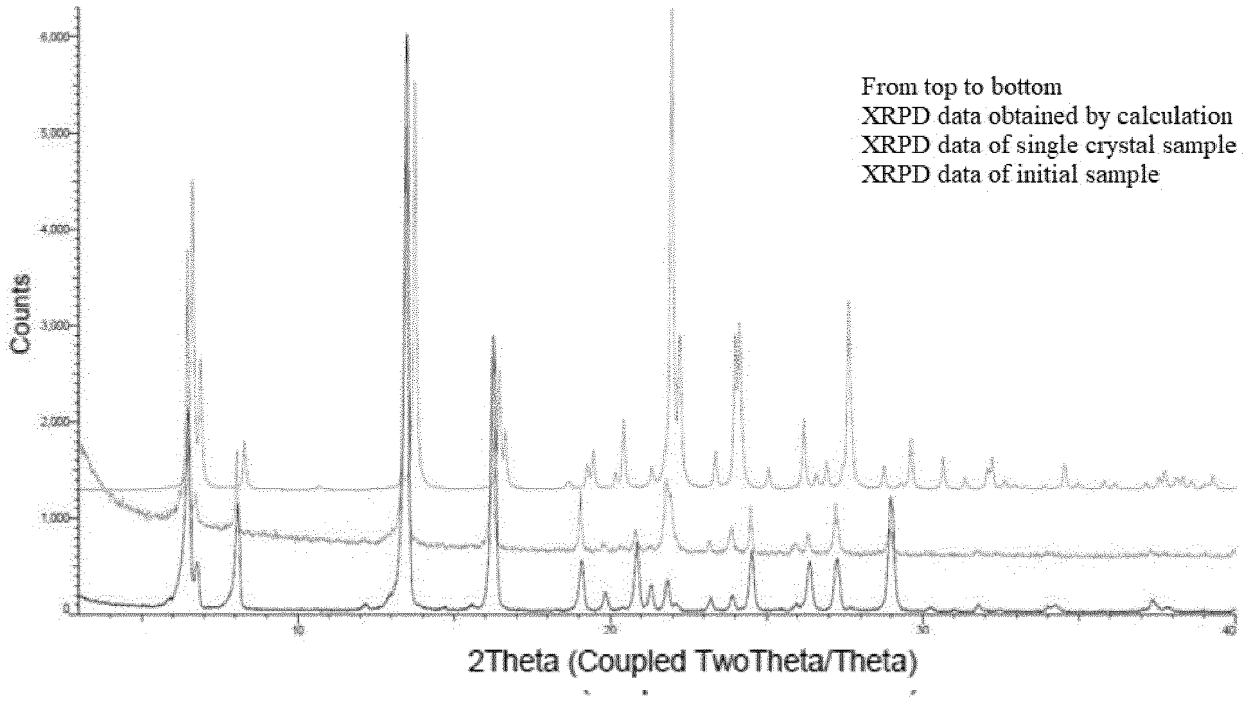


FIG. 5

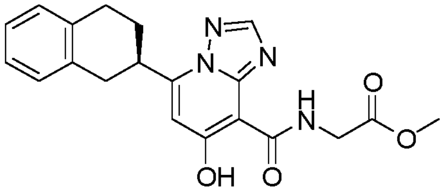

Chemical Formula: $C_{20}H_{20}N_4O_4$
Molecular Weight: 380.40

FIG. 6

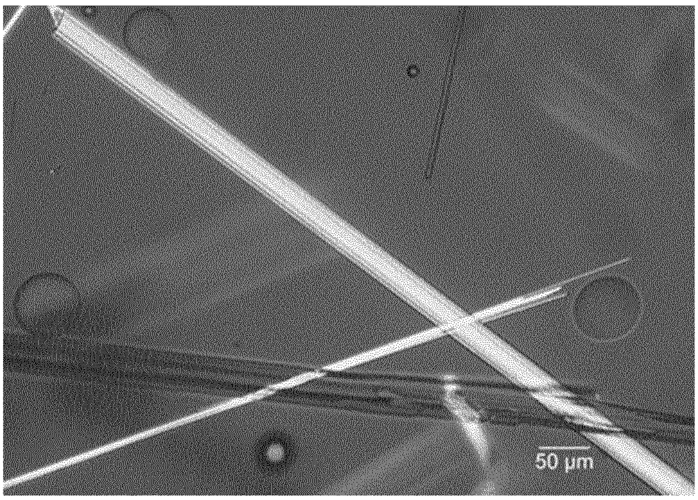


FIG. 7

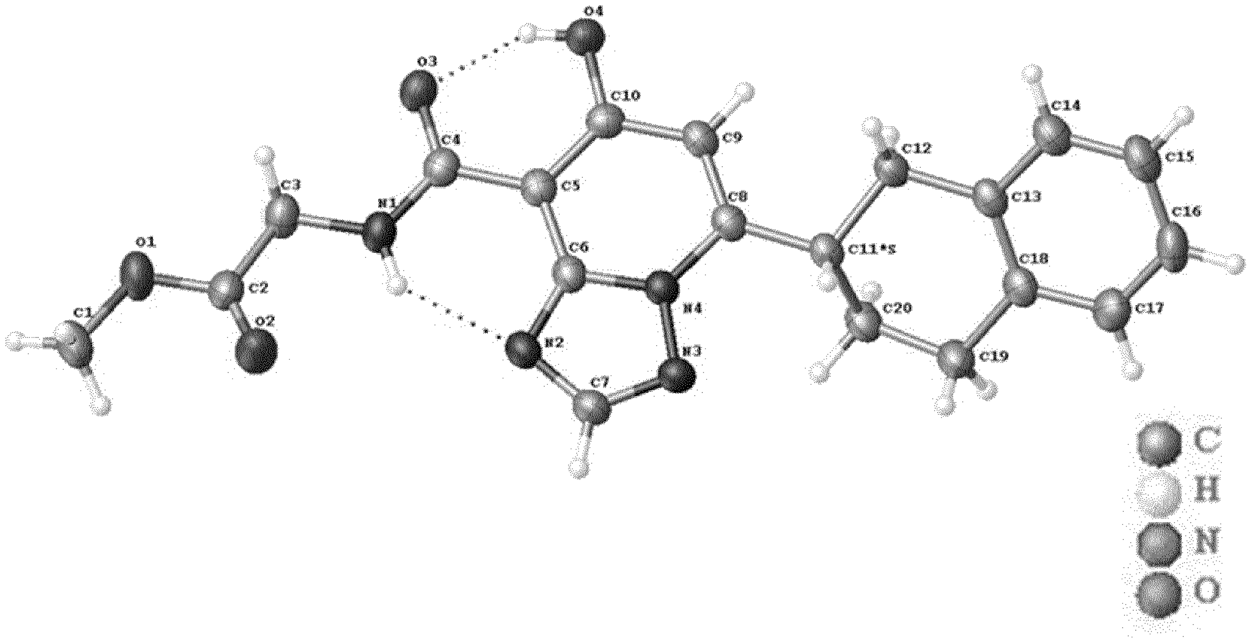

FIG. 8

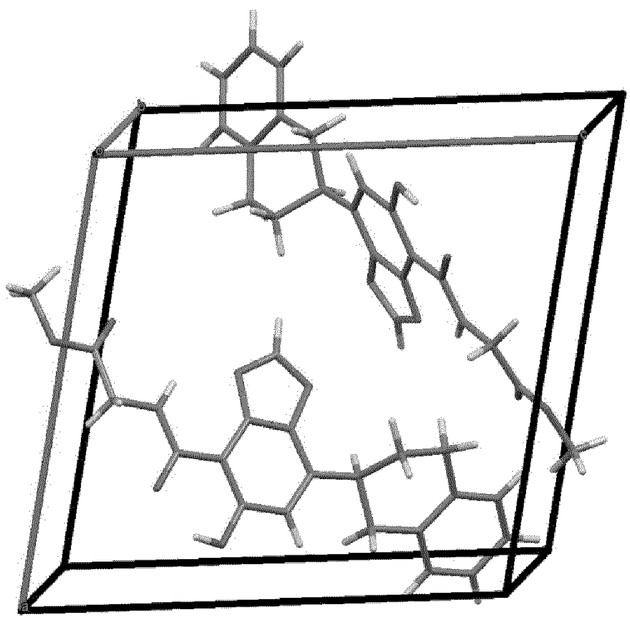

FIG. 9

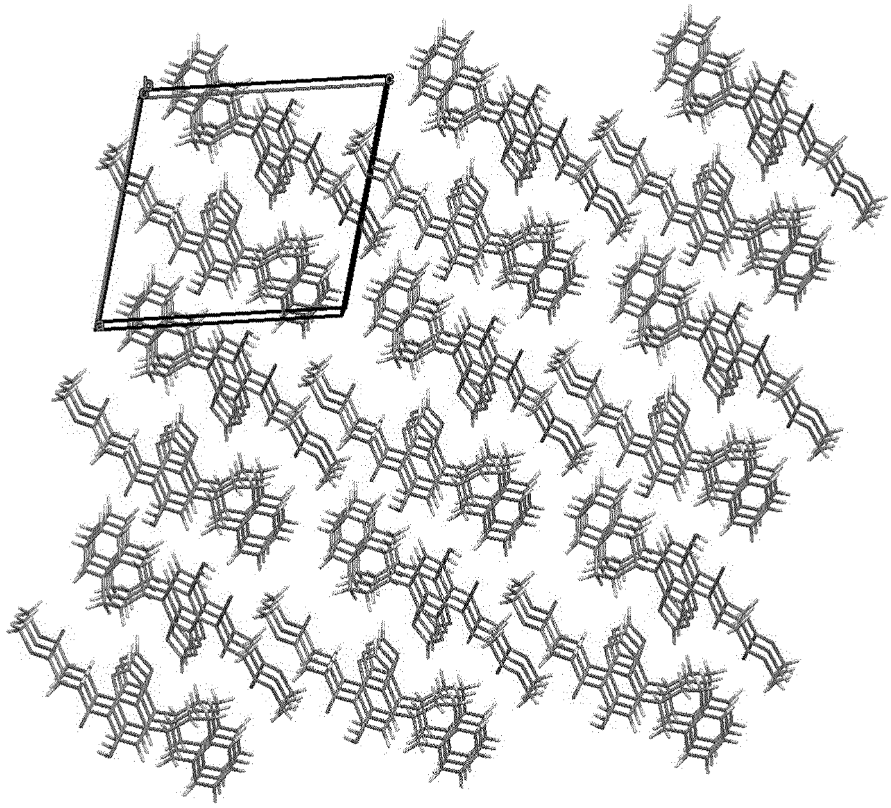

FIG. 10

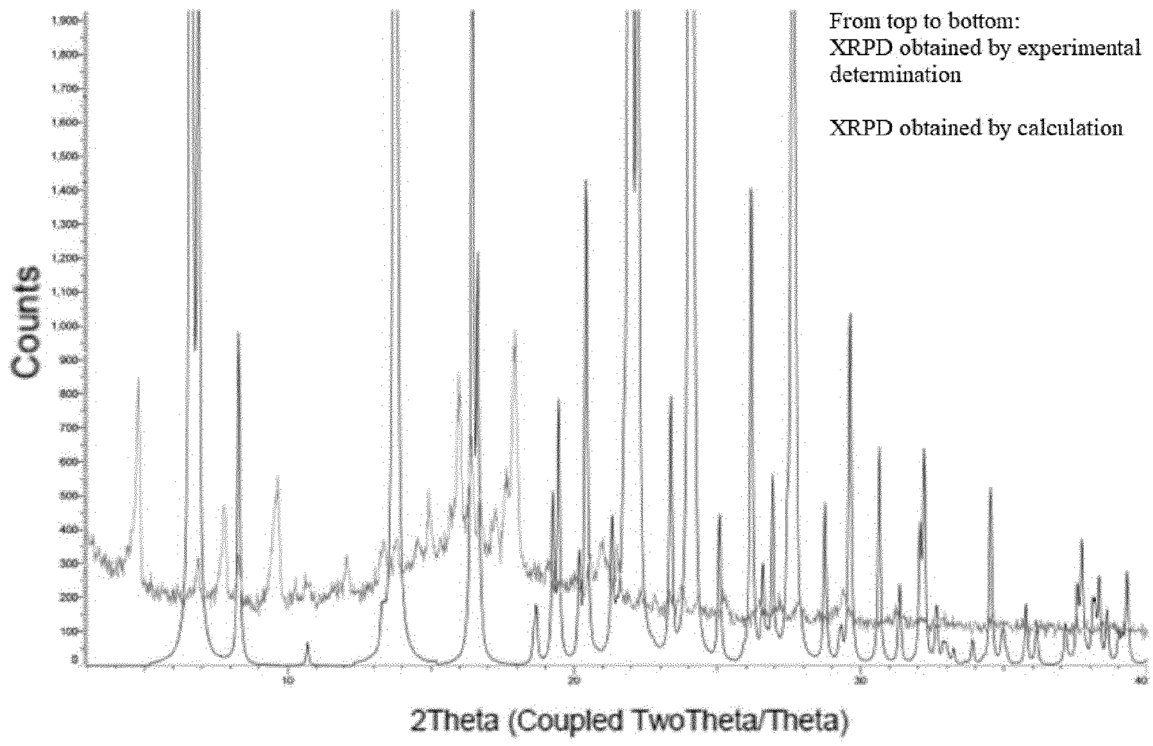
From top to bottom:
XRPD obtained by experimental determination
XRPD obtained by calculation
Counts
2Theta (Coupled TwoTheta/Theta)

FIG. 11

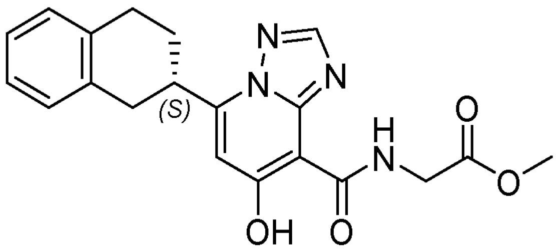

INI-B

Chemical Formula: $C_{20}H_{20}N_4O_4$
Molecular Weight: 380.40

FIG. 12

**INTERNATIONAL SEARCH REPORT**

International application No. **PCT/CN2023/119468**

**A. CLASSIFICATION OF SUBJECT MATTER**

C07D 471/04(2006.01)i; A61P 7/06(2006.01)i; A61P 9/10(2006.01)i; A61K 31/4375(2006.01)i; A61K 31/5377(2006.01)i

According to International Patent Classification (IPC) or to both national classification and IPC

**B. FIELDS SEARCHED**

Minimum documentation searched (classification system followed by classification symbols)

IPC: C07D A61P A61K

Documentation searched other than minimum documentation to the extent that such documents are included in the fields searched

Electronic data base consulted during the international search (name of data base and, where practicable, search terms used)

CNABS, CNTXT, CNKI, 万方数据库, WANFANG DATABASE, 百度学术, BAIDU SCHOLAR, VEN, ENTXT, STN: 苏中药业, 吡啶并三唑, 甘氨酸, 脯氨酰羟化酶, 脯氨酸羟化酶, 缺氧诱导性因子, 促红细胞生成素; pyridino-triazole, glycin, prolyl hydroxylase domain, Proline Hydroxylase domain, hypoxia inducible factor, HIF, Erythropoietin, EPO.

**C. DOCUMENTS CONSIDERED TO BE RELEVANT**

| Category* | Citation of document, with indication, where appropriate, of the relevant passages | Relevant to claim No. |
|---|---|---|
| PX | CN 116496269 A (SZYY GROUP PHARMACEUTICAL LIMITED et al.) 28 July 2023 (2023-07-28)<br>claims 1-10 | 1-10 |
| X | WO 2016104451 A1 (FUJI YAKUHIN CO., LTD.) 30 June 2016 (2016-06-30)<br>pages 5-7, 13-16, 82, 84-85, 87-95, and 112-116; examples 1 to 3, and embodiments 5, 20-25, 28, 41, 44, 50-55, 61-63, 68, 71-74, 76, 81-84, 86-90, and 201-227 | 1-10 |
| X | 刘思凡 等 (LIU, Sifan et al.). "三氮唑并吡啶类脯氨酸羟化酶抑制剂的设计合成研究 (Design and Synthesis of Proline Hydroxylase Domain Inhibitor)"<br>*合成化学研究 (Non-official translation: Synthetic Chemistry Research),*<br>Vol. 6, No. 1, 06 February 2018 (2018-02-06),<br>ISSN: 2332-7960,<br>pages 1-7 | 1-10 |
| X | . "RN 2201926-56-7"<br>*STN Registry,* 29 March 2018 (2018-03-29),<br>page 1 | 1-2 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2023** | **17 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/119468**

**C. DOCUMENTS CONSIDERED TO BE RELEVANT**

| Category* | Citation of document, with indication, where appropriate, of the relevant passages | Relevant to claim No. |
|---|---|---|
| A | CN 102471337 A (JAPAN TOBACCO INC.) 23 May 2012 (2012-05-23) entire document | 1-10 |
| A | WO 2022150623 A1 (AKEBIA THERAPEUTICS INC.) 14 July 2022 (2022-07-14) entire document | 1-10 |
| A | US 2010303928 A1 (FIBROGEN, INC.) 02 December 2010 (2010-12-02) entire document | 1-10 |
| A | CN 101460497 A (FIBROGEN, INC.) 17 June 2009 (2009-06-17) entire document | 1-10 |

Form PCT/ISA/210 (second sheet) (July 2022)

**INTERNATIONAL SEARCH REPORT**
**Information on patent family members**

International application No.
**PCT/CN2023/119468**

| Patent document cited in search report | | | Publication date (day/month/year) | Patent family member(s) | | | Publication date (day/month/year) |
|---|---|---|---|---|---|---|---|
| CN | 116496269 | A | 28 July 2023 | None | | | |
| WO | 2016104451 | A1 | 30 June 2016 | TW | 201625614 | A | 16 July 2016 |
| | | | | JP | 2018039733 | A | 15 March 2018 |
| | | | | AR | 103231 | A1 | 26 April 2017 |
| CN | 102471337 | A | 23 May 2012 | EP | 2746282 | A1 | 25 June 2014 |
| | | | | JP | 2015003933 | A | 08 January 2015 |
| | | | | JP | 2020111612 | A | 27 July 2020 |
| | | | | JP | 2012144571 | A | 02 August 2012 |
| | | | | JP | 2022126843 | A | 30 August 2022 |
| | | | | CO | 6430426 | A2 | 30 April 2012 |
| | | | | US | 2011077267 | A1 | 31 March 2011 |
| | | | | US | 8283465 | B2 | 09 October 2012 |
| | | | | KR | 20170018105 | A | 15 February 2017 |
| | | | | KR | 101850661 | B1 | 19 April 2018 |
| | | | | US | 2016145254 | A1 | 26 May 2016 |
| | | | | JP | 2019019144 | A | 07 February 2019 |
| | | | | HK | 1168346 | A1 | 28 December 2012 |
| | | | | EP | 3594213 | A1 | 15 January 2020 |
| | | | | ES | 2477968 | T3 | 18 July 2014 |
| | | | | ES | 2477968 | T9 | 13 November 2014 |
| | | | | JP | 2016040321 | A | 24 March 2016 |
| | | | | TW | 201105671 | A | 16 February 2011 |
| | | | | TWI | 485150 | B | 21 May 2015 |
| | | | | RU | 2012105467 | A | 27 August 2013 |
| | | | | RU | 2538963 | C2 | 10 January 2015 |
| | | | | WO | 2011007856 | A1 | 20 January 2011 |
| | | | | EP | 2455381 | A1 | 23 May 2012 |
| | | | | EP | 2455381 | B1 | 28 May 2014 |
| | | | | KR | 20120051704 | A | 22 May 2012 |
| | | | | KR | 101706803 | B1 | 14 February 2017 |
| | | | | AU | 2010271732 | A1 | 08 March 2012 |
| | | | | AU | 2010271732 | B2 | 25 August 2016 |
| | | | | DK | 2455381 | T3 | 21 July 2014 |
| | | | | DK | 2455381 | T5 | 09 February 2015 |
| | | | | JP | 2017214404 | A | 07 December 2017 |
| | | | | JP | 6434575 | B2 | 05 December 2018 |
| | | | | US | 2013096155 | A1 | 18 April 2013 |
| | | | | KR | 20180042443 | A | 25 April 2018 |
| | | | | CA | 2768505 | A1 | 20 January 2011 |
| | | | | CA | 2768505 | C | 12 June 2018 |
| | | | | JP | 2011037841 | A | 24 February 2011 |
| | | | | JP | 5021797 | B2 | 12 September 2012 |
| WO | 2022150623 | A1 | 14 July 2022 | TW | 202237112 | A | 01 October 2022 |
| US | 2010303928 | A1 | 02 December 2010 | HK | 1148284 | A1 | 02 September 2011 |
| | | | | WO | 2009073669 | A1 | 11 June 2009 |
| | | | | EP | 2227475 | A1 | 15 September 2010 |
| | | | | EP | 2227475 | B1 | 19 February 2014 |
| | | | | US | 8269008 | B2 | 18 September 2012 |
| CN | 101460497 | A | 17 June 2009 | ZA | 200808410 | B | 30 December 2009 |

Form PCT/ISA/210 (patent family annex) (July 2022)

**INTERNATIONAL SEARCH REPORT**
**Information on patent family members**

International application No.
**PCT/CN2023/119468**

| Patent document cited in search report | Publication date (day/month/year) | Patent family member(s) | | | Publication date (day/month/year) |
|---|---|---|---|---|---|
| | | AU | 2007234408 | A1 | 11 October 2007 |
| | | AU | 2007234408 | B2 | 19 May 2011 |
| | | HK | 1127046 | A1 | 02 October 2009 |
| | | IL | 194311 | A | 24 September 2015 |
| | | JP | 2009532502 | A | 10 September 2009 |
| | | JP | 5111491 | B2 | 09 January 2013 |
| | | CA | 2647596 | A1 | 11 October 2007 |
| | | CA | 2647596 | C | 12 June 2012 |
| | | RU | 2008143564 | A | 10 May 2010 |
| | | RU | 2461557 | C2 | 20 September 2012 |
| | | EP | 3124489 | A1 | 01 February 2017 |
| | | EP | 3124489 | B1 | 15 July 2020 |
| | | KR | 20080112350 | A | 24 December 2008 |
| | | KR | 101084635 | B1 | 18 November 2011 |
| | | BRPI | 0710527 | A2 | 05 June 2012 |
| | | BRPI | 0710527 | B1 | 03 September 2019 |
| | | NO | 20084627 | L | 26 November 2008 |
| | | MX | 2008012734 | A | 29 January 2009 |
| | | US | 2008004309 | A1 | 03 January 2008 |
| | | US | 7696223 | B2 | 13 April 2010 |
| | | WO | 2007115315 | A2 | 11 October 2007 |
| | | EP | 2016078 | A2 | 21 January 2009 |
| | | EP | 2016078 | B1 | 15 June 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 16757333 B